(19) Europäisches Patentamt
European Patent Office
Office européen des brevets

(11) EP 0 966 454 B1

(12) **EUROPÄISCHE PATENTSCHRIFT**

(45) Veröffentlichungstag und Bekanntmachung des Hinweises auf die Patenterteilung:
**07.05.2003 Patentblatt 2003/19**

(21) Anmeldenummer: **98909468.5**

(22) Anmeldetag: **16.02.1998**

(51) Int Cl.[7]: **C07D 277/64**, A61K 31/425, C07D 235/14, C07D 409/06, C07D 209/14, C07D 495/04

(86) Internationale Anmeldenummer:
**PCT/EP98/00865**

(87) Internationale Veröffentlichungsnummer:
**WO 98/037075 (27.08.1998 Gazette 1998/34)**

(54) **DISUBSTITUIERTE BICYCLISCHE HETEROCYCLEN, IHRE HERSTELLUNG UND IHRE VERWENDUNG ALS ARZNEIMITTEL**

DISUBSTITUTED BICYCLIC HETEROCYCLES, THEIR PRODUCTION AND USE AS MEDICAMENTS

HETEROCYCLES BICYCLIQUES DISUBSTITUES, PRODUCTION ET UTILISATION COMME MEDICAMENTS

(84) Benannte Vertragsstaaten:
**AT BE CH DE DK ES FI FR GB GR IE IT LI LU MC NL PT SE**
Benannte Erstreckungsstaaten:
**LT LV RO SI**

(30) Priorität: **18.02.1997 DE 19706229**
**24.11.1997 DE 19751939**

(43) Veröffentlichungstag der Anmeldung:
**29.12.1999 Patentblatt 1999/52**

(73) Patentinhaber: **Boehringer Ingelheim Pharma GmbH & Co.KG**
**55218 Ingelheim am Rhein (DE)**

(72) Erfinder:
- **HAUEL, Norbert**
  **D-88433 Schemmerhofen (DE)**
- **RIES, Uwe**
  **D-88400 Biberach (DE)**
- **PRIEPKE, Henning**
  **D-88447 Warthausen (DE)**
- **WIENEN, Wolfgang**
  **D-88400 Biberach (DE)**
- **STASSEN, Jean, Marie**
  **D-88447 Warthausen (DE)**

(74) Vertreter: **Laudien, Dieter, Dr.**
**Boehringer Ingelheim GmbH**
**Abteilung Patente**
**55216 Ingelheim (DE)**

(56) Entgegenhaltungen:
**EP-A- 0 540 051** **EP-A- 0 655 439**
**DE-A- 4 129 603** **US-A- 4 675 405**
**US-A- 5 416 099**

- **NAGAHARA T ET AL: "DEBASIC (AMIDINOARY) PROPANOIC ACID DERIVATIVES AS NOVEL BLOOD COAGULATION FACTOR XA INHIBITORS" JOURNAL OF MEDICINAL CHEMISTRY, Bd. 37, Nr. 8, 15.April 1994, Seiten 1200-1207, XP000608128**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 966 454 B1

## Beschreibung

Gegenstand der vorliegenden Erfindung sind neue disubstituierte bicyclische Heterocyclen der allgemeinen Formel

$$R_a - A - Het - B - Ar - E , \qquad (I)$$

deren Tautomere, deren Stereoisomere, deren Gemische und deren Salze, insbesondere deren physiologisch verträgliche Salze mit anorganischen oder organischen Säuren oder Basen, welche wertvolle Eigenschaften aufweisen.

Die Verbindungen der obigen allgemeinen Formel I, in denen E eine Cyanogruppe darstellt, stellen wertvolle Zwischenprodukte zur Herstellung der übrigen Verbindungen der allgemeinen Formel I dar, und die Verbindungen der obigen allgemeinen Formel I, in denen E eine $R_b$NH-C(=NH)-Gruppe darstellt, sowie deren Tautomere und deren Stereoisomere weisen wertvolle pharmakologische Eigenschaften auf, insbesondere eine Thrombin-hemmende und die Thrombinzeit verlängernde Wirkung.

Gegenstand der vorliegenden Anmeldung sind somit die neuen Verbindungen der obigen allgemeinen Formel I sowie deren Herstellung, die die pharmakologisch wirksamen Verbindungen enthaltende Arzneimittel und deren Verwendung.

In der obigen allgemeinen Formel bedeutet

A eine mit dem Benzo-, Pyrido- oder Thienoteil des Restes Het verknüpfte Carbonyl- oder Sulfonylgruppe,

B eine Ethylengruppe, in der die Methylengruppe, die mit dem Rest Ar verknüpft ist, durch ein Sauerstoff- oder Schwefelatom oder durch eine -$NR_1$-Gruppe ersetzt sein kann, wobei

$R_1$ ein Wasserstoffatom oder eine $C_{1-4}$-Alkylgruppe darstellt,

E eine $R_b$NH-C(=NH)-Gruppe, in der

$R_b$ ein Wasserstoffatom, eine Hydroxy-, $C_{1-9}$-Alkoxycarbonyl-, Cyclohexyloxycarbonyl-, Phenyl-$C_{1-3}$-alkoxycarbonyl-, Benzoyl-, p-$C_{1-3}$-Alkyl-benzoyl- oder Pyridinoylgruppe, wobei der Ethoxyteil in 2-Stellung der vorstehend erwähnten $C_{1-9}$-Alkoxycarbonylgruppe zusätzlich durch eine $C_{1-3}$-Alkylsulfonyl- oder 2-($C_{1-3}$-Alkoxy)-ethylgruppe substituiert sein kann, darstellt,

Ar eine gegebenenfalls durch ein Chloratom, durch eine Methyl Ethyl- oder Methoxygruppe substituierte 1,4-Phenylengruppe oder eine 2,5-Thienylengruppe,

Het einen 1-($C_{1-3}$-Alkyl)-2,5-benzimidazolylen-, 1-Cycopropyl-2,5-benzimidazolylen-, 2,5-Benzthiazolylen-, 1-($C_{1-3}$-Alkyl)-2,5-indolylen-, 1-($C_{1-3}$-Alkyl)-2,5-imidazo[4,5-b]pyridinylen-, 3-($C_{1-3}$-Alkyl)-2,7-imidazo[1,2-a]pyridinylen- oder 1-($C_{1-3}$-Alkyl)-2,5-thieno[2,3-d]imidazolylengruppe und

$R_a$ eine $R_2NR_3$-Gruppe, in der

$R_2$ eine $C_{1-4}$-Alkylgruppe, die durch eine Carboxy-, $C_{1-6}$-Alkyloxycarbonyl-, Benzyloxycarbonyl-, $C_{1-3}$-Alkylsulfonylaminocarbonyl- oder 1H-Tetrazol-5-ylgruppe substituiert sein kann,

eine durch eine Hydroxy-, Benzyloxy-, Carboxy-$C_{1-3}$-alkylamino-, $C_{1-3}$-Alkoxycarbonyl-$C_{1-3}$-alkylamino-, N-($C_{1-3}$-Alkyl)-carboxy-$C_{1-3}$-alkylamino- oder N-($C_{1-3}$-Alkyl)-$C_{1-3}$-alkoxycarbonyl-$C_{1-3}$-alkylaminogruppe substituierte $C_{2-4}$-Alkylgruppe, wobei in den vorstehend erwähnten Gruppen das zum benachbarten Stickstoffatom stehende $\alpha$-Kohlenstoffatom nicht substituiert sein kann,

$R_3$ eine $C_{3-7}$-Cycloalkylgruppe, eine Propargylgruppe, wobei der ungesättigte Teil nicht direkt mit dem Stickstoffatom der $R_2NR_3$-Gruppe verknüpft sein kann, eine gegebenenfalls durch ein Fluor- oder Chloratom, durch eine Methyl- oder Methoxygruppe substituierte Phenylgruppe, eine gegebenenfalls durch eine Methylgruppe substituierte Pyrazolyl-, Pyridazolyl- oder Pyridinylgruppe oder

$R_2$ und $R_3$ zusammen mit dem dazwischenliegenden Stickstoffatom eine gegebenenfalls durch eine Carboxy-

oder $C_{1-4}$-Alkoxycarbonylgruppe substituierte 5- bis 7-gliedrige Cycloalkyleniminogruppe, an welche zusätzlich ein Phenylring ankondensiert sein kann, darstellen.

bedeuten, deren Tautomere, deren Stereoisomere und deren Salze.

Bevorzugte Verbindungen der obigen allgemeinen Formel I sind diejenigen, in denen

A eine mit dem Benzo-, Pyrido- oder Thienoteil des Restes Het verknüpfte Carbonyl- oder Sulfonylgruppe,

B eine Ethylengruppe, in der die Methylengruppe, die mit dem Rest Ar verknüpft ist, durch ein Sauerstoff- oder Schwefelatom oder durch eine $-NR_1$-Gruppe ersetzt sein kann, wobei

$R_1$ ein Wasserstoffatom oder eine Methylgruppe darstellt,

E eine $R_bNH-C(=NH)$-Gruppe, in der

$R_b$ ein Wasserstoffatom, eine Hydroxy-, $C_{1-9}$-Alkoxycarbonyl-, Cyclohexyloxycarbonyl-, Benzyloxycarbonyl-, Benzoyl-, p-$C_{1-3}$-Alkyl-benzoyl- oder Nicotinoylgruppe, wobei der Ethoxyteil in 2-Stellung der vorstehend erwähnten $C_{1-9}$-Alkoxycarbonylgruppe zusätzlich durch eine $C_{1-3}$-Alkylsulfonyl- oder 2-($C_{1-3}$-Alkoxy)-ethylgruppe substituiert sein kann, darstellt,

Ar eine gegebenenfalls durch ein Chloratom, durch eine Methyl-, Ethyl- oder Methoxygruppe substituierte 1,4-Phenylengruppe oder eine 2,5-Thienylengruppe,

Het einen 1-Methyl-2,5-benzimidazolylen-, 1-Cyclopropyl-2,5-benzimidazolylen-, 2,5-Benzthiazolylen-, 1-Methyl-2,5-indolylen-, 1-Methyl-2,5-imidazo[4,5-b]pyridinylen-, 3-Methyl-2,7-imidazo[1,2-a]pyridinylen- oder 1-Methyl-2,5-thieno[2,3-d]-imidazolylengruppe und

$R_a$ eine $R_2NR_3$-Gruppe, in der

$R_2$ eine $C_{1-3}$-Alkylgruppe, die durch eine Carboxy-, $C_{1-6}$-Alkyloxycarbonyl-, Benzyloxycarbonyl-, Methylsulfonylaminocarbonyl- oder 1H-Tetrazol-5-ylgruppe substituiert sein kann,

eine durch eine Hydroxy-, Benzyloxy-, Carboxy-$C_{1-3}$-alkylamino-, $C_{1-3}$-Alkoxycarbonyl-$C_{1-3}$-alkylamino-, N-($C_{1-3}$-Alkyl)-carboxy-$C_{1-3}$-alkylamino- oder N-($C_{1-3}$-Alkyl)-$C_{1-3}$-alkoxycarbonyl-$C_{1-3}$-alkylaminogruppe substituierte $C_{2-3}$-Alkylgruppe, wobei in den vorstehend erwähnten Gruppen das zum benachbarten Stickstoffatom stehende $\alpha$-Kohlenstoffatom nicht substituiert sein kann, und

$R_3$ eine Propargylgruppe, wobei der ungesättigte Teil nicht direkt mit dem Stickstoffatom der $R_2NR_3$-Gruppe verknüpft sein kann, eine gegebenenfalls durch ein Fluor- oder Chloratom, durch eine Methyl- oder Methoxygruppe substituierte Phenylgruppe oder eine Pyridinylgruppe darstellen,

insbesondere diejenigen, in denen

A eine mit dem Benzo- oder Thienoteil des Restes Het verknüpfte Carbonylgruppe,

B eine Ethylengruppe, in der die Methylengruppe, die mit dem Rest Ar verknüpft ist, durch eine $-NR_1$-Gruppe ersetzt sein kann, wobei

$R_1$ ein Wasserstoffatom oder eine Methylgruppe darstellt,

E eine $R_bNH-C(=NH)$-Gruppe, in der

$R_b$ ein Wasserstoffatom, eine Hydroxy-, $C_{1-9}$-Alkoxycarbonyl-, Cyclohexyloxycarbonyl-, Benzyloxycarbonyl-, Benzoyl-, p-$C_{1-3}$-Alkyl-benzoyl- oder Nicotinoylgruppe, wobei der Ethoxyteil in 2-Stellung der vorstehend erwähnten $C_{1-9}$-Alkoxycarbonylgruppe zusätzlich durch eine Methylsulfonyl - oder 2-Ethoxy-ethylgruppe substituiert sein kann, darstellt,

Ar eine gegebenenfalls durch eine Methoxygruppe substituierte 1,4-Phenylengruppe oder eine 2,5-Thienylengruppe,

Het einen 1-Methyl-2,5-benzimidazolylen-, 2,5-Benzthiazolylen-, 1-Methyl-2,5-indolylen- oder 1-Methyl-2,5-thieno [2,3-d]imidazolylengruppe und

$R_a$ eine $R_2NR_3$-Gruppe, in der

$R_2$ eine $C_{1-3}$-Alkylgruppe, die durch eine Carboxy-, $C_{1-6}$-Alkyloxycarbonyl-, Benzyloxycarbonyl-, Methylsulfonylaminocarbonyl- oder 1H-Tetrazol-5-ylgruppe substituiert sein kann,

eine durch eine Hydroxy-, Benzyloxy-, Carboxy-$C_{1-3}$-alkylamino-, $C_{1-3}$-Alkoxycarbonyl-$C_{1-3}$-alkylamino-, N-($C_{1-3}$-Alkyl)-carboxy-$C_{1-3}$-alkylamino- oder N-($C_{1-3}$-Alkyl)-$C_{1-3}$-alkoxycarbonyl-$C_{1-3}$-alkylaminogruppe substituierte $C_{2-3}$-Alkylgruppe, wobei in den vorstehend erwähnten Gruppen das zum benachbarten Stickstoffatom stehende $\alpha$-Kohlenstoffatom nicht substituiert sein kann, und

$R_3$ eine gegebenenfalls durch ein Fluoratom substituierte Phenylgruppe oder eine 2-Pyridinylgruppe darstellen,

bedeuten, deren Tautomere, deren Stereoisomere und deren Salze.

Beispielsweise seien als besonders bevorzugte Verbindungen folgende erwähnt:

(a) 2-[N-(4-Amidinophenyl)-aminomethyl]-benzthiazol-5-carbonsäure-N-phenyl-N-(2-carboxyethyl)-amid,

(b) 2-[N-(4-Amidinophenyl)-N-methyl-aminomethyl]-benzthiazol-5-yl-carbonsäure-N-phenyl-N-(2-hydroxycarbonylethyl)-amid,

(c) 1-Methyl-2-[N-(4-amidinophenyl)-aminomethyl]-benzimidazol-5-yl-carbonsäure-N-phenyl-N-(2-hydroxycarbonylethyl)-amid,

(d) 1-Methyl-2-[N-(4-amidinophenyl)-aminomethyl]-benzimidazol-5-yl-carbonsäure-N-phenyl-N-(3-hydroxycarbonylpropyl)-amid,

(e) 1-Methyl-2-[N-(4-amidinophenyl)-aminomethyl]-benzimidazol-5-yl-carbonsäure-N-(2-pyridyl)-N-(hydroxycarbonylmethyl)-amid,

(f) 1-Methyl-2-[2-(2-amidinothiophen-5-yl)ethyl]-benzimidazol-5-yl-carbonsäure-N-(2-pyridyl)-N-(2-hydroxycarbonylethyl)-amid,

(g) 1-Methyl-2-[N-(4-amidinophenyl)-aminomethyl]-benzimidazol-5-yl-carbonsäure-N-(2-pyridyl)-N-(2-hydroxycarbonylethyl)-amid,

(h) 1-Methyl-2-[2-(4-amidinophenyl)ethyl]-benzimidazol-5-yl-carbonsäure-N-(2-pyridyl)-N-(2-hydroxycarbonylethyl)-amid,

(i) 1-Methyl-2-[2-(4-amidinophenyl)ethyl]-benzimidazol-5-yl-carbonsäure-N-phenyl-N-(2-hydroxycarbonylethyl)-amid,

(j) 1-Methyl-2-[2-(4-amidinophenyl)ethyl]-benzimidazol-5-yl-carbonsäure-N-phenyl-N-[2-(1H-tetrazol-5-yl)ethyl]-amid,

(k) 1-Methyl-2-[N-(4-amidinophenyl)-aminomethyl]-benzimidazol-5-yl-carbonsäure-N-phenyl-N-[2-(1H-tetrazol-5-yl)ethyl]-amid,

(l) 1-Methyl-2-[N-(4-amidinophenyl)-N-methyl-aminomethyl]-benzimidazol-5-yl-carbonsäure-N-(2-pyridyl)-N-(2-hydroxycarbonylethyl)-amid,

(m) 1-Methyl-2-[N-(4-amidinophenyl)-N-methyl-aminomethyl]-benzimidazol-5-yl-carbonsäure-N-(3-pyridyl) -N-(2-hydroxycarbonylethyl)-amid,

(n) 1-Methyl-2-[N-(4-amidinophenyl)-N-methyl-aminomethyl]-benzimidazol-5-yl-carbonsäure-N-phenyl-N-(2-hydroxycarbonylethyl)-amid,

(o) 1-Methyl-2-[N-(4-amidinophenyl)-aminomethyl]-benzimidazol-5-yl-carbonsäure-N-phenyl-N-[(N-hydroxycarbonylethyl-N-methyl)-2-aminoethyl]-amid,

(p) 1-Methyl-2-[N-(4-amidinophenyl)-aminomethyl]-benzimidazol-5-yl-carbonsäure-N-(3-fluorphenyl)-N-(2-hydroxycarbonylethyl)-amid,

(q) 1-Methyl-2-[N-(4-amidinophenyl)-aminomethyl]-benzimidazol-5-yl-carbonsäure-N-(4-fluorphenyl)-N-(2-hydroxycarbonylethyl)-amid,

(r) 1-Methyl-2-[N-(4-amidino-2-methoxy-phenyl)-aminomethyl]-benzimidazol-5-yl-carbonsäure-N-phenyl-N-(2-hydroxycarbonylethyl)-amid,

(s) 1-Methyl-2-[N-(4-amidino-2-methoxy-phenyl)-aminomethyl]-benzimidazol-5-yl-carbonsäure-N-(2-pyridyl) -N-(2-hydroxycarbonylethyl)-amid,

(t) 1-Methyl-2-[N-(4-amidinophenyl)aminomethyl]-indol-5-yl-carbonsäure-N-phenyl-N-(2-methoxycarbonylethyl)-amid und

(u) 1-Methyl-2-[N-(4-amidinophenyl)aminomethyl]-thieno[2.3-d]-imidazol-5-yl-carbonsäure-N-phenyl-N-(2-hydroxycarbonylethyl)-amid,

deren Tautomere, deren Stereoisomere und deren Salze.

Die neuen Verbindungen lassen sich nach an sich bekannten Verfahren herstellen, beispielsweise nach folgenden Verfahren:

a. Zur Herstellung einer Verbindung der allgemeinen Formel I, in der E eine $R_b$NH-C(=NH)-Gruppe bedeutet, in der $R_b$ ein Wasserstoffatom, eine Hydroxy- oder $C_{1-3}$-Alkylgruppe darstellt:

Umsetzung einer gegebenenfalls im Reaktionsgemisch gebildeten Verbindung der allgemeinen Formel

$$R_a - A - Het - B - Ar - C(=NH) - Z_1 , \qquad (II)$$

in der
A, B, Ar, Het und $R_a$ wie eingangs definiert sind und
$Z_1$ eine Alkoxy- oder Aralkoxygruppe wie die Methoxy-, Ethoxy-, n-Propoxy-, Isopropoxy- oder Benzyloxygruppe oder eine Alkylthio- oder Aralkylthiogruppe wie die Methylthio-, Ethylthio-, n-Propylthio- oder Benzylthiogruppe darstellt, mit einem Amin der allgemeinen Formel

$$H_2N - R_b' , \qquad (III)$$

in der
$R_b'$ ein Wasserstoffatom, eine Hydroxy- oder $C_{1-3}$-Alkylgruppe darstellt.

Die Umsetzung wird zweckmäßigerweise in einem Lösungsmittel wie Methanol, Ethanol, n-Propanol, Wasser, Methanol/Wasser, Tetrahydrofuran oder Dioxan bei Temperaturen zwischen 0 und 150°C, vorzugsweise bei Temperaturen zwischen 20 und 120°C, mit einer Verbindung der allgemeinen Formel III oder mit einem entsprechenden Säureadditionssalz wie beispielsweise Ammoniumcarbonat durchgeführt.

Eine Verbindung der allgemeinen Formel II erhält man beispielsweise durch Umsetzung einer Verbindung der allgemeinen Formel I, in der E eine Cyanogruppe darstellt, mit einem entsprechenden Alkohol wie Methanol, Ethanol, n-Propanol, Isopropanol oder Benzylalkohol in Gegenwart einer Säure wie Salzsäure oder durch Umsetzung eines entsprechenden Amids mit einem Trialkyloxoniumsalz wie Triethyloxonium-tetrafluorborat in einem Lösungsmittel wie Methylenchlorid, Tetrahydrofuran oder Dioxan bei Temperaturen zwischen 0 und 50°C, vorzugsweise jedoch bei 20°C, oder eines entsprechenden Nitrils mit Schwefelwasserstoff zweckmäßigerweise in einem Lösungsmittel wie Pyridin oder Dimethylformamid und in Gegenwart einer Base wie Triethylamin und anschließender Alkylierung des gebildeten Thioamids mit einem entsprechenden Alkyl- oder Aralkylhalogenid.

b. Zur Herstellung einer Verbindung der allgemeinen Formel I, in der die $R_a$-A-Gruppe und E mit der Maßgabe wie eingangs erwähnt definiert sind, daß die $R_a$-A-Gruppe eine Carboxygruppe enthält und E wie eingangs definiert ist oder die $R_a$-A-Gruppe wie eingangs erwähnt definiert ist und E eine $NH_2$-C(=NH)-Gruppe darstellt oder die $R_a$-A-Gruppe eine Carboxygruppe enthält und E eine $NH_2$-C(=NH)-Gruppe darstellt:

Überführung einer Verbindung der allgemeinen Formel

$$R_a' - A - Het - B - Ar - E' , \qquad (IV)$$

in der

A, B, Ar und Het wie eingangs definiert sind und

die $R_a$'-A-Gruppe und E' die für die $R_a$-A-Gruppe und E eingangs erwähnten Bedeutungen mit der Maßgabe besitzen, daß die $R_a$'-A-Gruppe eine durch Hydrolyse, Behandeln mit einer Säure oder Base, Thermolyse oder Hydrogenolyse in eine Carboxylgruppe überführbare Gruppe enthält und E wie eingangs definiert ist oder E' eine durch Hydrolyse, Behandeln mit einer Säure oder Base, Thermolyse oder Hydrogenolyse in eine $NH_2$-C(=NH) -Gruppe überführbare Gruppe darstellt und die $R_a$'-A-Gruppe die für die $R_a$-A-Gruppe eingangs erwähnten Bedeutungen aufweist oder die $R_a$'-A-Gruppe eine durch Hydrolyse, Behandeln mit einer Säure oder Base, Thermolyse oder Hydrogenolyse in eine Carboxylgruppe überführbare Gruppe enthält und E' eine durch Hydrolyse, Behandeln mit einer Säure oder Base, Thermolyse oder Hydrogenolyse in eine $NH_2$-C(=NH)-Gruppe überführbare Gruppe darstellt, mittels Hydrolyse, Behandeln mit einer Säure oder Base, Thermolyse oder Hydrogenolyse in eine Verbindung der allgemeinen Formel I übergefürt wird, in der die $R_a$-A-Gruppe und E mit der Maßgabe wie eingangs erwähnt definiert sind, daß die $R_a$-A-Gruppe eine Carboxygruppe enthält und E wie eingangs definiert ist oder die $R_a$-A-Gruppe die eingangs erwähnten Bedeutungen aufweist und E eine $NH_2$-C(=NH)-Gruppe darstellt oder die $R_a$-A-Gruppe eine Carboxygruppe enthält und E eine $NH_2$-C(=NH)-Gruppe darstellt.

Als eine in eine Carboxygruppe überführbare Gruppe kommt beispielsweise eine durch einen Schutzrest geschützte Carboxylgruppe wie deren funktionelle Derivate, z. B. deren unsubstituierte oder substituierte Amide, Ester, Thioester, Trimethylsilylester, Orthoester oder Iminoester, welche zweckmäßigerweise mittels Hydrolyse in eine Carboxylgruppe übergeführt werden,

deren Ester mit tertiären Alkoholen, z.B. der tert. Butylester, welche zweckmäßigerweise mittels Behandlung mit einer Säure oder Thermolyse in eine Carboxylgruppe übergeführt werden, und

deren Ester mit Aralkanolen, z.B. der Benzylester, welche zweckmäßigerweise mittels Hydrogenolyse in eine Carboxylgruppe übergeführt werden, in Betracht.

Die Hydrolyse wird zweckmäßigerweise entweder in Gegenwart einer Säure wie Salzsäure, Schwefelsäure, Phosphorsäure, Essigsäure, Trichloressigsäure, Trifluoressigsäure oder deren Gemischen oder in Gegenwart einer Base wie Lithiumhydroxid, Natriumhydroxid oder Kaliumhydroxid in einem geeigneten Lösungsmittel wie Wasser, Wasser/Methanol, Wasser/Ethanol, Wasser/Isopropanol, Methanol, Ethanol, Wasser/Tetrahydrofuran oder Wasser/Dioxan bei Temperaturen zwischen -10 und 120°C, z.B. bei Temperaturen zwischen Raumtemperatur und der Siedetemperatur des Reaktionsgemisches, durchgeführt.

Enthält die $R_a$'-A-Gruppe und/oder E' in einer Verbindung der Formel IV beispielsweise die tert. Butyl- oder tert.Butyloxycarbonylygruppe, so können diese auch durch Behandlung mit einer Säure wie Trifluoressigsäure, Ameisensäure, p-Toluolsulfonsäure, Schwefelsäure, Salzsäure, Phosphorsäure oder Polyphosphorsäure gegebenenfalls in einem inerten Lösungsmittel wie Methylenchlorid, Chloroform, Benzol, Toluol, Diethylether, Tetrahydrofuran oder Dioxan vorzugsweise bei Temperaturen zwischen -10 und 120°C, z.B. bei Temperaturen zwischen 0 und 60°C, oder auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 und 120°C, abgespalten werden.

Enthält die $R_a$'-A-Gruppe und/oder E' in einer Verbindung der Formel IV beispielsweise die Benzyloxy- oder Benzyloxycarbonylgruppe, so können diese auch hydrogenolytisch in Gegenwart eines Hydrierungskatalysators wie Palladium/Kohle in einem geeigneten Lösungsmittel wie Methanol, Ethanol, Ethanol/Wasser, Eisessig, Essigsäureethylester, Dioxan oder Dimethylformamid vorzugsweise bei Temperaturen zwischen 0 und 50°C, z.B. bei Raumtemperatur, und einem Wasserstoffdruck von 1 bis 5 bar abgespalten werden.

c. Zur Herstellung einer Verbindung der allgemeinen Formel I, in der die $R_a$-A-Gruppe eine der bei der Definition der $R_a$-A-Gruppe eingangs erwähnten Estergruppen enthält:
Umsetzung einer Verbindung der allgemeinen Formel

$$R_a'' - A - Het - B - Ar - E, \qquad (V)$$

in der
B, E, Ar und Het wie eingangs definiert sind und
$R_a''$-A-Gruppe die für die $R_a$-A-Gruppe eingangs erwähnten Bedeutungen mit der Maßgabe aufweist, daß die $R_a''$-A-Gruppe eine Carboxylgruppe oder eine mittels eines Alkohols in eine entsprechende Estergruppe überführbare Gruppe enthält, mit einem Alkohol der allgemeinen Formel

$$HO - R_7 , \qquad (VI)$$

in der
$R_7$ eine $C_{1-6}$-Alkyl- oder Benzylgruppe, oder mit deren Formamidacetalen
oder mit einer Verbindung der allgemeinen Formel

$$Z_2 - R_7 , \qquad (VII)$$

in der
$R_7$ wie oben definiert ist und
$Z_2$ eine Austrittsgruppe wie ein Halogenatom, z. B. ein Chloroder Bromatom, darstellen.

Die Umsetzung mit einem Alkohol der allgemeinen Formel VI wird zweckmäßigerweise in einem Lösungsmittel oder Lösungsmittelgemisch wie Methylenchlorid, Benzol, Toluol, Chlorbenzol, Tetrahydrofuran, Benzol/Tetrahydrofuran oder Dioxan, vorzugsweise jedoch in einem Alkohol der allgemeinen Formel VI, gegebenenfalls in Gegenwart einer Säure wie Salzsäure oder in Gegenwart eines wasserentziehenden Mittels, z.B. in Gegenwart von Chlorameisensäureisobutylester, Thionylchlorid, Trimethylchlorsilan, Salzsäure, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid, N,N'-Carbonyldiimidazol- oder N,N'-Thionyldiimidazol, Triphenylphosphin/Tetrachlorkohlenstoff oder Triphenylphosphin/Azodicarbonsäurediethylester gegebenenfalls in Gegenwart einer Base wie Kaliumcarbonat, N-Ethyl-diisopropylamin oder N,N-Dimethylamino-pyridin zweckmäßigerweise bei Temperaturen zwischen 0 und 150°C, vorzugsweise bei Temperaturen zwischen 0 und 80°C, durchgeführt.

Mit einer Verbindung der allgemeinen Formel VII wird die Umsetzung zweckmäßigerweise in einem Lösungsmittel wie Methylenchlorid, Tetrahydrofuran, Dioxan, Dimethylsulfoxid, Dimethylformamid oder Aceton gegebenenfalls in Gegenwart eines Reaktionsbeschleunigers wie Natrium- oder Kaliumiodid und vorzugsweise in Gegenwart einer Base wie Natriumcarbonat oder Kaliumcarbonat oder in Gegenwart einer tertiären organischen Base wie N-Ethyldiisopropylamin oder N-Methyl-morpholin, welche gleichzeitig auch als Lösungsmittel dienen können, oder gegebenenfalls in Gegenwart von Silberkarbonat oder Silberoxid bei Temperaturen zwischen -30 und 100°C, vorzugsweise jedoch bei Temperaturen zwischen -10 und 80°C, durchgeführt.

d. Zur Herstellung einer Verbindung der allgemeinen Formel I, in der $R_b$ einen in vivo abspaltbaren Rest darstellt:
Umsetzung einer Verbindung der allgemeinen Formel

$$R_a - A - Het - B - Ar - C(=NH) - NH_2 , \qquad (VIII)$$

in der
$R_a$, A, Het, B und Ar wie eingangs definiert sind, mit einer Verbindung der allgemeinen Formel

$$Z_2 - R_8 , \qquad (IX)$$

in der

$R_8$ eine und
$Z_2$ eine nukleofuge Austrittsgruppe wie ein Halogenatom, z.B. ein Chlor-, Brom- oder Jodatom, bedeuten.

Die Umsetzung wird vorzugsweise in einem Lösungsmittel wie Methanol, Ethanol, Methylenchlorid, Tetrahydrofuran, Toluol, Dioxan, Dimethylsulfoxid oder Dimethylformamid gegebenenfalls in Gegenwart einer anorganischen oder einer tertiären organischen Base, vorzugsweise bei Temperaturen zwischen 20°C und der Siedetemperatur des verwendeten Lösungsmittel, durchgeführt.

Mit einer Verbindung der allgemeinen Formel IX, in der $Z_2$ eine nukleofuge Austrittsgruppe darstellt, wird die Umsetzung vorzugsweise in einem Lösungsmittel wie Methylenchlorid, Acetonitril, Tetrahydrofuran, Toluol, Dimethylformamid oder Dimethylsulfoxid gegebenenfalls in Gegenwart einer Base wie Natriumhydrid, Kaliumcarbonat, Kalium-tert.butylat oder N-Ethyldiisopropylamin bei Temperaturen zwischen 0 und 60°C, durchgeführt. tertiären organische Base wie Triethylamin, N-Ethyl-diisopropylamin oder N-Methyl-morpholin bei Temperaturen zwischen 0 und 150°C, vorzugsweise bei Temperaturen zwischen 50 und 100°C, durchgeführt.

e. Zur Herstellung einer Benzimidazolyl- oder Benzthiazolyl verbindung der allgemeinen Formel I, in der B eine Ethylengruppe darstellt:

Umsetzung einer Verbindung der allgemeinen Formel

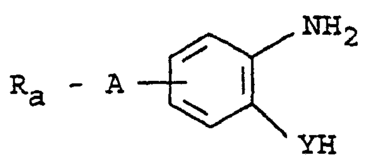

, (XV)

in der
$R_a$ und A wie eingangs erwähnt definiert sind und Y ein Schwefelatom oder ein durch eine $C_{1-3}$-Alkyl- oder Cyclopropylgruppe substituiertes Stickstoffatom bedeutet, mit einer Verbindung der allgemeinen Formel

$$HO\text{-}CO - CH_2CH_2 - Ar - E , \qquad (XVI)$$

in der
Ar und E wie eingangs erwähnt definiert sind, oder mit deren reaktionsfähigen Derivaten.

Die Umsetzung wird zweckmäßigerweise in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan gegebenenfalls in Gegenwart eines wasserentziehenden Mittels, z.B. in Gegenwart von Chlorameisensäureisobutylester, Orthokohlensäuretetraethylester, Orthoessigsäuretrimethylester, 2,2-Dimethoxypropan, Tetramethoxysilan, Thionylchlorid, Trimethylchlorsilan, Phosphortrichlorid, Phosphorpentoxid, N,N'-Dicyclohexylcarbodiimid, N, N'-Dicyclohexylcarbodiimid/N-Hydroxysuccinimid, N,N'-Dicyclohexylcarbodiimid/1-Hydroxy-benztriazol, 2-(1H-Benzotriazol-1-yl)-1,1,3,3-tetramethyluronium-tetrafluorborat, 2-(1H-Benzotriazol-1-yl)-1,1,3,3-tetramethyluronium-tetrafluorborat/1-Hydroxybenztriazol, N,N'-Carbonyldiimidazol oder Triphenylphosphin/Tetrachlorkohlenstoff, und gegebenenfalls unter Zusatz einer Base wie Pyridin, 4-Dimethylaminopyridin, N-Methyl-morpholin oder Triethylamin zweckmäßigerweise bei Temperaturen zwischen 0 und 150°C, vorzugsweise bei Temperaturen zwischen 0 und 100°C, durchgeführt.

Die Umsetzung einer entsprechenden reaktionsfähigen Verbindung der allgemeinen Formel XVI wie deren Ester, Imidazolide oder Halogeniden mit einem Amin der allgemeinen Formel XV wird vorzugsweise in einem Lösungsmittel wie Methylenchlorid, Ether oder Tetrahydrofuran und vorzugsweise in Gegenwart einer tertiären organische Base wie Triethylamin, N-Ethyl-diisopropylamin oder N-Methyl-morpholin, welche gleichzeitig als Lösungsmittel dienen können, bei Temperaturen zwischen 0 und 150°C, vorzugsweise bei Temperaturen zwischen 50 und 100°C, durchgeführt.

f. Zur Herstellung einer Verbindung der allgemeinen Formel I, in der $R_2$ eine $C_{1-4}$-Alkylgruppe, die durch eine Alkylsulfonylaminocarbonylgruppe substituiert ist:

Umsetzung einer Verbindung der allgemeinen Formel

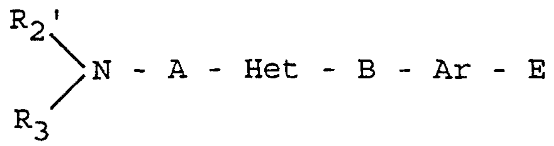

, (IXX)

in der

$R_3$, A, B, E, und Het wie eingangs erwähnt definiert sind und

$R_2$' eine $C_{1-4}$-Alkylgruppe, die durch eine Carboxygruppe substituiert ist, darstellt oder deren reaktionsfähigen Derivaten, mit einem Salz einer Verbindung der allgemeinen Formel

$$C_{1-3}\text{-Alkyl-}SO_2\text{-}NH_2 \qquad (XX).$$

Die Umsetzung wird vorzugsweise mit einer entsprechenden reaktionsfähigen Verbindung der allgemeinen Formel IXX wie deren Ester, Imidazolide oder Halogenide mit einem Salz einer Verbindung der allgemeinen Formel XX, vorzugsweise mit dessen Alkalisalz wie dessen Natriumsalz, in einem Lösungsmittel wie Methylenchlorid, Ether, Ethanol, Tetrahydrofuran oder Dimethylformamid bei Temperaturen zwischen 0 und 150°C, vorzugsweise bei Temperaturen zwischen 50 und 100°C, durchgeführt.

Bei den vorstehend beschriebenen Umsetzungen können gegebenenfalls vorhandene reaktive Gruppen wie Hydroxy-, Carboxy-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, tert.Butyl-, Trityl-, Benzyl- oder Tetrahydropyranylgruppe,
als Schutzreste für eine Carboxylgruppe die Trimethylsilyl-, Methyl-, Ethyl-, tert.Butyl-, Benzyl- oder Tetrahydropyranylgruppe und
als Schutzrest für eine Amino-, Alkylamino- oder Iminogruppe die Acetyl-, Trifluoracetyl-, Benzoyl-, Ethoxycarbonyl-, tert.Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z.B. in Gegenwart von Jodtrimethylsilan, bei Temperaturen zwischen 0 und 100°C, vorzugsweise bei Temperaturen zwischen 10 und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0 und 50°C, vorzugsweise jedoch bei Raumtemperatur, erfolgen.

Die Abspaltung eines 2,4-Dimethoxybenzylrestes erfolgt jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Die Abspaltung eines Allyloxycarbonylrestes erfolgt durch Behandlung mit einer katalytischen Menge Tetrakis-(triphenylphosphin)-palladium(O) vorzugsweise in einem Lösungsmittel wie Tetrahydrofuran und vorzugsweise in Gegenwart eines Überschusses von einer Base wie Morpholin oder 1,3-Dimedon bei Temperaturen zwischen 0 und 100°C, vorzugsweise bei Raumtemperatur und unter Inertgas, oder durch Behandlung mit einer katalytischen Menge von Tris-(triphenylphosphin)-rhodium(I)chlorid in einem Lösungsmittel wie wässrigem Ethanol und gegebenenfalls in

Gegenwart einer Base wie 1,4-Diazabicyclo[2.2.2]octan bei Temperaturen zwischen 20 und 70°C.

Die als Ausgangsstoffe verwendeten Verbindungen der allgemeinen Formeln II bis XX, welche teilweise literaturbekannt sind, erhält man nach literaturbekannten Verfahren, des weiteren wird ihre Herstellung in den Beispielen beschrieben.

So erhält man beispielsweise eine Verbindung der allgemeinen Formel II durch Umsetzung eines entsprechenden Nitrils, welches seinerseits zweckmäßigerweise gemäß den Verfahren f bis h erhalten wird, mit einem entsprechenden Thio- oder Alkohol in Gegenwart von Chlor- oder Bromwasserstoff.

Eine als Ausgangsstoff verwendete Verbindung der allgemeinen Formeln IV, V, VIII, X und IXX erhält man zweckmäßigerweise gemäß einem Verfahren der vorliegenden Erfindung.

Eine Ausgangsverbindung der allgmeinen Formel XI, in der U eine Halogenmethylgruppe darstellt, erhält man zweckmäßigerweise durch Ringschluß eines entsprechenden Esters, der in o-Stellung durch ein geeignetes Halogenatom und eine Methoxyacetamidogruppe substituiert ist, zu einer entsprechenden bicyclischen 2-Alkoxymethylverbindung, gegebenenfalls anschließende Hydrolyse und gegebenenfalls anschließende Amidierung einer so erhaltenen Carbonsäure mit einem entsprechenden Amin, Überführung der so erhaltenen Alkoxymethylverbindung in die entsprechende Halogenmethylverbindung, welche erforderlichenfalls anschließend mittels einer entsprechenden Verbindung in die gewünschte Verbindung übergeführt werden kann. Führt man hierbei den Ringschluß mit einem geeigneten Kohlensäurederivat durch, so erhält man eine Ausgangsverbindung der allgmeinen Formel XI, in der U eine Hydroxy-, Mercapto- oder Aminogruppe darstellt.

Eine Ausgangsverbindung der allgemeinen Formel XIII erhält man durch Ringschluß eines entsprechenden o-disubstituierten Esters, anschließende Verseifung des so erhaltenen Esters und anschließende Amidierung der so erhaltenen Carbonsäure mit einem entsprechenden Amin.

Ferner kann ein durch Ringschluß erhaltenes in 5-Stellung durch eine Methylgruppe substituiertes Imidazopyridin über das entsprechende N-Oxid in die entsprechende Hydroxymethylverbindung übergeführt werden, welche mittels Oxidation in die gewünschte Carbonsäure der allgemeinen Formel XIII überführt wird.

Die als Ausgangsstoffe verwendeten Verbindungen der allgemeinen Formeln III, VI, VII, IX und XII erhält man nach trivialen Methoden, beispielsweise durch Reduktion eines aromatischen

Esters, der in o-Stellung durch eine gegebenfalls substituierte Aminogruppe und eine Nitrogruppe substituiert ist, und gegebenenfalls anschließendem Ringschluß der so erhaltenen o-Diaminoverbindung mit einer entsprechenden Carbonsäure.

Ferner können die erhaltenen Verbindungen der allgemeinen Formel I in ihre Enantiomeren und/oder Diastereomeren aufgetrennt werden.

So lassen sich beispielsweise die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestes 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Apfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise der (+)- oder (-)-Menthyloxycarbonylrest in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxygruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Ethanolamin, Diethanolamin und Triethanolamin in Betracht.

Wie bereits eingangs erwähnt, weisen die neuen Verbindungen der allgemeinen Formel I und deren Salze wertvolle Eigenschaften auf. So stellen die Verbindungen der allgemeinen Formel I, in der E eine Cyanogruppe darstellt,

wertvolle Zwischenprodukte zur Herstellung der übrigen Verbindungen der allgemeinen Formel I dar und die Verbindungen der allgemeinen Formel I, in der E eine $R_b$NH-C(=NH)-Gruppe darstellt, sowie deren Tautomeren, deren Stereoisomeren, deren physiologisch verträglichen Salze wertvolle pharmakologische Eigenschaften auf, insbesondere eine thrombinhemmende Wirkung, eine die Thrombinzeit verlängernde Wirkung und eine Hemmwirkung auf verwandte Serinproteasen wie z. B. Trypsin, Urokinase Faktor VIIa, Faktor Xa, Faktor IX, Faktor XI und Faktor XII, wobei auch einige Verbindungen wie beispielsweise die Verbindung des Beispiels 16 gleichzeitig auch eine geringe thrombozytenaggregationshemmende Wirkung aufweist.

Beispielsweise wurden die Verbindungen

A = 2-[N-(4-Amidinophenyl)-aminomethyl]-benzthiazol-5-carbonsäure-N-phenyl-N-(2-carboxyethyl)-amid,

B = 1-Methyl-2-[2-(4-amidinophenyl)ethyl]-benzimidazol-5-yl-carbonsäure-N-phenyl-N-(3-hydroxycarbonylpropyl)-amid,

C = 1-Methyl-2-[(4-amidinophenyl)oxymethyl]-benzimidazol-5-yl-carbonsäure-N-phenyl-N-(hydroxycarbonylmethyl)-amid,

D = 1-Methyl-2-[N-(4-amidinophenyl)-aminomethyl]-benzimidazol-5-yl-carbonsäure-N-phenyl-N-(2-hydroxycarbonylethyl)-amid,

E = 1-Methyl-2-[N-(4-amidinophenyl)-aminomethyl]-benzimidazol-5-yl-carbonsäure-N-(2-pyridyl)-N-(hydroxycarbonylmethyl)-amid,

F = 1-Methyl-2-[2-(4-amidinophenyl)ethyl]-benzimidazol-5-yl-carbonsäure-N-phenyl-N-[2-(1H-tetrazol-5-yl)ethyl]-amid und

G = 1-Methyl-2-[N-(4-amidinophenyl)-aminomethyl]-benzimidazol-5-yl-carbonsäure-N-(2-pyridyl)-N-(2-hydroxycarbonylethyl)-amid

auf ihre Wirkung auf die Thrombinzeit wie folgt untersucht:

Material: Plasma, aus humanem Citratblut.
Test-Thrombin (Rind), 30 U/ml, Behring Werke, Marburg
Diethylbarbituratacetat-Puffer, ORWH 60/61, Behring Werke, Marburg
Biomatic B10 Koagulometer, Sarstedt

Durchführung:

Die Bestimmung der Thrombinzeit erfolgte mit einem Biomatic B10-Koagulometer der Firma Sarstedt.

Die Testsubstanz wurde in die vom Hersteller vorgeschriebenen Testgefäßen mit 0,1 ml humanem Citrat-Plasma und 0,1 ml Diethylbarbiturat-Puffer (DBA-Puffer) gegeben. Der Ansatz wurde für eine Minute bei 37°C inkubiert. Durch Zugabe von 0,3 U Test-Thrombin in 0,1 ml DBA-Puffer wurde die Gerinnungsreaktion gestartet. Gerätebedingt erfolgt mit der Eingabe von Thrombin die Messung der Zeit bis zur Gerinnung des Ansatzes. Als Kontrolle dienten Ansätze bei denen 0,1 ml DBA-Puffer zugegeben wurden.

Gemäß der Definition wurde über eine Dosis-Wirkungskurve die effective Substanzkonzentration ermittelt, bei der die Thrombinzeit gegenüber der Kontrolle verdoppelt wurde.

Die nachfolgende Tabelle enthält die gefundenen Werte:

| Substanz | Thrombinzeit ($ED_{200}$ in µM) |
|---|---|
| A | 0.04 |
| B | 0.06 |
| C | 0.15 |
| D | 0.03 |
| E | 0.09 |
| F | 0.03 |

(fortgesetzt)

| Substanz | Thrombinzeit ($ED_{200}$ in µM) |
|---|---|
| G | 0.03 |

Beispielsweise konnte an Ratten bei der Applikation der Verbindungen A, D, E und G bis zu einer Dosis von 1 mg/kg i.v. keine akuten toxischen Nebenwirkungen beobachtet werden. Diese Verbindungen sind demnach gut verträglich.

Aufgrund ihrer pharmakologischen Eigenschaften eignen sich die neuen Verbindungen und deren physiologisch verträglichen Salze zur Vorbeugung und Behandlung venöser und arterieller thrombotischer Erkrankungen, wie zum Beispiel der Behandlung. von tiefen Beinvenen-Thrombosen, der Verhinderung von Reocclusionen nach Bypass-Operationen oder Angioplastie (PT(C)A), sowie der Occlusion bei peripheren arteriellen Erkrankungen wie Lungenembolie, der disseminierten intravaskulären Gerinnung, der Prophylaxe der Koronarthrombose, der Prophylaxe des Schlaganfalls und der Verhinderung der Occlusion von Shunts oder Stents. Zusätzlich sind die erfindungsgemäßen Verbindungen zur antithrombotischen Unterstützung bei einer thrombolytischen Behandlung, wie zum Beispiel mit rt-PA oder Streptokinase, zur Verhinderung der Langzeitrestenose nach PT(C)A, zur Verhinderung der Metastasierung und des Wachstums von koagulationsabhängigen Tumoren und von fibrinabhängigen Entzündungsprozessen geeignet.

Die zur Erzielung einer entsprechenden Wirkung erforderliche Dosierung beträgt zweckmäßigerweise bei intravenöser Gabe 0,1 bis 30 mg/kg, vorzugsweise 0,3 bis 10 mg/kg, und bei oraler Gabe 0,1 bis 50 mg/kg, vorzugsweise 0,3 bis 30 mg/kg, jeweils 1 bis 4 x täglich. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen oder Zäpfchen einarbeiten.

Die nachfolgenden Beispiele sollen die Erfindung nähers erläutern:

Vorbemerkungen

Bei der Bestimmung der $R_f$-Werte wurden, soweit nichts anderes angegeben wurde, immer Polygram-Kieselgelplatten der Firma E. Merck, Darmstadt, verwendet.

Die EKA-Massenspektren (Elektrospray-Massenspektren von Kationen) werden beispielsweise in Chemie unserer Zeit 6,308-316 (1991) beschrieben.

Beispiel 1

3-Methyl-2-[2-(4-amidinophenyl)ethyl]-imidazo[4,5-b]pyridin-6-carbonsäure-N-phenyl-N-(2-ethoxycarbonylethyl)-amid

a) 6-Methylamino-5-nitro-nicotinsäuremethylester

1.6 g (7.4 mMol) 6-Chlor-5-nitro-nicotinsäuremethylester (siehe Bernie et al. in J. Chem. Soc. 1951, 2590) wurden in 20 ml wässriger 40%iger Methylaminlösung 30 Minuten bei Raumtemperatur gerührt. Das Reaktionsgemisch wurde anschließend mit Eiswasser verdünnt, der ausgefallene gelbe Niederschlag abfiltriert und getrocknet.
Ausbeute: 1.2 g (80 % der Theorie),
$R_f$-Wert: 0.66 (Kieselgel; Essigester/Ethanol/Eisessig = 90:5:5)

b) 5-Amino-6-methylamino-nicotinsäuremethylester

Zu einer Lösung von 3.1 g (15 mMol) 6-Methylamino-5-nitronicotinsäuremethylester in 100 ml Ethanol/Dichlormethan (3:1) wurde 1 g Palladium auf Kohle (10%ig) gegeben und die resultierende Suspension bei 5 bar Wasserstoffdruck 1.5 Stunden bei Raumtemperatur hydriert. Anschließend wurde der Katalysator abfiltriert und das Lösungsmittel im Vakuum abdestilliert. Das erhaltene ölige Rohprodukt wurde direkt weiter umgesetzt.
Ausbeute: 2.4 g (92 % der Theorie),
$R_f$-Wert: 0.44 (Kieselgel; Essigester/Ethanol/Ammoniak = 90:10:1)

c) 5-[2-(4-Cyanophenyl)ethylcarbonylamino]-6-methylaminonicotinsäuremethylester

Eine Lösung von 2.6 g (15 mMol) 3-(4-Cyanophenyl)propionsäure in 25 ml absolutem Tetrahydrofuran wurde mit 2.4 g (15 mMol) N,N'-Carbonyldiimidazol versetzt und 20 Minuten bei Raumtemperatur gerührt. Anschließend versetzte man das Imidazolid mit einer Lösung von 2.3 g (13 mMol) 5-Amino-6-methylamino-nicotinsäuremethylester in 25 ml Dimethylformamid und erwärmte 3 Stunden auf 100°C. Nach dem Entfernen des Lösungsmittels im Vakuum wurde das erhaltene Rohprodukt in Essigester aufgenommen, die organische Phase mit Wasser gewaschen und nach Trocknen über Natriumsulfat erneut vom Lösungsmittel befreit. Der erhaltene Rückstand wurde durch Flash-Chromatographie gereinigt (Kieselgel; Gradient: Dichlormethan bis Dichlormethan/Ethanol = 19:1).
Ausbeute: 2.1 g beigefarbiger Feststoff (50 % der Theorie),
$R_f$-Wert: 0.54 (Kieselgel; Essigester/Ethanol/Ammoniak = 90:10:1)

d) 3-Methyl-2-[2-(4-cyanophenyl)ethyl]-imidazo[4,5-b]pyridin-6-carbonsäuremethylyester

Eine Lösung aus 2.0 g (5.9 mMol) 5-[2-(4-Cyanophenyl)ethylcarbonylamino]-6-methylamino-nicotinsäuremethylester in 50 ml Eisessig wurde 1 Stunde auf 100°C erhitzt. Nach Entfernen des Lösungsmittels wurde in Dichlormethan aufgenommen, mit Natriumhydrogencarbonat-Lösung gewaschen, mit Natriumsulfat getrocknet und erneut das Lösungsmittel abdestilliert.
Ausbeute: 1.7 g brauner Feststoff (89 % der Theorie),
$R_f$-Wert: 0.50 (Kieselgel; Essigester/Ethanol/Ammoniak = 90:10:1)

e) 3-Methyl-2-[2-(4-cyanophenyl)ethyl]-imidazo[4,5-b]pyridin-6-carbonsäure

Eine Lösung von 3.2 g (10 mMol) 3-Methyl-2-[2-(4-cyanophenyl)-ethyl]-imidazo[4.5-b]pyridin-6-carbonsäuremethylester in 150 ml Methanol wurde mit einer Lösung von 1.5 g Lithiumhydroxid in 20 ml Wasser versetzt und 24 Stunden bei Raumtemperatur gerührt. Anschließend wurde mit 50 ml Wasser verdünnt, der Alkohol abdestilliert und die wäßrige Phase mit Essigester gewaschen. Nach Ansäuern mit verdünnter Salzsäure wurde mehrmals mit Dichlormethan/Methanol (9:1) extrahiert, die organische Phase mit Natriumsulfat getrocknet und das Lösungsmittel abdestilliert.
Ausbeute: 2.1 g beigefarbiger Feststoff (70 % der Theorie),
$R_f$-Wert: 0.38 (Kieselgel; Essigester/Ethanol/Ammoniak = 50:45:5)

f) 3-Methyl-2-[2-(4-cyanophenyl)ethyl]-imidazo[4,5-b]pyridin-6-carbonsäure-N-phenyl-N-(2-ethoxycarbonylethyl)-amid

Eine Lösung aus 2.0 g (6.5 mMol) 3-Methyl-2-[2-(4-cyanophenyl)-ethyl]-imidazo[4,5-b]pyridin-6-carbonsäure in 100 ml Dichlormethan wurde mit 20 ml Thionylchlorid versetzt und 2 Stunden am Rückfluß gekocht. Nach Abdestillieren der flüssigen Komponenten wurde das Rohprodukt noch zweimal in Dichlormethan aufgenommen und jeweils das Lösungsmittel abdestilliert. Das so erhaltene rohe Säurechlorid (2 g) wurde in 100 ml Tetrahydrofuran suspendiert und mit 1.2 g (6.5 mMol) N-(2-Ethoxycarbonylethyl)anilin versetzt. Anschließend wurde innerhalb von 5 Minuten 0.73 g (7.2 mMol) Triethylamin zugetropft. Nach 1-stündigem Rühren wurde das Lösungsmittel im Vakuum abdestilliert, der Rückstand in Essigester aufgenommen, die organische Phase mit Wasser gewaschen und mit Natriumsulfat getrocknet. Nach Abdestillieren des Lösungsmittels und Flash-Chromatographie (Kieselgel; Dichlormethan bis Dichlormethan/Ethanol = 49:1) isolierte man die gewünschte Verbindung als bräunliches Öl.
Ausbeute: 1.9 g (65 % der Theorie),
$R_f$-Wert: 0.44 (Kieselgel; Essigester/Ethanol/Ammoniak = 90:10:1)

g) 3-Methyl-2-[2-(4-amidinophenyl)ethyl]-imidazo[4,5-b]pyridin-6-carbonsäure-N-phenyl-N-(2-ethoxycarbonylethyl)-amid

1.8 g (3.7 mMol) 3-Methyl-2-[2-(4-cyanophenyl)ethyl]-imidazo-[4,5-b]pyridin-6-carbonsäure-N-phenyl-N-(2-ethoxycarbonylethyl)-amid wurden in 100 1 mit Chlorwasserstoff gesättigtem Ethanol 16 Stunden erst bei 0°C und dann bei Raumtemperatur so lange gerührt, bis dünnschichtchromatographisch kein Ausgangsmaterial mehr nachweisbar war. Anschließend wurde das Lösungsmittel abdestilliert, der ölige Rückstand in 50 ml absolutem Ethanol aufgenommen und mit 3.6 g (37 mMol) Ammoniumcarbonat versetzt. Nach 4 Stunden wurde das Lösungsmittel im Vakuum abdestilliert, das erhaltene Rohprodukt durch Flash-Chromatographie (Kieselgel; Gradient: Dichlormethan/Ethanol 19:1 bis 4:1) gereinigt und erneut eingeengt.
Ausbeute: 1.6 g beigefarbener Feststoff (80 % der Theorie),

$R_f$-Wert: 0.30 (Kieselgel; Essigester/Ethanol/Ammoniak = 90:5:5)

Beispiel 2

3-Methyl-2-[2-(4-amidinophenyl)ethyl]-imidazo[4,5-b]pyridin-6-carbonsäure-N-phenyl-N-(2-hydroxycarbonylethyl)-amid

Eine Lösung von 535 mg (1.0 mMol) 3-Methyl-2-[2-(4-amidinophenyl) ethyl] -imidazo [4, 5-b]pyridin-6-carbonsäure-N-phenyl-N-(2-ethoxycarbonylethyl)-amid in 10 ml Ethanol wurde mit 5 ml 2N Natronlauge versetzt und 2 Stunden bei Raumtemperatur gerührt. Anschließend wurde mit 10 ml Wasser verdünnt, der Alkohol abdestilliert, die wäßrige Phase mit 20 ml Essigester gewaschen und mit konzentrierter Salzsäure angesäuert, wobei die gewünschte Verbindung als weiße Kristalle ausfiel.
Ausbeute: 375 mg (74 % der Theorie),
$R_f$-Wert: 0.23 (Kieselgel; Essigester/Ethanol/Ammoniak = 90:5:5)
$C_{26}H_{26}N_6O_3$ (470.54)
Massenspektrum: $(M+H)^+ = 471$

Beispiel 3

3-Methyl-2-[2-(4-amidinophenyl)ethyl]-imidazo[4,5-b]pyridin-6-yl-carbonsäure-N-(2-pyridyl)-N-(2-methoxycarbonylethyl)-amidhydrochlorid

Hergestellt analog Beispiel 1 aus 3-Methyl-2-[2-(4-cyanophenyl)ethyl]-imidazo[4,5-b]pyridin-6-yl-carbonsäure-N-(2-pyridyl)-N-(2-methoxycarbonylethyl)-amid, methanolischer Salzsäure, Methanol und Ammoniumcarbonat.
Ausbeute: 75 % der Theorie,
$C_{26}H_{27}N_7O_3$ (485.55)
$R_f$-Wert: 0.31 (Kieselgel; Essigester/Ethanol/Ammoniak = 50:45:5)
EKA-Massenspektrum: $(M+H)^+ = 486$

Beispiel 4

3-Methyl-2-[2-(4-amidinophenyl)ethyl]-imidazo[4,5-b]pyridin-6-yl-carbonsäure-N-phenyl-N-ethoxycarbonylmethyl-amid-hydrochlorid

Hergestellt analog Beispiel 1 aus 3-Methyl-2-[2-(4-cyanophenyl)ethyl]-imidazo[4,5-b]pyridin-6-yl-carbonsäure-N-phenyl-N-ethoxycarbonylmethyl-amid, ethanolischer Salzsäure, Ethanol und Ammoniumcarbonat.
Ausbeute: 84 % der Theorie,
$C_{27}H_{28}N_6O_3$ (484.56)
$R_f$-Wert: 0.44 (Kieselgel; Essigester/Ethanol/Ammoniak = 50:45:5)
EKA-Massenspektrum: $(M+H)^+ = 485$

Beispiel 5

3-Methyl-2-[2-(4-amidinophenyl)ethyl]-imidazo[4,5-b]pyridin-6-yl-carbonsäure-N-phenyl-N-hydroxycarbonylmethyl-amid-hydrochlorid

Hergestellt analog Beispiel 2 aus 3-Methyl-2-[2-(4-amidinophenyl)ethyl]-imidazo[4,5-b]pyridin-6-yl-carbonsäure-N-phenyl-N-ethoxycarbonylmethyl-amid-hydrochlorid und Natronlauge.
Ausbeute: 85 % der Theorie,
$C_{25}H_{24}N_6O_3$ (456.51)
$R_f$-Wert: 0.19 (Kieselgel; Essigester/Ethanol/Ammoniak = 50:45:5)
EKA-Massenspektrum: $(M+H)^+ = 457$

Beispiel 6

2-[2-(4-Amidinophenyl)ethyl]-3-methyl-6-(2-methoxycarbonyl-2,3-dihydroindol-1-yl-carbonyl)-imidazo[4,5-b]pyridin-hydrochlorid

Hergestellt analog Beispiel 1 aus 2-[2-(4-Cyanophenyl)ethyl]-3-methyl-6-(2-methoxycarbonyl-2,3-dihydroindol-1-yl-carbonyl)-imidazo[4,5-b]pyridin, methanolischer Salzsäure, Methanol und Ammoniumcarbonat.
Ausbeute: 20 % der Theorie,
$C_{27}H_{26}N_6O_3$ (482.54)
$R_f$-Wert: 0.30 (Kieselgel; Essigester/Ethanol/Ammoniak = 50:45:5)
EKA-Massenspektrum: $(M+H)^+ = 483$

Beispiel 7

2-[2-(4-Amidinophenyl) ethyl]-3-methyl-6-(2-carboxy-2,3-dihydroindol-1-yl-carbonyl)-imidazo[4,5-b]pyridin-hydrochlorid

Hergestellt analog Beispiel 2 aus 2-[2-(4-Amidinophenyl)ethyl]-3-methyl-6-(2-methoxycarbonyl-2,3-dihydroindol-1-yl-carbonyl)-imidazo[4,5-b]pyridin-hydrochlorid und Natronlauge.
Ausbeute: 90 % der Theorie,
$C_{26}H_{24}N_6O_3$ (468.52)
$R_f$-Wert: 0.24 (Kieselgel; Essigester/Ethanol/Ammoniak = 50:45:5)
EKA-Massenspektrum: $(M+H)^+ = 469$
$(M+Na)^+ = 491$

Beispiel 8

1-Methyl-2-[(4-amidinophenyl)oxymethyl]-imidazo[4,5-b]pyridin-5-yl-carbonsäure-N-(2-pyridyl)-N-(2-ethoxycarbonylethyl)-amid

a) 2-Amino-3-methylamino-6-methyl-pyridin

8.35 g (50 mMol) 2-Methyl-5-methylamino-6-nitro-pyridin (Heterocycles 38, 529 (1994)) wurden in 300 l Essigester gelöst und mit 1.5 g Raney-Nickel 3,5 Stunden bei Raumtemperatur hydriert. Anschließend wurde der Katalysator abfiltriert und das Filtrat eingeengt. Nach Kristallisation des erhaltenen Rückstandes aus Petrolether erhielt man 5.75 g (84 % der Theorie) als olivgrüne Kristalle.
$C_7H_{11}N_3$ (137.20)
Schmelzpunkt: 112-113°C

b) 1,5-Dimethyl-2-[(4-cyanophenyl)oxymethyl]-imidazo[4,5-b]-pyridin

11.4 g (63 mMol) 4-Cyano-phenoxyessigsäure wurden in 200 ml absolutem Tetrahydrofuran gelöst und bei Raumtemperatur mit 10.2 g (63 mMol) N,N'-Carbonyldiimidazol versetzt. Nach 15 Minuten bei 60°C wurden 5.70 g (41.5 mMol) 2-Amino-3-methylamino-6-methyl-pyridin zugesetzt. Nach 2 Stunden bei 60°C wurde das Lösungsmittel abdestilliert, der kristalline Rückstand mit Wasser versetzt, mit Wasser gewaschen und getrocknet. Nach Kristallisation aus Ethanol erhielt man 9.95 g (91 % der Theorie) als weiße Kristalle.
$C_{16}H_{14}N_4O$ (278.32)
Massenspektrum: $M^+ = 278$

c) 1,5-Dimethyl-2-[(4-cyanophenyl)oxymethyl]-imidazo[4,5-b]-pyridin-4-N-oxid

2,62 g (10 mMol) 1,5-Dimethyl-2-[(4-cyanophenyl)oxymethyl]-imidazo[4,5-b]pyridin wurden in 125 ml Dichlormethan suspendiert und mit 2,62 g (12,7 mMol) m-Chlor-perbenzoesäure versetzt, wobei eine klare Lösung entstand. Nach 2 Stunden bei Raumtemperatur wurde das Lösungsmittel abdestilliert und der erhaltene Rückstand mit einer Natriumhydrogencarbonat-Lösung versetzt. Nach 30 Minuten wurde das erhaltene weiße kristalline Produkt abgesaugt, mit Wasser gewaschen und bei 40°C getrocknet.
Ausbeute: 2,45 g (83 % der Theorie),
$C_{16}H_{14}N_4O_2$ (294.30)

Massenspektrum: $M^+$ = 294

d) 1-Methyl-2-[(4-cyanophenyl)oxymethyl]-5-hydroxymethyl-imidazo[4,5-b]pyridin

2.40 g (8.2 mMol) 1,5-Dimethyl-2-[(4-cyanophenyl)oxymethyl]-imidazo[4,5-b]pyridin-4-N-oxid wurden in 75 ml Dichlormethan suspendiert und mit 2.4 ml Trifluoressigsäureanhydrid (16.9 mMol) vesetzt, wobei eine klare Lösung entstand. Nach 16 Stunden bei Raumtemperatur wurde das Lösungsmittel abdestilliert, der erhaltene viskose Rückstand in 50 ml Dichlormethan aufgenommen und mit 50 ml 2M Natriumhydrogencarbonat-Lösung überschichtet. Nach 3-stündigem kräftigem Rühren wurde der gebildete Niederschlag abgesaugt, mit Wasser gewaschen und bei 40°C getrocknet.
Ausbeute: 1.85 g weißes Pulver (78 % der Therorie),
$C_{16}H_{14}N_4O_2$ (294.30)
Schmelzpunkt: 172°C

e) 1-Methyl-2-[(4-cyanophenyl)oxymethyl]-imidazo[4,5-b]pyridin-5-carbaldehyd

3.65 g (12.5 mMol) 1-Methyl-2-[(4-cyanophenyl)oxymethyl]-5-hydroxymethyl-imidazo[4,5-b]pyridin wurden in 500 ml Dichlormethan gelöst und mit 15.0 g Mangandioxyd versetzt. Nach 96 Stunden bei Raumtemperatur wurde über Kieselgur filtriert und das Lösungsmittel abdestilliert. Das erhaltene Filtrat wurde eingeengt, der kristalline Niederschlag mit Ether verrieben, abgesaugt und getrocknet.
Ausbeute: 3.05 g weißes Pulver (84 % der Theorie),
$C_{16}H_{12}N_4O_2$ (292.30)
Schmelzpunkt: 231-234°C

f) 1-Methyl-2-[(4-cyanophenyl)oxymethyl]-5-carboxy-imidazo-[4.5-b]pyridin

1.25 g (4.3 mMol) 1-Methyl-2-[(4-cyanophenyl)oxymethyl]-imidazo[4,5-b]pyridin-5-carbaldehyd wurden in 10 ml Ameisensäure gelöst und bei 0°C mit 1.0 ml Wasserstoffperoxid (33%ig) versetzt. Nach 12 Stunden bei 4°C wurde der gebildete weiße Niederschlag abgesaugt, mit Wasser gewaschen und bei 40°C getrocknet.
Ausbeute: 0.81 g (61 % der Theorie),
$C_{16}H_{12}N_4O_3$ (308.7)

g)1-Methyl-2-[(4-cyanophenyl)oxymethyl]-imidazo[4,5-b]pyridin-5-yl-carbonsäure-N-(2-pyridyl)-N-(2-methoxycarbonylethyl)-amid

308 mg (1.0 mMol) 1-Methyl-2-[(4-cyanophenyl)oxymethyl]-5-carboxy-imidazo[4.5-b]pyridin wurden in 5 ml Dimethylformamid suspendiert und mit 303 mg (3.0 mMol) N-Methyl-morpholin und 321 mg (1.0 mMol) O-(Benzotriazol-1-yl)-N,N,N',N'-tetramethyluroniumtetrafluoroborat versetzt. Nach 10 Minuten bei Raumtemperatur wurde eine Lösung von 215 mg (1.2 mMol) N-(2-Pyridyl)-3-amino-propionsäuremethylester in 2 ml Dimethylformamid zugegeben, wobei eine klare Lösung enstand. Nach 12 Stunden bei Raumtemperatur wurde die Reaktionslösung in Eiswasser eingerührt. Nach dreimaliger Extraktion mit Essigester wurden die vereinigten organischen Extrakte mit einer Kochsalzlösung gewaschen, über Natriumsulfat getrocknet und eingedampft. Der erhaltene Rückstand wurde an Kieselgel mit Dichlormethan/Ethanol (90:1 bis 25:1) chromatographiert.
Ausbeute: 165 mg weißes Pulver (35 % der Theorie),
$C_{25}H_{12}N_6O_4$ (407.50)
Schmelzpunkt: 139-140°C

h) 1-Methyl-2-[(4-amidinophenyl)oxymethyl]-imidazo[4.5-b]-pyridin-5-yl-carbonsäure-N-(2-pyridyl)-N-(2-ethoxycarbonylethyl)-amid

Hergestellt durch Umsetzung von 140 mg (0.3 mMol) 1-Methyl-2-[(4-cyanophenyl)oxymethyl]-imidazo[4,5-b]pyridin-5-yl-carbonsäure-N-(2-pyridyl)-N-(2-methoxycarbonylethyl)-amid mit durch Chlorwasserstoff gesättigtem Ethanol und mit Ammoniumcarbonat/Ethanol analog Beispiel 1g. Das erhaltene Produkt wurde durch Chromatographie über Kieselgel mit Dichlormethan/Ethanol (19:1 bis 4:1) gereinigt.
Ausbeute: 48 mg weißes Pulver.(36 % der Theorie),
$C_{26}H_{27}N_7O_4$ (501.57)
Massenspektrum: $(M+H)^+$ = 502

Beispiel 9

2-[N-(4-Amidinophenyl)-aminomethyl]-benzthiazol-5-carbonsäure-N-phenyl-N-(2-ethoxycarbonylethyl)-amid

a) 4-Fluor-3-methoxyacetamido-benzoesäureethylester

Eine Lösung aus 2.8 g (15.3 mMol) 3-Amino-4-fluor-benzoesäureethylester (siehe L.S. Fosdick, A.F. Dodds in J. Amer. Chem. Soc. 65, 2305 (1943)) und 1.56 ml (1.85 g = 17.0 mMol) Methoxyacetylchlorid in 50 ml Chlorbenzol wurde 1 Stunde bei 50°C und anschließend 15 Minuten bei Rückfluß gerührt. Dann wurde das Lösungsmittel im Vakuum abdestilliert und das erhaltene Rohprodukt durch Flash-Chromatographie (Kieselgel; Dichlormethan/Ethanol = 100:1) gereinigt. Die zunächst ölig anfallende gewünschte Verbindung erstarrte innerhalb einiger Tage.
Ausbeute: 3.8 g (98 % der Theorie),
$R_f$-Wert: 0.38 (Kieselgel; Dichlormethan/Ethanol =19:1)

b) 2-Methoxymethyl-benzthiazol-5-carbonsäureethylester

Ein Gemisch aus 3.0 g (11.7 mMol) 4-Fluor-3-methoxyacetamidobenzoesäure und 2.1 g (5.2 mMol) Lawessons Reagenz wurde 6 Stunden in 90 ml Toluol unter Rückfluß erhitzt, erneut mit 1.0 g Lawessons Reagenz versetzt und weitere 6 Stunden auf 120°C erhitzt. Nach Ersetzen des Lösungsmittels durch Xylol wurde weitere 8 Stunden in einem Druckgefäß auf 180°C erhitzt. Anschließend wurde das Lösungsmittel im Vakuum abdestilliert, das erhaltene Rohprodukt durch Flash-Chromatographie (Kieselgel; Essigester/Petrolether = 5:95) gereinigt und erneut eingeengt.
Ausbeute: 2.1 g gelbe Kristalle (72 % der Theorie),
$R_f$-Wert: 0.55 (Kieselgel; Essigester/Petrolether = 3:7)

c) 2-Methoxymethyl-benzthiazol-5-carbonsäure

Ein Gemisch aus 2.1 g (8.36 mMol) 2-Methoxymethyl-benzthiazol-5-carbonsäureethylester und 16 ml 2N Natronlauge wurde in 60 ml Ethanol 1 Stunde bei Raumtemperatur gerührt. Danach wurde der Alkohol abdestilliert, das Rohprodukt in 20 ml Wasser aufgenommen, mit 50 ml Diethylether gewaschen und die wäßrige Phase mit konzentrierter Salzsäure unter Eiskühlung angesäuert. Die daraufhin ausgefallene beige-rosa-farbige Verbindung wurde abgesaugt, mit Wasser gewaschen und getrocknet.
Ausbeute: 1.6 g (86 % der Theorie),
$R_f$-Wert: 0.12 (Kieselgel; Dichlormethan/Ethanol = 29:1)

d) 2-Methoxymethyl-benzthiazol-5-carbonsäure-N-phenyl-N-(2-ethoxycarbonylethyl)-amid

Eine Suspension aus 1.6 g (7.2 mMol) 2-Methoxymethyl-benzthiazol-5-carbonsäure in 60 ml Dichlormethan wurde mit 1.6 ml (22 mMol) Thionylchlorid versetzt und 1 Stunde unter Rückfluß gekocht. Dabei löste sich der Feststoff nach 20 Minuten auf. Nach Abdestillieren der flüssigen Komponenten wurde das Rohprodukt noch zweimal in Dichlormethan aufgenommen und jeweils das Lösungsmittel abdestilliert. Das so erhaltene rohe Säurechlorid wurde in 50 ml Tetrahydrofuran aufgenommen, zu einem Gemisch aus 1.4 g (7.2 mMol) N-(2-Ethoxycarbonylethyl)anilin und 3.0 ml (21 mMol) Triethylamin in 50 ml Tetrahydrofuran getropft und über Nacht bei Raumtemperatur gerührt. Anschließend wurde das Lösungsmittel im Vakuum abdestilliert, der Rückstand in 30 ml Dichlormethan aufgenommen, diese Lösung mit Wasser gewaschen und mit Natriumsulfat getrocknet. Nach Abdestillieren des Lösungsmittels und Flash-Chromatographie (Kieselgel; Gradient: Dichlormethan/Ethanol 98.5:1.5 bis 80:20) isolierte man die gewünschte Verbindung als bräunliches Öl.
Ausbeute: 2.05 (72 % der Theorie),
$R_f$-Wert: 0.40 (Kieselgel; Essigester/Petrolether = 1:1)

e) 2-[N-(4-Cyanophenyl)-aminomethyl]-benzthiazol-5-carbonsäure-N-phenyl-N-(2-ethoxycarbonylethyl)-amid

Ein Gemisch aus 2.05 g (5.14 mMol) 2-Methoxymethyl-benzthiazol-5-carbonsäure-N-phenyl-N-(2-ethoxycarbonylethyl)-amid und 5.7 ml (5.7 mMol) einer 1M Lösung aus Bortribromid in Dichlormethan wurde in weiteren 60 ml Dichlormethan gelöst und 16 Stunden bei Raumtemperatur gerührt. Anschließend wurde mit 40 ml gesättigter Natriumhydrogencarbonat-Lösung gewaschen, die organische Phase mit Natriumsulfat getrocknet und das Lösungsmittel abdestilliert. Das so erhaltene rohe 2-Brommethyl-benzthiazol-5-carbonsäure-N-phenyl-N-(2-ethoxycarbonylethyl)-amid (2.4 g) wurde in 5.0 ml N,N-Diisopropyl-ethylamin aufgenommen und mit 0.64 g (5.4 mMol) 4-Amino-benzonitril versetzt. Nach 1-stündigem Erhitzen auf 130°C wurde das Lösungsmittel im Vakuum abdestilliert und das erhaltene

Rohprodukt durch Flash-Chromatographie (Kieselgel; Gradient: Essigester/Petrolether = 1:3 bis 1:1) gereinigt, wobei beim Einengen der Eluate ein orangefarbiger Schaum erhalten wurde.
Ausbeute: 1.1 g (44 % der Theorie),
$R_f$-Wert: 0.35 (Kieselgel; Essigester/Petrolether = 7:3)

f) 2-[N-(4-Amidinophenyl)-aminomethyl]-benzthiazol-5-carbonsäure-N-phenyl-N-(2-ethoxycarbonylethyl)-amid

1.1 g (2.27 mMol) 2-[N-(4-Cyanophenyl)-aminomethyl]-benzthiazol-5-carbonsäure-N-phenyl-N-(2-ethoxycarbonylethyl)-amid wurden in 100 ml mit Chlorwasserstoff gesättigtem Ethanol 5 Stunden erst bei 0°C und dann bei Raumtemperatur so lange gerührt, bis dünnschichtchromatographisch kein Ausgangsmaterial mehr nachweisbar war. Anschließend wurde das Lösungsmittel bei maximal 30°C Badtemperatur abdestilliert, der ölige Rückstand in 100 ml absolutem Ethanol aufgenommen und mit 1.6 g (22 mMol) Ammoniumcarbonat versetzt. Nach 18-stündigem Rühren bei Raumtemperatur wurde das Lösungsmittel im Vakuum abdestilliert und das Rohprodukt durch Flash-Chromatographie (Kieselgel; Gradient: Wasser/Methanol = 19:1 bis 4:1) gereinigt. Beim Einengen der Eluate erhält man die gewünschte Verbindung als weißen Schaum.
Ausbeute: 0.77 g (63 % der Theorie),
$R_f$-Wert: 0.19 (Kieselgel; Dichlormethan/Ethanol = 3:7)
$C_{27}H_{27}N_5O_3S$ (501.60)
Massenspektrum: $(M+H)^+ = 502$

Beispiel 10

2-[N-(4-Amidinophenyl)-aminomethyl]-benzthiazol-5-carbonsäure-N-phenyl-N-(2-carboxyethyl)-amid

0.45 g (0.84 mMol) 2-[N-(4-Amidinophenyl)-aminomethyl]-benzthiazol-5-carbonsäure-N-phenyl-N-(2-ethoxycarbonylethyl)-amid wurden in 15 ml Ethanol gelöst, mit 2 ml 2N Natronlauge versetzt und 4 Stunden bei Raumtemperatur gerührt. Anschließend wurde mit 3 ml 2N Salzsäure angesäuert und das Lösungsmittel abdestilliert. Das erhaltene Rohprodukt wurde in 5 ml Dichlormethan/Ethanol (2:1) aufgenommen und vom unlöslichen Natriumchlorid abfiltriert. Nach dem Abdestillieren des Lösungsmittels erhielt man die gewünschte Verbindung als gelben Schaum.
Ausbeute: 0.26 g (67 % der Theorie),
$R_f$-Wert: 0.47 (Kieselgel; Methanol/5 % wäßriges Natriumchlorid = 6:4)
$C_{25}H_{23}N_5O_3S$ (473.55)
Massenspektrum: $(M+H)^+ = 474$

Beispiel 11

2-[N-(4-Amidinophenyl)-aminomethyl]benzthiazol-5-yl-carbonsäure-N-(2-pyridyl)-N-(2-methoxycarbonylethyl)-amid-dihydrochlorid

Hergestellt analog Beispiel 9 aus 2-[N-(4-Cyanophenyl)-aminomethyl]benzthiazol-5-yl-carbonsäure-N-(2-pyridyl)-N-(2-methoxycarbonylethyl)-amid, methanolischer Salzsäure, Methanol und Ammoniumcarbonat.
Ausbeute: 68 % der Theorie,
$C_{25}H_{24}N_6O_3S$ (488.57)
$R_f$-Wert: 0.13 (Kieselgel; Methylenchlorid/Ethanol = 4:1 + einige Tropfen Essigsäure)
EKA-Massenspektrum : $(M+H)^+ = 489$

Beispiel 12

2-[2-(4-Amidinophenyl)ethyl]-benzthiazol-5-yl-carbonsäure-N-(2-pyridyl)-N-(ethoxycarbonylmethyl)-amid-dihydrochlorid

Hergestellt analog Beispiel 9 aus 2-[2-(4-Cyanophenyl)ethyl]-benzthiazol-5-yl-carbonsäure-N-(2-pyridyl)-N-(ethoxycarbonylmethyl)-amid, ethanolischer Salzsäure, Ethanol und Ammoniumcarbonat.
Ausbeute: 95 % der Theorie,
$C_{26}H_{25}N_5O_3S$ (487.58)
$R_f$-Wert: 0.20 (Kieselgel; Methylenchlorid/Ethanol = 4:1 + einige Tropfen Essigsäure)
EKA-Massenspektrum: $(M+H)^+ = 488$

Beispiel 13

2-[N-(4-Amidinophenyl)-aminomethyl]-benzthiazol-5-yl-carbonsäure-N-(2-pyridyl)-N-(ethoxycarbonylmethyl)-amid-dihydrochlorid

Hergestellt analog Beispiel 9 aus 2-[N-(4-Cyanophenyl)-aminomethyl]-benzthiazol-5-yl-carbonsäure-N-(2-pyridyl)-N-(ethoxycarbonylmethyl)-amid, ethanolischer Salzsäure, Ethanol und Ammoniumcarbonat.
Ausbeute: 68 % der Theorie,
$C_{25}H_{24}N_6O_3S$ (488.57)
$R_f$-Wert: 0.14 (Kieselgel; Methylenchlorid/Ethanol = 4:1 + einige Tropfen Essigsäure)
EKA-Massenspektrum: $(M+H)^+$ = 489

Beispiel 14

2-[N-(4-Amidinophenyl)-aminomethyl]-benzthiazol-5-yl-carbonsäure-N-(2-pyridyl)-N-(hydroxycarbonylmethyl)-amid-dihydrochlorid

Hergestellt analog Beispiel 10 aus 2-[N-(4-Amidinophenyl)-aminomethyl] -benzthiazol-5-yl-carbonsäure-N-(2-pyridyl)-N-(ethoxycarbonylmethyl)-amid-dihydrochlorid und Natronlauge.
Ausbeute: 90 % der Theorie,
$C_{23}H_{20}N_6O_3S$ (460.52)
$R_f$-Wert:
EKA-Massenspektrum: $(M+H)^+$ = 461
$(M+Na)^+$ = 483
$(M+2Na)^{++}$ = 253

Beispiel 15

2-[N-(4-Amidinophenyl)-N-methyl-aminomethyl]-benzthiazol-5-ylcarbonsäure-N-phenyl-N-(2-ethoxycarbonylethyl)-amid-hydrochlorid

a) 2-[N-(4-Cyanophenyl)-N-methyl-aminomethyl]-benzthiazol-5-ylcarbonsäure-N-phenyl-N-(2-ethoxycarbonylethyl)-amid

Hergestellt analog Beispiel 9e aus 4-Cyano-N-methyl-anilin und 2-Methoxymethyl-benzthiazol-5-carbonsäure-N-phenyl-N-(2-ethoxycarbonylethyl)-amid.
Ausbeute: 57 % der Theorie,
Rf-Wert: 0,46 (Kieselgel; Dichlormethan/Ethanol = 19:1).

b) 2-[N-(4-Amidinophenyl)-N-methyl-aminomethyl]-benzthiazol-5-yl-carbonsäure-N-phenyl-N-(2-ethoxycarbonylethyl)-amid-hydrochlorid

Hergestellt analog Beispiel 9 aus 2-[N-(4-Cyanophenyl)-N-methyl-aminomethyl]-benzthiazol-5-yl-carbonsäure-N-phenyl-N-(2-ethoxycarbonylethyl)-amid, ethanolischer Salzsäure, Ethanol und Ammoniumcarbonat.
Ausbeute: 73 % der Theorie,
$C_{28}H_{29}N_5O_3S$ (515.64)
$R_f$-Wert:0.29 (Kieselgel; Methylenchlorid/Ethanol = 4:1 + einige Tropfen Essigsäure)
EKA-Massenspektrum: $(M+H)^+$ = 516

Beispiel 16

2-[N-(4-Amidinophenyl)-N-methyl-aminomethyl]-benzthiazol-5-ylcarbonsäure-N-phenyl-N-(2-hydroxycarbonylethyl)-amid-hydrochlorid

Hergestellt analog Beispiel 10 aus 2-[N-(4-Amidinophenyl)-N-methyl-aminomethyl]-benzthiazol-5-yl-carbonsäure-N-phenyl-N-(2-ethoxycarbonylethyl)-amid-hydrochlorid und Natronlauge. Ausbeute: 96 % der Theorie,
$C_{26}H_{25}N_5O_3S$ (487.58)
$R_f$-Wert: 0.48 (Merck RP-8, Methanol/5%ige-NaCl-Lösung = 6:4)

EKA-Massenspektrum: $(M+H)^+$ = 488
$(M+2Na)^{++}$ = 266.5

Beispiel 17

2-[(4-Amidinophenyl)thiomethyl]-benzthiazol-5-yl-carbonsäure-N-phenyl-N-(2-ethoxycarbonylethyl)-amid-hydrochlorid

Hergestellt analog Beispiel 9 aus 2-[(4-Cyanophenyl)thiomethyl]-benzthiazol-5-yl-carbonsäure-N-phenyl-N-(2-ethoxycarbonylethyl)-amid, ethanolischer Salzsäure, Ethanol und Ammoniumcarbonat.
Ausbeute: 61 % der Theorie,
$C_{27}H_{26}N_4O_3S_2$ (518.66)
$R_f$-Wert: 0.27 (Kieselgel; Methylenchlorid/Ethanol = 4:1 + einige Tropfen Essigsäure)
EKA-Massenspektrum: $(M+H)^+$ = 519

Beispiel 18

2-[(4-Amidinophenyl)thiomethyl]-benzthiazol-5-yl-carbonsäure-N-phenyl-N-(2-hydroxycarbonylethyl)-amid-hydrochlorid

Hergestellt analog Beispiel 10 aus 2-[(4-Amidinophenyl)thiomethyl]-benzthiazol-5-yl-carbonsäure-N-phenyl-N-(2-ethoxycarbonylethyl)-amid-hydrochlorid und Natronlauge.
Ausbeute: 95 % der Theorie,
$C_{25}H_{22}N_4O_3S_2$ (490.61)
$R_f$-Wert: 0.25 (Merck RP-8, Methanol/5%ige NaCl-Lösung = 6:4)
EKA-Massenspektrum: $(M+H)^+$ = 491
$(M+Na)^+$ = 513

Beispiel 19

2-[N-(4-Amidinophenyl)-aminomethyl]-benzthiazol-5-yl-carbonsäure-N-phenyl-N-(ethoxycarbonylmethyl)-amid-hydrochlorid

Hergestellt analog Beispiel 9 aus 2-[N-(4-Cyanophenyl)-aminomethyl]-benzthiazol-5-yl-carbonsäure-N-phenyl-N-(ethoxycarbonylmethyl)-amid, ethanolischer Salzsäure, Ethanol und Ammoniumcarbonat.
Ausbeute: 82 % der Theorie,
$C_{26}H_{25}N_5O_3S$ (487.58)
$R_f$-Wert: 0.21 (Kieselgel; Methylenchlorid/Ethanol = 4:1 + einige Tropfen Essigsäure)
EKA-Massenspektrum: $(M+H)^+$ = 488

Beispiel 20

2-[N-(4-Amidinophenyl)-aminomethyl]-benzthiazol-5-yl-carbonsäure-N-phenyl-N-(hydroxycarbonylmethyl)-amid-hydrochlorid

Hergestellt analog Beispiel 10 aus 2-[N-(4-Amidinophenyl)-aminomethyl]-benzthiazol-5-yl-carbonsäure-N-phenyl-N-(ethoxycarbonylmethyl)-amid-hydrochlorid und Natronlauge.
Ausbeute: 75 % der Theorie,
$C_{24}H_{21}N_5O_3S$ (459.53)
$R_f$-Wert: 0.14 (Kieselgel; Methylenchlorid/Ethanol = 4:1 + einige Tropfen Essigsäure)
EKA-Massenspektrum: $(M+H)^+$ = 460
$(M+Na)^+$ = 482

Beispiel 21

2-[2-(4-Amidinophenyl)ethyl]-benzthiazol-5-yl-carbonsäure-N-phenyl-N-(2-ethoxycarbonylethyl)-amid-hydrochlorid

Hergestellt analog Beispiel 9 aus 2-[2-(4-Cyanophenyl)ethyl]-benzthiazol-5-yl-carbonsäure-N-phenyl-N-(2-ethoxycarbonylethyl)-amid, ethanolischer Salzsäure, Ethanol und Ammoniumcarbonat.
Ausbeute: 80 % der Theorie,
$C_{28}H_{28}N_4O_3S$ (500.62)
$R_f$-Wert: 0.30 (Kieselgel; Methylenchlorid/Ethanol = 4:1 + einige Tropfen Essigsäure)
EKA-Massenspektrum: $(M+H)^+$ = 501

Beispiel 22

2-[2-(4-Amidinophenyl)ethyl]-benzthiazol-5-yl-carbonsäure-N-phenyl-N-(2-hydroxycarbonylethyl)-amid-hydrochlorid

Hergestellt analog Beispiel 10 aus 2-[2-(4-Amidinophenyl)-ethyl]-benzthiazol-5-yl-carbonsäure-N-phenyl-N-(2-ethoxycarbonylethyl)-amid-hydrochlorid und Natronlauge.
Ausbeute: 77 % der Theorie,
$C_{26}H_{24}N_4O_3S$ (472.57)
$R_f$-Wert: 0.18 (Kieselgel; Methylenchlorid/Ethanol = 4:1 + einige Tropfen Essigsäure)
EKA-Massenspektrum: $(M+H)^+$ = 473
$(M+Na)^+$ = 495
$(M+H+Na)^{++}$ = 259

Beispiel 23

2-[N-(4-Amidinophenyl)-aminomethyl]-benzthiazol-5-yl-carbonsäure-N-(n-propyl)-N-(2-ethoxycarbonylethyl)-amid-hydrochlorid

Hergestellt analog Beispiel 9 aus 2-[N-(4-Cyanophenyl)-aminomethyl]-benzthiazol-5-yl-carbonsäure-N-(n-propyl)-N-(2-ethoxycarbonylethyl)-amid, ethanolischer Salzsäure, Ethanol und Ammoniumcarbonat.
Ausbeute: 83 % der Theorie,
$C_{24}H_{29}N_5O_3$ (467.59)
$R_f$-Wert: 0.31 (Kieselgel; Methylenchlorid/Ethanol = 4:1 + einige Tropfen Essigsäure)
EKA-Massenspektrum: $(M+H)^+$ = 468
$(2M+H)^+$ = 935

Beispiel 24

2-[N-(4-Amidinophenyl)-aminomethyl]-benzthiazol-5-yl-carbonsäure-N-(n-propyl)-N-(2-hydroxycarbonylethyl)-amid-hydrochlorid

Hergestellt analog Beispiel 10 aus 2-[N-(4-Amidinophenyl)-aminomethyl]-benzthiazol-5-yl-carbonsäure-N-(n-propyl)-N-(2-ethoxycarbonylethyl)-amid-hydrochlorid und Natronlauge.
Ausbeute: 75 % der Theorie,
$C_{22}H_{25}N_5O_3S$ (439.54)
$R_f$-Wert: 0.14 (Kieselgel; Methylenchlorid/Ethanol = 4:1 + einige Tropfen Essigsäure)
EKA-Massenspektrum: $(M+H)^+$ = 440
$(M+H+Na)^{++}$ = 231.6

Beispiel 25

1-Methyl-2-[N-(4-amidinophenyl)-aminomethyl]-benzimidazol-5-yl-carbonsäure-N-phenyl-N-(2-ethoxycarbonylethyl) -amid-hydrochlorid

a) 4-Methylamino-3-nitro-benzoesäure-N-phenyl-N-(2-ethoxycarbonylethyl)-amid

Zu einer Lösung von 24.7 g (0.115 Mol) 4-Methylamino-3-nitrobenzoesäurechlorid und 22.3 g (0.115 Mol) N-(2-Ethoxycarbonylethyl)-anilin in 300 ml Tetrahydrofuran wurden unter Rühren bei Raumtemperatur 13.1 g (0.13 Mol) Triethylamin innerhalb von 15 Minuten zugetropft. Nach 2-stündigem Rühren wurde das Lösungsmittel im Wasserstrahlvakuum abdestilliert und der Rückstand unter Rühren mit 700 ml Wasser versetzt. Das Gemisch wurde dreimal mit je 200 ml Dichlormethan extrahiert, der organische Extrakt mit 200 ml 2N Salzsäure und zweimal mit je 300 ml Wasser gewaschen und über Natriumsulfat getrocknet. Das Lösungsmittel wurde dann abdestilliert und das so erhaltene ölige Produkt durch Säulenchromatographie (1 kg Kieselgel; Laufmittel: Petrolether/Essigester = 2:1) gereinigt.
Ausbeute: 35.0 g (82 % der Theorie),
$R_f$-Wert: 0.28 (Kieselgel; Dichlormethan/Ethanol = 50:1)

b) 3-Amino-4-methylamino-benzoesäure-N-phenyl-N-(2-ethoxycarbonylethyl)-amid

12.1 g (0.0326 Mol) 4-Methylamino-3-nitro-benzoesäure-N-phenyl-N-(2-ethoxycarbonylethyl)-amid wurden in 300 ml Ethanol und 150 ml Dichlormethan nach Zugabe von ca. 4 g Palladium/Kohle (10%ig) bei Raumtemperatur und einem Wasserstoffdruck von 5 bar hydriert. Danach wurde vom Katalysator abfiltriert und das Filtrat eingeengt. Das so erhaltene Rohprodukt wurde ohne weitere Reinigung umgesetzt.
Ausbeute: 10.6 g (95 % der Theorie),
$R_f$-Wert: 0.19 (Kieselgel; Dichlormethan/Ethanol = 50:1)

c) 1-Methyl-2-[N-(4-cyanophenyl)-aminomethyl]-benzimidazol-5-yl-carbonsäure-N-phenyl-N-(2-ethoxycarbonylethyl)-amid

6.17 g (0.035 Mol) N-(4-Cyanophenyl)glycin und 5.68 g (0.035 Mol) N,N'-Carbonyldiimidazol wurden in 300 ml Tetrahydrofuran 30 Minuten lang zum Rückfluß erhitzt, dann 10.6 g (0.032 Mol) 3-Amino-4-methylamino-benzoesäure-N-phenyl-N-(2-ethoxycarbonylethyl)-amid hinzugefügt und weitere fünf Stunden lang zum Rückfluß erhitzt. Dann wurde das Lösungsmittel im Vakuum abdestilliert, der Rückstand in 150 ml Eisessig gelöst und eine Stunde lang zum Rückfluß erhitzt. Anschließend wurde der Eisessig im Vakuum abdestilliert, der Rückstand in ca. 300 ml Dichlormethan gelöst, die Lösung zweimal mit je ca. 150 ml Wasser gewaschen und anschließend über Natriumsulfat getrocknet. Nach Abdampfen des Lösungsmittels wurde das so erhaltene Rohprodukt durch Säulenchromatographie (800 g Kieselgel; Laufmittel: Dichlormethan mit 1-2 % Ethanol) gereinigt.
Ausbeute: 8.5 g (57 % der Theorie),
$R_f$-Wert: 0.51 (Kieselgel; Dichlormethan/Ethanol = 19:1)

d) l-Methyl-2-[N-(4-amidinophenyl)-aminomethyl]-benzimidazol-5-yl-carbonsäure-N-phenyl-N-(2-ethoxycarbonylethyl)-amid-hydrochlorid

1.2 g (2.49 mMol) 1-Methyl-2-[N-(4-cyanophenyl)-aminomethyl]-benzimidazol-5-yl-carbonsäure-N-phenyl-N-(2-ethoxycarbonylethyl)-amid wurden in 100 ml gesättigter ethanolischer Salzsäure 6 Stunden lang bei Raumtemperatur gerührt. Anschließend wurde im Vakuum bis zur Trockne eingeengt, der Rückstand in 100 ml Ethanol gelöst, mit 2.5 g (26 mMol) Ammoniumcarbonat versetzt und über Nacht bei Raumtemperatur gerührt. Nach Abdestillieren des Lösungsmittels wurde das so erhaltene Rohprodukt durch Säulenchromatographie (100 g Kieselgel; Laufmittel: Dichlormethan/Ethanol = 4:1) gereinigt. Beim Einengen der Eluate erhielt man die gewünschte Verbindung als weißen, amorphen Feststoff.
Ausbeute: 1.10 g (83 % der Theorie),
$R_f$-Wert: 0.18 (Kieselgel; Dichlormethan/Ethanol = 4:1)
$C_{28}H_{30}N_6O_3$ x HCl (498.6)
EKA-Massenspektrum: $(M+H)^+$ = 499
$(M+2H)^{++}$ = 250
$(M+H+Na)^{++}$ = 261

Beispiel 26

1-Methyl-2-[N-(4-amidinophenyl)-aminomethyl]-benzimidazol-5-yl-carbonsäure-N-phenyl-N-(2-hydroxycarbonylethyl)-amid

Eine Mischung aus 300 mg (0.56 mMol) 1-Methyl-2-[N-(4-amidinophenyl)-aminomethyl]-benzimidazol-5-yl-carbonsäure-N-phenyl-N-(2-ethoxycarbonylethyl)-amid-hydrochlorid, 15 ml Ethanol, 4 ml Wasser und 120 mg (3.0 mMol) Natriumhydroxid wurde zwei Stunden lang bei Raumtemperatur gerührt. Anschließend wurde mit ca. 20 ml Wasser verdünnt und mit Eisessig schwach sauer gestellt. Das dabei auskristallisierte Produkt wurde abgesaugt, mit Wasser gewaschen und bei 60°C im Vakuum getrocknet. Ausbeute: 250 mg (95 % der Theorie),
$C_{26}H_{26}N_6O_3$ (470.5)
EKA-Massenspektrum: $(M+H)^+$ = 471
$(M+H+Na)^{++}$ = 247
$(M+2Na)^{++}$ = 258

Beispiel 27

1-Methyl-2-[(4-amidinophenyl)thiomethyl]-benzimidazol-5-yl-carbonsäure-N-(n-propyl)-N-(2-ethoxycarbonylethyl)-amid-hydrochlorid

a) 4-Methylamino-3-chloracetamido-benzoesäure-N-(n-propyl)-N-(2-ethoxycarbonylethyl)-amid

Eine Lösung aus 1.8 g (5.9 mMol) 3-Amino-4-methylamino-benzoesäure-N-(n-propyl)-N-(2-ethoxycarbonylethyl)-amid [Die Herstellung erfolgt analog zu 3-Amino-4-ethylamino-benzoesäure-N-phenyl-N-(2-ethoxycarbonylethyl)-amid], 1.1g (6.8 mMol) N,N'-Carbonyldiimidazol und 0.65 g (6.9 mMol) Chloressigsäure in 75 ml Tetrahydrofuran wurde 1 Stunde bei Raumtemperatur gerührt. Anschließend wurde das Lösungsmittel im Vakuum abdestilliert, und das Rohprodukt wurde durch Flash-Chromatographie (Kieselgel; Methylenchlorid/Ethanol = 49:1) gereinigt.
Ausbeute: 1.7 g (77% der Theorie) gelbes Öl,
$R_f$-Wert: 0.58 (Kieselgel; Essigester/Ethanol/Ammoniak = 90:10:1)

b) 2-Chlormethyl-1-methyl-benzimidazol-5-yl-carbonsäure-N-(n-propyl)-N-(2-ethoxycarbonylethyl)-amid

1.6 g (4.3 mMol) 4-Methylamino-3-chloracetamido-benzoesäure-N-(n-propyl)-N-(2-ethoxycarbonylethyl)-amid wurden in 25 ml Essigsäure 30 Minuten auf 100°C erhitzt. Anschließend wurde das Lösungsmittel abdestilliert, das Rohprodukt wurde in 40 ml Methylenchlorid/Ethanol (9:1) aufgenommen und mit 20 ml gesättigter Natriumhydrogencarbonat-Lösung gewaschen. Die organische Phase wurde mit Natriumsulfat getrocknet und eingeengt. Ausbeute: 1.5 g (100% der Theorie) braunes Öl,
$R_f$-Wert: 0.63 (Kieselgel;Essigester/Ethanol/Ammoniak = 90:10:1)

c) 1-Methyl-2-[(4-cyanophenyl)thiomethyl]-benzimidazol-5-yl-carbonsäure-N-(n-propyl)-N-(2-ethoxycarbonylethyl)-amid

Ein Gemisch aus 1.5 g (4.1 mMol) 2-Chlormethyl-1-methyl-benzimidazol-5-yl-carbonsäure-N-(n-propyl)-N-(2-ethoxycarbonylethyl)-amid und 0.65 g (4.8 mMol) p-Cyanothiophenol wurde in 10 ml Dimethylformamid und 10 ml Diisopropylethylamin 1 Stunde lang auf 100°C erhitzt. Das Lösungsmittel wurde im Vakuum abdestilliert, das Rohprodukt wurde in 30 ml Essigester gelöst, mit 30 ml Wasser gewaschen, und nach Aufkonzentrierung durch Flash-Chromatographie (Kieselgel; Methylenchlorid/Ethanol (49:1 bis 19:1) gereinigt.
Ausbeute: 1.5 g (79% der Theorie) braunes Öl,
$R_f$-Wert: 0.65 (Kieselgel; Essigester/Ethanol/Ammoniak = 90:10:1)

d) 1-Methyl-2-[(4-amidinophenyl)thiomethyl]-benzimidazol-5-yl-carbonsäure-N-(n-propyl)-N-(2-ethoxycarbonylethyl)-amid-hydrochlorid

1.4 g (3.01 mMol) 1-Methyl-2-[(4-cyanophenyl)thiomethyl]-benzimidazol-5-yl-carbonsäure-N-(n-propyl)-N-(2-ethoxycarbonylethyl)-amid wurden in 50 ml mit Chlorwasserstoff gesättigtem Ethanol 5 Stunden erst bei 0°C, später bei Raumtemperatur gerührt, bis dünnschichtchromatographisch kein Ausgangsmaterial mehr nachweisbar war. Anschließend wurde das Lösungsmittel bei maximal 30°C Badtemperatur abdestilliert, der ölige Rückstand in 40 ml absolutem Ethanol aufgenommen und mit 2.8 g Ammoniumcarbonat versetzt. Nach 18 Stunden wurde das Lösungsmittel im Vakuum abdestilliert und das Rohprodukt durch Flash-Chromatographie (Kieselgel; Methylenchlorid/Ethanol = 19:1 bis 4:1) gereinigt.
Ausbeute: 1.3 g (83% der Theorie) als hellbeiger Feststoff,
$R_f$-Wert: 0.29 (Kieselgel; Essigester/Ethanol/Ammoniak = 50:45:5)
$C_{25}H_{31}N_6O_3S$ (481.62)
EKA-Massenspektrum: $(M+H)^+$ = 482

Beispiel 28

1-Methyl-2-[(4-amidinophenyl)thiomethyl]-benzimidazol-5-yl-carbonsäure-N-(n-propyl)-N-(2-hydroxycarbonylethyl)-amid-hydrochlorid

0.52 g (1.0 mMol) 1-Methyl-2-[(4-amidinophenyl)thiomethyl]-benzimidazol-5-yl-carbonsäure-N-(n-propyl)-N-(2-ethoxycarbonylethyl)-amid-hydrochlorid wurden in 15 ml Ethanol gelöst, mit 5 ml 2N Natronlauge versetzt und 2 Stunden bei Raumtemperatur gerührt. Anschließend wurden 5 ml Wasser zugegeben, der Alkohol wurde abdestilliert,

und es wurde mit konzentrierter Salzsäure angesäuert. Das Wasser wurde im Vakuum abdestilliert, und das Rohprodukt wurde in 5 ml Ethanol aufgenommen und vom unlöslichen Natriumchlorid abfiltriert. Nach dem Abdestillieren des Lösungsmittels fiel die Titelverbindung als weißer Feststoff an.
Ausbeute: 0.43 g (88% der Theorie),
$R_f$-Wert: 0.19 (Kieselgel; Essigester/Ethanol/Ammoniak = 50:45:5)
$C_{23}H_{27}N_5O_3S$ (453.57)
EKA-Massenspektrum: $(M+H)^+ = 454$
$(M+Na)^+ = 476$

Beispiel 29

1-Methyl-2-[(4-amidinophenyl)thiomethyl]-benzimidazol-5-yl-carbonsäure-N-(2-methylpropyl)-N-(2-ethoxycarbonylethyl)-amid-hydrochlorid

Hergestellt analog Beispiel 27 aus 1-Methyl-2-[(4-cyanophenyl)thiomethyl]-benzimidazol-5-yl-carbonsäure-(N-(2-methylpropyl-N-(2-ethoxycarbonylethyl)-amid, ethanolischer Salzsäure, Ethanol und Ammoniumcarbonat.
Ausbeute: 83 % der Theorie,
$C_{25}H_{31}N_6O_3S$ (495.65)
$R_f$-Wert: 0.30 (Kieselgel; Essigester/Ethanol/Ammoniak = 50:45:5)
EKA-Massenspektrum: $(M+H)^+ = 496$

Beispiel 30

1-Methyl-2-[(4-amidinophenyl)thiomethyl]-benzimidazol-5-yl-carbonsäure-N-phenyl-N-(2-ethoxycarbonylethyl)-amid-hydrochlorid

Hergestellt analog Beispiel 27 aus 1-Methyl-2-[(4-cyanophenyl)-thiomethyl]- benzimidazol-5-yl-carbonsäure-N-phenyl-N-(2-ethoxycarbonylethyl)-amid, und ethanolischer Salzsäure, Ethanol und Ammoniumcarbonat.
Ausbeute: 90 % der Theorie,
$C_{28}H_{29}N_5O_3S$ (515.64)
$R_f$-Wert:0.24 (Kieselgel; Essigester/Ethanol/Ammoniak = 50:45:5)
EKA-Massenspektrum: $(M+H)^+ = 516$
$(M+H+Na)^{++} = 269.7$

Beispiel 31

1-Methyl-2-[(4-amidinophenyl)thiomethyl]-benzimidazol-5-yl-carbonsäure-N-phenyl-N-(2-hydroxycarbonylethyl)-amid-hydrochlorid

Hergestellt analog Beispiel 28 aus 1-Methyl-2-[(4-amidinophenyl) thiomethyl-benzimidazol-5-yl-carbonsäure-N-phenyl-N-(2-ethoxycarbonylethyl)-amid-hydrochlorid und Natronlauge.
Ausbeute: 76 % der Theorie,
$C_{26}H_{25}N_5O_3S$ (487.58)
$R_f$-Wert: 0.31 (Kieselgel; Essigester/Ethanol/Ammoniak = 50:45:5)
EKA-Massenspektrum: $(M+H)^+ = 488$
$(M+Na)^+ = 510$

Beispiel 32

1-Methyl-2-[(4-amidinophenyl)oxymethyl]-benzimidazol-5-yl-sulfonsäure-N-(1-methyl-piperidin-4-yl)-N-methyl-amid-hydrochlorid

a) 4-Chlor-3-nitrobenzolsulfonsäure-N-(1-methyl-piperidin-4-yl)-N-methyl-amid

Zu einer Lösung von 2.2 ml (15 mMol) 1-Methyl-4-methylamino-piperidin in 60 ml Pyridin wurden unter Eiskühlung 3.8 g (15 mMol) 4-Chlor-3-nitro-benzolsulfonsäurechlorid portionsweise zugegeben. Danach wurde noch zwei Stunden lang unter Kühlung gerührt, anschließend zur Trockne eingedampft, der Rückstand mit ca. 50 ml Wasser versetzt und unter heftigem Rühren mit konzentriertem Ammoniak alkalisch gestellt. Das ausgefallene Rohprodukt

wurde abgesaugt und durch Säulenchromatographie (250 g Kieselgel, Laufmittel: Dichlormethan mit 1.5% Ethanol) gereinigt.
Ausbeute: 1.6 g (31% der Theorie),
$C_{13}H_{18}ClN_3O_4S$ (347.8)
Rf-Wert: 0.19 (Kieselgel; Dichlormethan/Ethanol = 19:1)

b) 4-Methylamino-3-nitrobenzolsulfonsäure-N-methyl-N-(1-methylpiperidin-4-yl)-amid

1.6 g (4.6 mMol) 4-Chlor-3-nitrobenzolsulfonsäure-N-methyl-N-(1-methyl-piperidin-4-yl)-amid wurden mit 30 ml 40%iger Methylaminlösung versetzt und im geschlossenen Kolben vier Stunden lang bei Raumtemperatur gerührt. Dann wurde mit ca. 40 ml Wasser verdünnt, das ausgefallene Produkt abgesaugt, mit Wasser gewaschen und getrocknet.
Ausbeute: 1.5 g (95% der Theorie),
$C_{14}H_{22}N_4O_4S$ (343.4)
$R_f$-Wert: 0.45 (Kieselgel; Dichlormethan/Ethanol = 4:1)

c) 3-Amino-4-methylaminobenzolsulfonsäure-N-methyl-N-(1-methyl-piperidin-4-yl)-amid

1.5 g (4.4 mMol) 4-Methylamino-3-nitrobenzolsulfonsäure-N-methyl-N-(1-methyl-piperidin-4-yl)-amid wurden in 100 ml Methanol gelöst und bei Raumtemperatur und 5 bar Wasserstoffdruck katalytisch hydriert (10% Palladium auf Kohle). Dann wurde der Katalysator abfiltriert und das Filtrat eingeengt. Das so erhaltene ölige Produkt wurde ohne Reinigung weiter umgesetzt.
Ausbeute: 1.4 g (100% der Theorie),
$C_{14}H_{24}N_4O_2S$ (312.4)
$R_f$-Wert: 0.33 (Kieselgel; Dichlormethan/Ethanol = 4:1)

d) 1-Methyl-2-[(4-cyanophenyl)oxymethyl]-benzimidazol-5-yl-sulfonsäure-N-methyl-N-(1-methyl-piperidin-4-yl)-amid

532 mg (3.0 mMol) 4-Cyanophenyloxyessigsäure und 486 mg (3.0 mMol) 1,1'-Carbonyldiimidazol wurden in 40 ml Tetrahydrofuran gelöst und 15 Minuten lang zum Rückfluß erhitzt. Dann wurden 700 mg (2.24 mMol) 3-Amino-4-methylaminobenzolsulfonsäure-N-methyl-N-(1-methyl-piperidin-4-yl)-amid hinzugefügt und weitere acht Stunden gekocht. Danach wurde eingedampft und der so erhaltene ölige Rückstand in 30 ml Eisessig eine Stunde lang zum Rückfluß erhitzt. Der Eisessig wurde abdestilliert, der Rückstand mit ca. 30 ml Wasser versetzt und mit konzentriertem Ammoniak alkalisch gestellt, und die Lösung dreimal mit je ca. 20 ml Dichlormethan extrahiert. Die organischen Phasen wurden getrocknet und eingeengt. Das so erhaltene Produkt wurde ohne Reinigung weiter umgesetzt.
Ausbeute: 400 mg (39% der Theorie),
$C_{23}H_{27}N_5O_3S$ (453.6)
$R_f$-Wert: 0.37 (Kieselgel; Dichlormethan/Ethanol = 4:1)

e) 1-Methyl-2-[(4-amidinophenyl)oxymethyl]-benzimidazol-5-yl-sulfonsäure-N-methyl-N-(1-methylpiperidin-4-yl)-amid-hydrochlorid

Hergestellt analog Beispiel 25d aus 400 mg 1-Methyl-2-[(4-cyanophenyl) oxymethyl] -benzimidazol-5-yl-sulfonsäure-N-methyl-N-(1-methylpiperidin-4-yl)-amid mit ethanolischer Salzsäure und Ammoniumcarbonat.
Ausbeute: 370 mg (83% der Theorie),
$C_{23}H_{30}N_6O_3S$ (470.6)
EKA-Massenspektrum: $(M+H)^+$ = 471
$(M+2H)^{++}$ = 236

Beispiel 33

1-Methyl-2-[(4-amidinophenyl)oxymethyl]-benzimidazol-5-yl-sulfonsäure-N-methyl-N-phenyl-amid-hydrochlorid

Hergestellt analog Beispiel 32 aus 1-Methyl-2-[(4-cyanophenyl)-oxymethyl]-benzimidazol-5-yl-sulfonsäure-N-methyl-N-phenyl-amid und ethanolischer Salzsäure, Ethanol und Ammoniumcarbonat.
Ausbeute: 46 % der Theorie,
$C_{23}H_{23}N_5O_3S$ (449.5)
EKA-Massenspektrum: $(M+H)^+$ = 450

(M+H+Methanol)$^+$ = 482
(M+2H)$^{++}$ = 223

Beispiel 34

1-Methyl-2-[(4-amidinophenyl)oxymethyl]-benzimidazol-5-yl-sulfonsäure-N-(3-ethoxycarbonyl-n-propyl)-N-phenyl-amid-hydrochlorid

Hergestellt analog Beispiel 32 aus 1-Methyl-2-[(4-cyanophenyl)-oxymethyl]-benzimidazol-5-yl-sulfonsäure-N- (3-ethoxycarbonyl-n-propyl)-N-phenyl-amid, ethanolischer Salzsäure, Ethanol und Ammoniumcarbonat.
Ausbeute: 57 % der Theorie,
$C_{28}H_{31}N_5O_5S$ (549.7)
EKA-Massenspektrum: (M+H)$^+$ = 550

Beispiel 35

1-Methyl-2-[(3-amidinophenyl)oxymethyl]-benzimidazol-5-yl-sulfonsäure-pyrrolidid-hydrochlorid

Hergestellt analog Beispiel 32 aus 1-Methyl-2-[(3-cyanophenyl)-oxymethyl] -benzimidazol-5-yl-sulfonsäure-pyrrolidid, ethanolischer Salzsäure, Ethanol und Ammoniumcarbonat.
Ausbeute: 71 % der Theorie,
$C_{20}H_{23}N_5O_3S$ (413.5)
EKA-Massenspektrum: (M+H)$^+$ = 414

Beispiel 36

1-Methyl-2-[2-(4-amidinophenyl)ethyl]-benzimidazol-5-yl-carbonsäure-N-phenyl-N-(3-methoxycarbonylpropyl)-amid-dihydrochlorid

Hergestellt analog Beispiel 25d aus 1-Methyl-2-[2-(4-cyanophenyl)ethyl]-benzimidazol-5-yl-carbonsäure-N-phenyl-N-(3-tert.butyloxycarbonylpropyl)-amid und methanolischer Salzsäure, Methanol und Ammoniumcarbonat.
Ausbeute: 83.5 % der Theorie,
$R_f$-Wert: 0.17 (Kieselgel; Dichlormethan/Ethanol = 4:1)
$C_{29}H_{31}N_5O_3$ (497.6)
EKA-Massenspektrum: (M+H)$^+$ = 498
(M+H+Na)$^{++}$ = 260.7

Beispiel 37

1-Methyl-2-[2-(4-amidinophenyl)ethyl]-benzimidazol-5-yl-carbonsäure-N-phenyl-N-(3-hydroxycarbonylpropyl)-amid-hydrochlorid

Hergestellt analog Beispiel 26 aus 1-Methyl-2-[(4-amidinophenyl)aminomethyl]-benzimidazol-5-yl-carbonsäure-N-phenyl-N-(3-methoxycarbonylpropyl)-amid-dihydrochlorid und Natronlauge.
Ausbeute: 92 % der Theorie,
$R_f$-Wert: 0.09 (Kieselgel; Dichlormethan/Ethanol = 4:1)
$C_{28}H_{29}N_5O_3$ (483.6)
EKA-Massenspektrum: (M+H)$^+$ = 484
(M+Na)$^+$ = 506
(M+H+Na)$^{++}$ = 253.7

Beispiel 38

1-Methyl-2-[N-(4-amidinophenyl)-aminomethyl]-benzimidazol-5-yl-carbonsäure-N-phenyl-N-(3-ethoxycarbonylpropyl)-amid-dihydrochlorid

a) 1-Methyl-2-[N-(4-cyanophenyl)-aminomethyl]-benzimidazol-5-yl-carbonsäure-N-phenyl-N-(3-tert.butyloxycarbonylpropyl)-amid

Hergestellt analog Beispiel 25c aus N-(4-Cyanophenyl)-glycin und 3-Amino-4-methylamino-benzoesäure-N-phenyl-N-(3-tert.butyloxycarbonylpropyl)-amid.
Ausbeute: 65 % der Theorie,
$R_f$-Wert: 0.17 (Kieselgel; Dichlormethan/Methanol = 19:1)

b) 1-Methyl-2-[N-(4-amidinophenyl)-aminomethyl]-benzimidazol-5-yl-carbonsäure-N-phenyl-N-(3-ethoxycarbonylpropyl)-amid-dihydrochlorid

Hergestellt analog Beispiel 25d aus 1-Methyl-2-[N-(4-cyanophenyl)-aminomethyl]-benzimidazol-5-yl-carbonsäure-N-phenyl-N-(3-tert.butyloxycarbonylpropyl)-amid und ethanolischer Salzsäure, Ethanol und Ammoniumcarbonat.
Ausbeute: 68 % der Theorie,
$R_f$-Wert: 0.12 (Kieselgel; Dichlormethan/Ethanol = 4:1)
$C_{29}H_{32}N_6O_3$ (512.6)
EKA-Massenspektrum: $(M+H)^+$ = 513
$(M+H+Na)^{++}$ = 268

Beispiel 39

1-Methyl-2-[N-(4-amidinophenyl)-aminomethyl]-benzimidazol-5-yl-carbonsäure-N-phenyl-N-(3-hydroxycarbonylpropyl)-amid-hydrochlorid

Hergestellt analog Beispiel 26 aus 1-Methyl-2-[N-(4-amidinophenyl)-aminomethyl]-benzimidazol-5-yl-carbonsäure-N-phenyl-N-(3-ethoxycarbonylpropyl)-amid-dihydrochlorid und Natronlauge.
Ausbeute: 73.5 % der Theorie,
$C_{27}H_{28}N_6O_3$ (484.6)
EKA-Massenspektrum: $(M+H)^+$ = 485
$(M+2H)^{++}$ = 243
$(M+H+Na)^{++}$ = 254

Beispiel 40

1-Methyl-2-[2-(4-amidinophenyl)ethyl]-benzimidazol-5-yl-carbonsäure-N-phenyl-N-(ethoxycarbonylmethyl)-amid-hydrochlorid

Hergestellt analog Beispiel 25d aus 1-Methyl-2-[2-(4-cyanophenyl)ethyl]-benzimidazol-5-yl-carbonsäure-N-phenyl-N-(ethoxycarbonylmethyl)-amid und ethanolischer Salzsäure, Ethanol und Ammoniumcarbonat.
Ausbeute: 73 % der Theorie,
$R_f$-Wert: 0.15 (Kieselgel; Dichlormethan/Ethanol = 4:1)
$C_{28}H_{29}N_5O_3$ (483.6)
EKA-Massenspektrum: $(M+H)^+$ = 484
$(M+H+Na)^{++}$ = 253.7

Beispiel 41

1-Methyl-2-[2-(4-amidinophenyl)ethyl]-benzimidazol-5-yl-carbonsäure-N-phenyl-N-(hydroxycarbonylmethyl)-amid-hydrochlorid

Hergestellt analog Beispiel 26 aus 1-Methyl-2-[2-(4-amidinophenyl)ethyl]benzimidazol-5-yl-carbonsäure-N-phenyl-N-(ethoxycarbonylmethyl)-amid-hydrochlorid und Natronlauge.

Ausbeute: 97 % der Theorie,
$C_{26}H_{25}N_5O_3$ (455.5)
EKA-Massenspektrum: $(M+H)^+ = 456$
$(M+Na)^+ = 478$
$(M+2Na)^{++} = 250.6$

Beispiel 42

1-Methyl-2-[(4-amidinophenyl)oxymethyl]-benzimidazol-5-yl-carbonsäure-N-phenyl-N-(ethoxycarbonylmethyl)-amid-hydrochlorid

Hergestellt analog Beispiel 25d aus 1-Methyl-2-[(4-cyanophenyl)oxymethyl]-benzimidazol-5-yl-carbonsäure-N-phenyl-N-(ethoxycarbonylmethyl)-amid und ethanolischer Salzsäure, Ethanol und Ammoniumcarbonat.
Ausbeute: 76 % der Theorie,
$R_f$-Wert: 0.17 (Kieselgel; Dichlormethan/Ethanol = 4:1)
$C_{27}H_{27}N_5O_4$ (485.6)
EKA-Massenspektrum: $(M+H)^+ = 486$
$(M+H+Na)^{++} = 254.7$

Beispiel 43

1-Methyl-2-[(4-amidinophenyl)oxymethyl]-benzimidazol-5-yl-carbonsäure-N-phenyl-N-(hydroxycarbonylmethyl) -amid-hydrochlorid

Hergestellt analog Beispiel 26 aus 1-Methyl-2-[(4-amidinophenyl)oxymethyl]-benzimidazol-5-yl-carbonsäure-N-phenyl-N-(ethoxycarbonylmethyl)-amid-hydrochlorid und Natronlauge.
Ausbeute: 58 % der Theorie,
$C_{25}H_{23}N_5O_4$ (457.5)
EKA-Massenspektrum: $(M+H)^+ = 458$
$(M+Na)^+ = 480$
$(M+2Na)^{++} = 251.6$

Beispiel 44

1-Methyl-2-[N-(4-amidinophenyl)-aminomethyl]-benzimidazol-5-yl-carbonsäure-N-phenyl-N-(ethoxycarbonylmethyl)-amid-hydrochlorid

Hergestellt analog Beispiel 25d aus 1-Methyl-2-[N-(4-cyanophenyl)-aminomethyl]-benzimidazol-5-yl-carbonsäure-N-phenyl-N-(ethoxycarbonylmethyl)-amid und ethanolischer Salzsäure, Ethanol und Ammoniumcarbonat.
Ausbeute: 74 % der Theorie,
$R_f$-Wert: 0.12 (Kieselgel; Dichlormethan/Ethanol = 4:1)
$C_{27}H_{28}N_6O_3$ (484.6)
EKA-Massenspektrum: $(M+H)^+ = 485$
$(M+H+Na)^{++} = 254$

Beispiel 45

1-Methyl-2-[N-(4-amidinophenyl)-aminomethyl]-benzimidazol-5-yl-carbonsäure-N-phenyl-N-(hydroxycarbonylmethyl)-amid-hydrochlorid

Hergestellt analog Beispiel 26 aus 1-Methyl-2-[N-(4-amidinophenyl)-aminomethyl]-benzimidazol-5-yl-carbonsäure-N-phenyl-N-(ethoxycarbonylmethyl)-amid-hydrochlorid und Natronlauge.
Ausbeute: 84 % der Theorie,
$C_{25}H_{24}N_6O_3$ (456.5)
EKA-Massenspektrum: $(M+H)^+ = 457$
$(M+Na)^+ = 479$
$(M+2Na)^{++} = 251$

Beispiel 46

1-Methyl-2-[(4-amidinophenyl)oxymethyl]-benzimidazol-5-yl-carbonsäure-N-(4-pyrimidyl)-N-(2-ethoxycarbonylethyl) -amid-hydrochlorid

Hergestellt analog Beispiel 25d aus 1-Methyl-2-[(4-cyanophenyl)oxymethyl]-benzimidazol-5-yl-carbonsäure-N-(4-pyrimidyl)-N-(2-ethoxycarbonylethyl)-amid und ethanolischer Salzsäure, Ethanol und Ammoniumcarbonat.
Ausbeute: 14 % der Theorie,
$C_{26}H_{27}N_7O_4$ (501.6)
Massenspektrum: $(M+H)^+ = 502$

Beispiel 47

1-Methyl-2-[(4-amidinophenyl)oxymethyl]-benzimidazol-5-yl-carbonsäure-N-(2-pyridyl)-N-(ethoxycarbonylmethyl)-amid-dihydrochlorid

Hergestellt analog Beispiel 25d aus 1-Methyl-2-[(4-cyanophenyl)oxymethyl]-benzimidazol-5-yl-carbonsäure-N-(2-pyridyl)-N-(ethoxycarbonylmethyl)-amid und ethanolischer Salzsäure, Ethanol und Ammoniumcarbonat.
Ausbeute: 44 % der Theorie,
$R_f$-Wert: 0.12 (Kieselgel; Dichlormethan/Ethanol = 4:1)
$C_{26}H_{26}N_6O_4$ (486.5)
EKA-Massenspektrum: $(M+H)^+ = 487$
$(M+2H)^{++} = 244$
$(M+H+Na)^{++} = 255$

Beispiel 48

1-Methyl-2-[(4-amidinophenyl)oxymethyl]-benzimidazol-5-yl-carbonsäure-N-(2-pyridyl)-N-(hydroxycarbonylmethyl)-amid-hydrochlorid

Hergestellt analog Beispiel 26 aus 1-Methyl-2-[(4-amidinophenyl)oxymethyl]-benzimidazol-5-yl-carbonsäure-N-(2-pyridyl)-N-(ethoxycarbonylmethyl)-amid-dihydrochlorid und Natronlauge.
Ausbeute: 85 % der Theorie,
$C_{24}H_{22}N_6O_4$ (458.5)
EKA-Massenspektrum: $(M+H)^+ = 459$
$(M+Na)^+ = 481$
$(M+2Na)^{++} = 252$

Beispiel 49

1-Methyl-2-[N-(4-amidinophenyl)-aminomethyl]-benzimidazol-5-ylcarbonsäure-N-(2-pyridyl)-N-(ethoxycarbonylmethyl)-amid-dihydrochlorid

a) 1-Methyl-2-[N-(4-cyanophenyl)-aminomethyl]-benzimidazol-5-yl-carbonsäure-N-(2-pyridyl)-N-ethoxycarbonylmethyl-amid

Hergestellt analog Beispiel 25c aus N-(4-Cyanophenyl)-glycin und 3-Amino-4-methylamino-benzoesäure-N-(2-pyridyl)-N-ethoxycarbonylmethyl-amid.
Ausbeute: 24 % der Theorie,
$R_f$-Wert: 0.56 (Kieselgel; Dichlormethan/Methanol = 4:1)

b)1-Methyl-2-[N-(4-amidinophenyl)-aminomethyl]-benzimidazol-5-yl-carbonsäure-N-(2-pyridyl)-N-(ethoxycarbonylmethyl)-amid-dihydrochlorid

Hergestellt analog Beispiel 25d aus 1-Methyl-2-[N-(4-cyanophenyl)-aminomethyl]-benzimidazol-5-yl-carbonsäure-N-(2-pyridyl)-N-(ethoxycarbonylmethyl)-amid und ethanolischer Salzsäure, Ethanol und Ammoniumcarbonat.
Ausbeute: 70 % der Theorie,
$R_f$-Wert: 0.16 (Kieselgel; Dichlormethan/Ethanol = 4:1)

$C_{26}H_{27}N_7O_3$ (485.6)
EKA-Massenspektrum: $(M+H)^+ = 486$
$(M+2H)^{++} = 243.7$
$(M+H-Na)^{++} = 254.6$

Beispiel 50

1-Methyl-2-[N-(4-amidinophenyl)-aminomethyl]-benzimidazol-5-ylcarbonsäure-N-(2-pyridyl)-N-(hydroxycarbonylmethyl)-amid-hydrochlorid

Hergestellt analog Beispiel 26 aus 1-Methyl-2-[N-(4-amidinophenyl)-aminomethyl]-benzimidazol-5-yl-carbonsäure-N-(2-pyridyl)-N-(ethoxycarbonylmethyl)-amid-dihydrochlorid und Natronlauge.
Ausbeute: 91 % der Theorie,
$C_{24}H_{23}N_7O_3$ (457.5)
EKA-Massenspektrum: $(M+H)^+ = 458$
$(M+Na)^+ = 480$
$(M+2Na)^{++} = 251.7$

Beispiel 51

1-Methyl-2-[2-(4-amidinophenyl)ethyl]-benzimidazol-5-yl-carbonsäure-N-(2-pyridyl)-N-(ethoxycarbonylmethyl)-amid-dihydrochlorid

Hergestellt analog Beispiel 25d aus 1-Methyl-2-[2-(4-cyanophenyl)ethyl]-benzimidazol-5-yl-carbonsäure-N-(2-pyridyl)-N-(ethoxycarbonylmethyl)-amid, ethanolischer Salzsäure, Ethanol und Ammoniumcarbonat.
Ausbeute: 90 % der Theorie,
$R_f$-Wert: 0.17 (Kieselgel; Dichlormethan/Ethanol = 4:1)
$C_{27}H_{28}N_6O_3$ (484.6)
EKA-Massenspektrum: $(M+H)^+ = 485$
$(M+2H)^{++} = 243$
$(M+H+Na)^{++} = 254$

Beispiel 52

1-Methyl-2-[2-(4-amidinophenyl)ethyl]-benzimidazol-5-yl-carbonsäure-N-(2-pyridyl)-N-(hydroxycarbonylmethyl)-amid-hydrochlorid

Hergestellt analog Beispiel 26 aus 1-Methyl-2-[2-(4-amidinophenyl)ethyl]-benzimidazol-5-yl-carbonsäure-N-(2-pyridyl)-N-(ethoxycarbonylmethyl)-amid-dihydrochlorid und Natronlauge.
Ausbeute: 89 % der Theorie,
$C_{25}H_{24}N_6O_3$ (456.5)
EKA-Massenspektrum: $(M+H)^+ = 457$
$(M+Na)^+ = 479$

Beispiel 53

1-Methyl-2-[N-(4-amidinophenyl)-aminomethyl]-benzimidazol-5-yl-carbonsäure-N-phenyl-N-(2-methoxyarbonylethyl)-amid-hydrochlorid

Hergestellt analog Beispiel 25d aus 1-Methyl-2-[N-(4-cyanophenyl)-aminomethyl]-benzimidazol-5-yl-carbonsäure-N-phenyl-N-(2-ethoxycarbonylethyl)-amid und methanolischer Salzsäure, Methanol und Ammoniumcarbonat.
Ausbeute: 87 % der Theorie,
$R_f$-Wert: 0.11 (Kieselgel; Dichlormethan/Ethanol = 4:1)
$C_{27}H_{28}N_6O_3$ (484.6)
EKA-Massenspektrum: $(M+H)^+ = 485$
$(M+2H)^{++} = 243$
$(M+H+Na)^{++} = 254$

Beispiel 54

1-Methyl-2-[(4-amidinophenyl)oxymethyl]-benzimidazol-5-yl-carbonsäure-N-phenyl-N-(2-ethoxycarbonylethyl)-amid-hydrochlorid

Hergestellt analog Beispiel 25d aus 1-Methyl-2-[(4-cyanophenyl)oxymethyl]-benzimidazol-5-yl-carbonsäure-N-phenyl-N-(2-ethoxycarbonylethyl)-amid und ethanolischer Salzsäure, Ethanol und Ammoniumcarbonat.
Ausbeute: 79.5 % der Theorie,
$C_{28}H_{29}N_5O_4$ (499.6)
$R_f$-Wert: 0.15 (Kieselgel; Dichlormethan/Ethanol = 4:1)
EKA-Massenspektrum: $(M+H)^+ = 500.0$
$(M+H+Na)^{++} = 261.7$

Beispiel 55

1-Methyl-2-[(4-amidinophenyl)oxymethyl]-benzimidazol-5-yl-carbonsäure-N-phenyl-N-(2-hydroxycarbonylethyl)-amid-hydrochlorid

Hergestellt analog Beispiel 26 aus 1-Methyl-2-[(4-amidinophenyl)oxymethyl]-benzimidazol-5-yl-carbonsäure-N-phenyl-N-(2-ethoxycarbonylethyl)-amid-hydrochlorid und Natronlauge.
Ausbeute: 82 % der Theorie,
$C_{26}H_{25}N_5O_4$ (471.5)
$R_f$-Wert: 0.11 (Kieselgel; Dichlormethan/Ethanol = 4:1)
EKA-Massenspektrum: $(M+H)^+ = 472$
$(M+H+Na)^{++} = 247.6$
$(M+Na)^+ = 494$
$(M+2Na)^{++} = 258.6$

Beispiel 56

1-Methyl-2-[2-(2-amidinothiophen-5-yl)ethyl]-benzimidazol-5-yl-carbonsäure-N-(2-pyridyl)-N-(2-ethoxycarbonylethyl)-amid-hydrochlorid

a) 1-Methyl-2-[2-(2-cyanothiophen-5-yl)-ethyl]-benzimidazol-5-yl-carbonsäure-N-(2-pyridyl)-N-(2-ethoxycarbonylethyl)-amid

Hergestellt analog Beispiel 25c aus 3-(2-Cyanothiophen-5-yl)-propionsäure und 3-Amino-4-methylamino-benzoesäure-N-(2-pyridyl)-N-(2-ethoxycarbonylethyl)amid.
Ausbeute: 18 % der Theorie,
$R_f$-Wert: 0.66 (Kieselgel; Dichlormethan/Methanol = 9:1)

b) 1-Methyl-2-[2-(2-amidinothiophen-5-yl)ethyl]-benzimidazol-5-yl-carbonsäure-N-(2-pyridyl)-N-(2-ethoxycarbonylethyl)-amid-hydrochlorid

Hergestellt analog Beispiel 25d aus 1-Methyl-2-[2-(2-cyanothiophen-5-yl)ethyl]-benzimidazol-5-yl-carbonsäure-N-(2-pyridyl)-N-(2-ethoxycarbonylethyl)-amid und ethanolischer Salzsäure, Ethanol und Ammoniumcarbonat.
Ausbeute: 53 % der Theorie,
$C_{26}H_{28}N_6O_3S$ (504.6)
$R_f$-Wert: 0.22 (Kieselgel; Dichlormethan/Methanol = 5:1)
EKA-Massenspektrum: $(M+H)^+ = 505$
$(M+H+Na)^{++} = 264$

Beispiel 57

1-Methyl-2-[2-(2-amidinothiophen-5-yl)ethyl]-benzimidazol-5-yl-carbonsäure-N-(2-pyridyl)-N-(2-hydroxycarbonylethyl)-amid

Hergestellt analog Beispiel 26 aus 1-Methyl-2-[2-(2-amidinothiophen-5-yl)ethyl]-benzimidazol-5-yl-carbonsäure-N-(2-pyridyl)-N-(2-ethoxycarbonylethyl)-amid-hydrochlorid und Natronlauge
Ausbeute: 98 % der Theorie,
$C_{24}H_{24}N_6O_3S$ (476.6)
EKA-Massenspektrum: $(M+H)^+ = 477$
$(M+Na)^+ = 499$
$(M+2H)^{++} = 239$

Beispiel 58

1-Methyl-2-[N-(4-amidinophenyl)-aminomethyl]-benzimidazol-5-yl-carbonsäure-N-(2-pyridyl)-N-(2-ethoxycarbonylethyl)-amid-hydrochlorid

a) 1-Methyl-2-[N-(4-cyanophenyl)-aminomethyl]-benzimidazol-5-yl-carbonsäure-N-(2-pyridyl)-N-(2-ethoxycarbonylethyl)-amid

Hergestellt analog Beispiel 25c aus N-(4-Cyanophenyl)-glycin und 3-Amino-4-methylamino-benzoesäure-N-(2-pyridyl)-N-(2-ethoxycarbonylethyl)-amid.
Ausbeute: 61 % der Theorie,
$R_f$-Wert: 0.62 (Kieselgel; Dichlormethan/Methanol = 19:1)

b) 1-Methyl-2-[N-(4-amidinophenyl)-aminomethyl]-benzimidazol-5-yl-carbonsäure-N-(2-pyridyl)-N-(2-ethoxycarbonylethyl)-amid-hydrochlorid

Hergestellt analog Beispiel 25d aus 1-Methyl-2-[N-(4-cyanophenyl)-aminomethyl]-benzimidazol-5-yl-carbonsäure-N-(2-pyridyl)-N-(2-ethoxycarbonylethyl)-amid und ethanolischer Salzsäure, Ethanol und Ammoniumcarbonat.
Ausbeute: 71 % der Theorie,
$C_{27}H_{29}N_7O_3$ (499.6)
$R_f$-Wert: 0.28 (Kieselgel; Dichlormethan/Methanol = 5:1)
EKA-Massenspektrum: $(M+H)^+ = 500$
$(M+H+Na)^{++} = 261.8$
$(M+2H)^{++} = 250.8$

Beispiel 59

1-Methyl-2-[N-(4-amidinophenyl)-aminomethyl]-benzimidazol-5-ylcarbonsäure-N-(2-pyridyl)-N-(2-hydroxycarbonylethyl)-amid

Hergestellt analog Beispiel 26 aus 1-Methyl-2-[N-(4-amidinophenyl)-aminomethyl]-benzimidazol-5-yl-carbonsäure-N-(2-pyridyl)-N-(2-ethoxycarbonylethyl)-amid-hydrochlorid und Natronlauge.
Ausbeute: 91 % der Theorie,
$C_{25}H_{25}N_7O_3$ (471.5)
EKA-Massenspektrum: $(M+H)^+ = 472$
$(M+H+Na)^{++} = 247.6$
$(M+2H)^{++} = 236.7$
$(M+2Na)^{++} = 258.6$

Beispiel 60

1-Methyl-2-[2-(4-amidinophenyl)ethyl]-benzimidazol-5-yl-carbonsäure-N-(2-pyridyl)-N-(2-ethoxycarbonylethyl)-amid-hydrochlorid

a) 1-Methyl-2-[2-(4-cyanophenyl)-ethyl]-benzimidazol-5-yl-carbonsäure-N-(2-pyridyl)-N-(2-ethoxycarbonylethyl)-amid

Hergestellt analog Beispiel 149a aus 3-(4-Cyanophenyl)-propionsäure und 3-Amino-4-methylamino-benzoesäure-N-(2-pyridyl)-N-(2-ethoxycarbonylethyl)-amid.
Ausbeute: 22 % der Theorie,
$R_f$-Wert: 0.68 (Kieselgel; Dichlormethan/Methanol = 19:1)

b) 1-Methyl-2-[2-(4-amidinophenyl)ethyl]-benzimidazol-5-yl-carbonsäure-N-(2-pyridyl)-N-(2-ethoxycarbonylethyl)-amid-hydrochlorid

Hergestellt analog Beispiel 25d aus 1-Methyl-2-[2-(4-cyanophenyl)ethyl]-benzimidazol-5-yl-carbonsäure-N-(2-pyridyl)-N-(2-ethoxycarbonylethyl)-amid und ethanolischer Salzsäure, Ethanol und Ammoniumcarbonat.
Ausbeute: 85 % der Theorie,
$C_{28}H_{30}N_6O_3$ (498.6)
$R_f$-Wert: 0.30 (Kieselgel; Dichlormethan/Methanol = 5:1)
EKA-Massenspektrum: $(M+H)^+$ = 499
$(M+H+Na)^{++}$ = 261

Beispiel 61

1-Methyl-2-[2-(4-amidinophenyl)ethyl]-benzimidazol-5-yl-carbonsäure-N-(2-pyridyl)-N-(2-hydroxycarbonylethyl)-amid

Hergestellt analog Beispiel 26 aus 1-Methyl-2-[2-(4-amidinophenyl)ethyl]-benzimidazol-5-yl-carbonsäure-N-(2-pyridyl)-N-(2-ethoxycarbonylethyl)-amid-hydrochlorid und Natronlauge. Ausbeute: 97 % der Theorie,
$C_{26}H_{26}N_6O_3$ (470.5)
EKA-Massenspektrum: $(M+H)^+$ = 471
$(M+H+Na)^{++}$ = 247
$(M+Na)^+$ = 493

Beispiel 62

1-Methyl-2-[2-(4-amidinophenyl)ethyl]-benzimidazol-5-yl-carbonsäure-N-phenyl-N-(2-ethoxycarbonylethyl)-amid-hydrochlorid

Hergestellt analog Beispiel 25d aus 1-Methyl-2-[2-(4-cyanophenyl)ethyl]-benzimidazol-5-yl-carbonsäure-N-phenyl-N-(2-ethoxycarbonylethyl)-amid und ethanolischer Salzsäure, Ethanol und Ammoniumcarbonat.
Ausbeute: 86 % der Theorie,
$C_{29}H_{31}N_5O_3$ (497.6)
Rf-Wert: 0.11 (Kieselgel; Dichlormethan/Ethanol = 4:1)
EKA-Massenspektrum: $(M+H)^+$ = 498
$(M+2H)^{++}$ = 249.8

Beispiel 63

1-Methyl-2-[2-(4-amidinophenyl)ethyl]-benzimidazol-5-yl-carbonsäure-N-phenyl-N-(2-hydroxycarbonylethyl)-amid-hydrochlorid

Hergestellt analog Beispiel 26 aus 1-Methyl-2-[2-(4-amidinophenyl)ethyl]-benzimidazol-5-yl-carbonsäure-N-phenyl-N-(2-ethoxycarbonylethyl)-amid-hydrochlorid und Natronlauge.
Ausbeute: 71 % der Theorie,
$C_{27}H_{27}N_5O_3$ (469.6)
EKA-Massenspektrum: $(M+H)^+$ = 470
$(M+H+Na)^{++}$ = 246.6
$(M+Na)^+$ = 492
$(M+2H)^{++}$ = 235.6

Beispiel 64

1-Methyl-2-[N-(4-amidinophenyl)-aminomethyl]-benzimidazol-5-yl-carbonsäure-N-(2-pyridyl)-N-(methoxycarbonylmethyl)-amid-dihydrochlorid

Hergestellt analog Beispiel 25d aus 1-Methyl-2-[N-(4-cyanophenyl)-aminomethyl]-benzimidazol-5-yl-carbonsäure-N-(2-pyridyl)-N-(methoxycarbonylmethyl)-amid und methanolischer Salzsäure, Methanol und Ammoniumcarbonat.
Ausbeute: 73 % der Theorie,

$C_{25}H_{25}N_7O_3$ (471.5)
$R_f$-Wert: 0.12 (Kieselgel; Dichlormethan/Ethanol = 4:1)
EKA-Massenspektrum: $(M+H)^+ = 472$
$(M+H+Na)^{++} = 247.8$

Beispiel 65

1-Methyl-2-[N-(4-amidinophenyl)-aminomethyl]-benzimidazol-5-yl-carbonsäure-N-(2-pyridyl)-N-(2-methoxycarbonylethyl)-amid-hydrochlorid

Hergestellt analog Beispiel 25d aus 1-Methyl-2-[N-(4-cyanophenyl)-aminomethyl]-benzimidazol-5-yl-carbonsäure-N-(2-pyridyl)-N-(2-methoxycarbonylethyl)-amid und methanolischer Salzsäure, Methanol und Ammoniumcarbonat.
Ausbeute: 78 % der Theorie,
$C_{26}H_{27}N_7O_3$ (485.6)
$R_f$-Wert: 0.31 (Kieselgel; Dichlormethan/Methanol = 5:1)
EKA-Massenspektrum: $(M+H)^+ = 486$
$(M+H+Na)^{++} = 254.8$

Beispiel 66

1-Methyl-2- [2- (4-amidinophenyl) ethyl] -benzimidazol-5-yl-carbonsäure-N-phenyl-N-[2-(1H-tetrazol-5-yl)ethyl]-amid-hydrochlorid

a) 1-Methyl-2-[2-(4-cyanophenyl)ethyl]-benzimidazol-5-yl-carbonsäure-N-phenyl-N-[2-(1H-tetrazol-5-yl)ethyl]-amid

Hergestellt analog Beispiel 25c aus 3-(4-Cyanophenyl)-propionsäure und 3-Amino-4-methylamino-benzoesäure-N-phenyl-N-[2-(1H-tetrazol-5-yl)ethyl]-amid.
Ausbeute: 67 % der Theorie,
IR-Massenspektrum (KBr): charakteristische Banden bei
3439.5 $cm^{-1}$ (N-H); 2235.5 $cm^{-1}$ (C≡N); 1631.6 $cm^{-1}$ (C=O)

b) 1-Methyl-2-[2-(4-amidinophenyl)ethyl]-benzimidazol-5-yl-carbonsäure-N-phenyl-N-[2-(1H-tetrazol-5-yl)ethyl]-amid-hydrochlorid

Hergestellt analog Beispiel 25d aus 1-Methyl-2-[2-(4-cyanophenyl)ethyl]-benzimidazol-5-yl-carbonsäure-N-phenyl-N-[2-(1H-tetrazol-5-yl)ethyl]-amid und ethanolischer Salzsäure, Ethanol und Ammoniumcarbonat.
Ausbeute: 92 % der Theorie,
$C_{27}H_{27}N_9O$ (493.6)
EKA-Massenspektrum: $(M+H)^+ = 494$
$(M+Na)^+ = 516$
$(M+2H)^{++} = 258.7$

Beispiel 67

1-Methyl-2-[N-(4-amidinophenyl)-aminomethyl]-benzimidazol-5-ylcarbonsäure-N-phenyl-N-[2-(1H-tetrazol-5-yl)ethyl]-amid-hydrochlorid

Hergestellt analog Beispiel 25d aus 1-Methyl-2-[N-(4-cyanophenyl)-aminomethyl]-benzimidazol-5-yl-carbonsäure-N-phenyl-N-[2-(1H-tetrazol-5-yl)ethyl]-amid und ethanolischer Salzsäure, Ethanol und Ammoniumcarbonat.
Ausbeute: 29 % der Theorie,
$C_{26}H_{26}N_{10}O$ (494.6)
EKA-Massenspektrum: $(M+H)^+ = 495$

Beispiel 68

1-Methyl-2-[N-(4-amidinophenyl)-aminomethyl]-benzimidazol-5-yl-carbonsäure-N-(2-pyridyl)-N-(2-n-hexyloxycarbonylethyl)-amid-hydrochlorid

In ca. 30 ml mit Chlorwasserstoff gesättigtem n-Hexanol wurden 0.60 g (1.1 mMol) 1-Methyl-2-[N-(4-amidinophenyl)-aminomethyl]-benzimidazol-5-yl-carbonsäure-N-(2-pyridyl)-N-(2-ethoxycarbonylethyl)-amid-hydrochlorid gegeben und 19 Stunden lang bei Raumtemperatur gerührt. Dann wurde im Vakuum das Hexanol abdestilliert, der Rückstand mit ca 5 ml 1N Ammoniaklösung unter Rühren versetzt und erneut eingedampft. Das so erhaltene Rohprodukt wurde durch Säulenchromatographie gereinigt (Kieselgel, Dichlormethan/Methanol = 5:1).
Ausbeute: 53 % der Theorie,
$C_{31}H_{37}N_7O_3$ (555.7)
$R_f$-Wert: 0.36 (Kieselgel; Dichlormethan/Methanol = 5:1 )
EKA-Massenspektrum: $(M+H)^+$ = 556

Beispiel 69

1-Methyl-2-[N-(4-amidinophenyl)-N-methyl-aminomethyl]-benzimidazol-5-yl-carbonsäure-N-(2-pyridyl)-N-(2-ethoxycarbonylethyl)-amid-hydrochlorid

a) 1-Methyl-2-[N-(4-cyanophenyl)-N-methyl-aminomethyl]-benzimidazol-5-yl-carbonsäure-N-(2-pyridyl)-N-(2-ethoxycarbonylethyl)-amid

Hergestellt analog Beispiel 25c aus N-(4-Cyanophenyl)-N-methylglycin und 3-Amino-4-methylamino-benzoesäure-N-(2-pyridyl)-N-(2-ethoxycarbonylethyl)-amid.
Ausbeute: 71 % der Theorie,
$R_f$-Wert: 0.66 (Kieselgel; Dichlormethan/Methanol = 19:1)

b) 1-Methyl-2-[N-(4-amidinophenyl)-N-methyl-aminomethyl]-benzimidazol-5-yl-carbonsäure-N-(2-pyridyl)-N-(2-ethoxycarbonylethyl)-amid-hydrochlorid

Hergestellt analog Beispiel 25d aus 1-Methyl-2-[N-(4-cyanophenyl)-N-methyl-aminomethyl]-benzimidazol-5-yl-carbonsäure-N-(2-pyridyl)-N-(2-ethoxycarbonylethyl)-amid und ethanolischer Salzsäure, Ethanol und Ammoniumcarbonat.
Ausbeute: 77 % der Theorie,
$C_{28}H_{31}N_7O_3$ (513.6)
EKA-Massenspektrum: $(M+H)^+$ = 514
$(M+H+Na)^{++}$ = 268.7

Beispiel 70

1-Methyl-2-[N-(4-amidinophenyl)-N-methyl-aminomethyl]-benzimidazol-5-yl-carbonsäure-N-(2-pyridyl)-N-(2-hydroxycarbonylethyl)-amid

Hergestellt analog Beispiel 26 aus 1-Methyl-2-[N-(4-amidinophenyl)-N-methyl-aminomethyl]-benzimidazol-5-yl-carbonsäure-N-(2-pyridyl)-N-(2-ethoxycarbonylethyl)-amid-hydrochlorid und Natronlauge.
Ausbeute: 66 % der Theorie,
$C_{26}H_{27}N_7O_3$ (485.6)
EKA-Massenspektrum: $(M+H)^+$ = 486
$(M+Na)^+$ = 508
$(M+2Na)^{++}$ = 265.6

Beispiel 71

1-Methyl-2-[2-(4-amidinophenyl)ethyl]-benzimidazol-5-yl-carbonsäure-N-cyclopentyl-N-(2-ethoxycarbonylethyl)-amid-hydrochlorid

Hergestellt analog Beispiel 25d aus 1-Methyl-2-[2-(4-cyanophenyl)ethyl]-benzimidazol-5-yl-carbonsäure-N-cyclopentyl-N-(2-ethoxycarbonylethyl)-amid und ethanolischer Salzsäure, Ethanol und Ammoniumcarbonat.
Ausbeute: 65 % der Theorie,
$C_{28}H_{35}N_5O_3$ (489.6)
EKA-Massenspektrum: $(M+H)^+ = 490$

Beispiel 72

1-Methyl-2-[2-(4-amidinophenyl)ethyl]-benzimidazol-5-yl-carbonsäure-N-cyclopentyl-N-(2-hydroxycarbonylethyl)-amid

Hergestellt analog Beispiel 26 aus 1-Methyl-2-[2-(4-amidinophenyl)ethyl]-benzimidazol-5-yl-carbonsäure-N-cyclopentyl-N-(2-ethoxycarbonylethyl)-amid-hydrochlorid und Natronlauge.
Ausbeute: 89 % der Theorie,
$C_{26}H_{31}N_5O_3$ (461.6)
EKA-Massenspektrum: $(M+H)^+ = 462$
$(M+H+Na)^{++} = 242.6$
$(M+Na)^+ = 484$
$(M+2H)^{++} = 231.6$

Beispiel 73

1-Methyl-2-[N-(4-amidinophenyl)-aminomethyl]-benzimidazol-5-yl-carbonsäure-N-cyclopentyl-N-(2-ethoxycarbonylethyl)-amid-hydrochlorid

Hergestellt analog Beispiel 25d aus 1-Methyl-2-[N-(4-cyanophenyl)-aminomethyl]-benzimidazol-5-yl-carbonsäure-N-cyclopentyl-N-(2-ethoxycarbonylethyl)-amid und ethanolischer Salzsäure, Ethanol und Ammoniumcarbonat.
Ausbeute: 60 % der Theorie,
$C_{27}H_{34}N_6O_3$ (490.6)
EKA-Massenspektrum: $(M+H)^+ = 491$

Beispiel 74

1-Methyl-2-[N-(4-amidinophenyl)-aminomethyl]-benzimidazol-5-yl-carbonsäure-N-cyclopentyl-N-(2-hydroxycarbonylethyl)-amid

Hergestellt analog Beispiel 26 aus 1-Methyl-2-[N-(4-amidinophenyl)-aminomethyl]-benzimidazol-5-yl-carbonsäure-N-cyclopentyl-N-(2-ethoxycarbonylethyl)-amid-hydrochlorid und Natronlauge. Ausbeute: 45 % der Theorie,
$C_{25}H_{30}N_3O_4$ (462.6)
EKA-Massenspektrum: $(M+H)^+ = 463$
$(M+H+Na)^{++} = 243$
$(M+Na)^+ = 485$
$(M+2Na)^{++} = 254$

Beispiel 75

1-Methyl-2-[N-(4-amidinophenyl)-N-methyl-aminomethyl]-benzimidazol-5-yl-carbonsäure-N-(2-pyridyl)-N-(ethoxycarbonylmethyl)-amid-hydrochlorid

Hergestellt analog Beispiel 25d aus 1-Methyl-2-[N-(4-cyanophenyl)-N-methyl-aminomethyl]-benzimidazol-5-yl-carbonsäure-N-(2-pyridyl)-N-(ethoxycarbonylmethyl)-amid und ethanolischer Salzsäure, Ethanol und Ammoniumcarbonat.
Ausbeute: 54 % der Theorie,
$C_{27}H_{29}N_7O_3$ (499.6)
EKA-Massenspektrum: $(M+H)^+ = 500$
$(M+2H\ )^{++} = 250.7$

Beispiel 76

1-Methyl-2-[N-(4-amidinophenyl)-N-methyl-aminomethyl]-benzimidazol-5-yl-carbonsäure-N-(2-pyridyl)-N-(hydroxycarbonylmethyl)-amid

Hergestellt analog Beispiel 26 aus 1-Methyl-2-[N-(4-amidinophenyl)-N-methyl-aminomethyl]-benzimidazol-5-yl-carbonsäure-N-(2-pyridyl)-N-(ethoxycarbonylmethyl)-amid-hydrochlorid und Natronlauge.
Ausbeute: 68 % der Theorie,
$C_{25}H_{25}N_7O_3$ (471.5)
EKA-Massenspektrum: $(M+H)^+ = 472$
$(M+Na)^+ = 494$
$(M+2Na)^{++} = 258.6$

Beispiel 77

1-Methyl-2-[2-(4-amidinophenyl)-ethyl]-benzimidazol-5-yl-carbonsäure-N-(3-pyridyl)-N-(2-ethoxycarbonylethyl)-amid-hydrochlorid

Hergestellt analog Beispiel 25d aus 1-Methyl-2-[2-(4-cyanophenyl)-ethyl]-benzimidazol-5-yl-carbonsäure-N-(3-pyridyl)-N-(2-ethoxycarbonylethyl)-amid und ethanolischer Salzsäure, Ethanol und Ammoniumcarbonat.
Ausbeute: 91 % der Theorie,
$C_{28}H_{30}N_6O_3$ (498.6)
$R_f$-Wert: 0.19 (Kieselgel; Dichlormethan/Ethanol = 4:1)
EKA-Massenspektrum: $(M+H)^+ = 499$

Beispiel 78

1-Methyl-2-[N-(4-amidinophenyl)-aminomethyl]-benzimidazol-5-yl-carbonsäure-N-(3-pyridyl)-N-(2-ethoxycarbonylethyl)-amid-dihydrochlorid

Hergestellt analog Beispiel 25d aus 1-Methyl-2-[N-(4-cyanophenyl)-aminomethvl]-benzimidazol-5-yl-carbonsäure-N-(3-pyridyl)-N-(2-ethoxycarbonylethyl)-amid und ethanolischer Salzsäure, Ethanol und Ammoniumcarbonat.
Ausbeute: 86 % der Theorie,
$C_{27}H_{29}N_7O_3$ (499.6)
$R_f$-Wert: 0.09 (Kieselgel; Dichlormethan/Ethanol = 4:1
EKA-Massenspektrum: $(M+H)^+ = 500$

Beispiel 79

1-Methyl-2-[N-(4-amidinophenyl)-aminomethyl]-benzimidazol-5-yl-carbonsäure-N-(3-pyridyl)-N-(2-hydroxycarbonylethyl)-amid

Hergestellt analog Beispiel 26 aus 1-Methyl-2-[N-(4-amidinophenyl)-aminomethyl] -benzimidazol-5-yl-carbonsäure-N- (3-pyridyl)-N-(2-ethoxycarbonylethyl)-amid-dihydrochlorid und Natronlauge.
Ausbeute: 85 % der Theorie,
$C_{25}H_{25}N_7O_3$ (471.5)
EKA-Massenspektrum: $(M+H)^+ = 472$
$(M+2H)^{++} = 236.6$
$(M+2Na)^{++} = 258.6$

Beispiel 80

1-Methyl-2-[N-(4-amidinophenyl)-N-methyl-aminomethyl]-benzimidazol-5-yl-carbonsäure-N-(3-pyridyl)-N-(2-ethoxycarbonylethyl)-amid-hydrochlorid

Hergestellt analog Beispiel 25d aus 1-Methyl-2-[N-(4-cyanophenyl)-N-methyl-aminomethyl]-benzimidazol-5-yl-carbonsäure-N-(3-pyridyl)-N-(2-ethoxycarbonylethyl)-amid und ethanolischer Salzsäure, Ethanol und Ammoniumcarbonat.

Ausbeute: 64 % der Theorie,
$C_{28}H_{31}N_7O_3$ (513.6)
EKA-Massenspektrum: $(M+H)^+ = 514$

Beispiel 81

1-Methyl-2-[N-(4-amidinophenyl)-N-methyl-aminomethyl]-benzimidazol-5-yl-carbonsäure-N-(3-pyridyl)-N-(2-hydroxycarbonylethyl)-amid

Hergestellt analog Beispiel 26 aus 1-Methyl-2-[N-(4-amidinophenyl)-N-methyl-aminomethyl] -benzimidazol-5-yl-carbonsäure-N-(3-pyridyl)-N-(2-ethoxycarbonylethyl)-amid-hydrochlorid und Natronlauge.
Ausbeute: 70 % der Theorie,
$C_{26}H_{27}N_7O_3$ (485.6)
EKA-Massenspektrum: $(M+H)^+ = 486$
$(M+Na)^+ = 508$
$(M+2Na)^{++} = 265.6$

Beispiel 82

1-Methyl-2-[N-(4-amidinophenyl)-N-methyl-aminomethyl]-benzimidazol-5-yl-carbonsäure-N-phenyl-N-(2-ethoxycarbonylethyl)-amidhydrochlorid

a) 1-Methyl-2-[N-(4-cyanophenyl)-N-methyl-aminomethyl]-benzi-midazol-5-yl-carbonsäure-N-phenyl-N-(2-pyridyl) -N-(2-ethoxycarbonylethyl)-amid

Hergestellt analog Beispiel 25c aus N-(4-Cyanophenyl)-N-methylglycin und 3-Amino-4-methylamino-benzoesäure-N-phenyl-N-(2-ethoxycarbonylethyl)-amid.
Ausbeute: 71 % der Theorie,
$R_f$-Wert: 0.38 (Kieselgel; Dichlormethan/Methanol = 19:1)

b)1-Methyl-2-[N-(4-amidinophenyl)-N-methyl-aminomethyl]-benzimidazol-5-yl-carbonsäure-N-phenyl-N-(2-ethoxycarbonylethyl)-amid-hydrochlorid

Hergestellt analog Beispiel 25d aus 1-Methyl-2-[N-(4-cyanophenyl)-N-methyl-aminomethyl]-benzimidazol-5-yl-carbonsäure-N-phenyl-N-(2-ethoxycarbonylethyl)-amid und ethanolischer Salzsäure, Ethanol und Ammoniumcarbonat.
Ausbeute: 74 % der Theorie,
$C_{29}H_{32}N_6O_3$ (512.6)
EKA-Massenspektrum: $(M+H)^+ = 513$
$(M+H+Na)^{++} = 268$
$(M+2H)^{++} = 257$

Beispiel 83

1-Methyl-2-[N-(4-amidinophenyl)-N-methyl-aminomethyl]-benzimidazol-5-yl-carbonsäure-N-phenyl-N-(2-hydroxycarbonylethyl)-amid

Hergestellt analog Beispiel 26 aus 1-Methyl-2-[N-(4-amidinophenyl)-N-methyl-aminomethyl]-benzimidazol-5-yl-carbonsäure-N-phenyl-N-(2-ethoxycarbonylethyl)-amid-hydrochlorid und Natronlauge.
Ausbeute: 80 % der Theorie,
$C_{27}H_{28}N_6O_3$ (484.6)
EKA-Massenspektrum: $(M+H)^+ = 485$
$(M+H+Na)^{++} = 254$
$(M+Na)^+ = 507$
$(M+2Na)^+ = 265$

Beispiel 84

1-Ethyl-2-[N-(4-amidinophenyl)-aminomethyl]-benzimidazol-5-yl-carbonsäure-N-(2-pyridyl)-N-(2-ethoxycarbonylethyl)-amid-hydrochlorid

Hergestellt analog Beispiel 25d aus 1-Ethyl-2-[N-(4-cyanophenyl)-aminomethyl]-benzimidazol-5-yl-carbonsäure-N-(2-pyridyl)-N-(2-ethoxycarbonylethyl)-amid und ethanolischer Salzsäure, Ethanol und Ammoniumcarbonat.
Ausbeute: 85 % der Theorie,
$C_{28}H_{31}N_7O_3$ (513.6)
Rf-Wert: 0.21 (Kieselgel; Dichlormethan/Methanol = 5:1)
EKA-Massenspektrum: $(M+H)^+$ = 514
$(M+H+Na)^{++}$ = 268.6
$(M+2H)^{++}$ = 257.7

Beispiel 85

1-Ethyl-2-[N-(4-amidinophenyl)-aminomethyl]-benzimidazol-5-yl-carbonsäure-N-(2-pyridyl)-N-(2-hydroxycarbonylethyl)-amid

Hergestellt analog Beispiel 26 aus 1-Ethyl-2-[N-(4-amidinophenyl)-aminomethyl]-benzimidazol-5-yl-carbonsäure-N-(2-pyridyl)-N-(2-ethoxycarbonylethyl)-amid-hydrochlorid und 2N Natronlauge.
Ausbeute: 49 % der Theorie,
$C_{26}H_{27}N_7O_3$ (485.6)
EKA-Massenspektrum: $(M+H)^+$ = 486
$(M+H+Na)^{++}$ = 254.6
$(M+2H)^{++}$ = 243.6
$(M+2Na)^{++}$ = 265.7

Beispiel 86

1-Methyl-2-[N-(4-amidinophenyl)-aminomethyl]-benzimidazol-5-yl-carbonsäure-N-(2-fluorphenyl)-N-(2-ethoxycarbonylethyl)-amid-hydrochlorid

Hergestellt analog Beispiel 25d aus 1-Methyl-2-[N-(4-cyanophenyl)-aminomethyl]-benzimidazol-5-yl-carbonsäure-N-(2-fluorphenyl)-N-(2-ethoxycarbonylethyl)-amid und ethanolischer Salzsäure, Ethanol und Ammoniumcarbonat.
Ausbeute: 88 % der Theorie,
$C_{28}H_{29}FN_6O_3$ (516.6)
$R_f$-Wert: 0.08 (Kieselgel; Dichlormethan/Ethanol = 4:1)
EKA-Massenspektrum: $(M+H)^+$ = 517
$(M+H+Na)^{++}$ = 270
$(M+2H)^{++}$ = 259

Beispiel 87

1-Methyl-2-[N-(4-amidinophenyl)-aminomethyl]-benzimidazol-5-yl-carbonsäure-N-(2-fluorphenyl)-N-(2-hydroxycarbonylethyl)-amid

Hergestellt analog Beispiel 26 aus 1-Methyl-2-[N-(4-amidinophenyl) -aminomethyl] -benzimidazol-5-yl-carbonsäure-N- (2-fluorphenyl)-N-(2-ethoxycarbonylethyl)-amid-hydrochlorid und Natronlauge.
Ausbeute: 45 % der Theorie,
$C_{26}H_{25}FN_6O_3$ (488.5)
$R_f$-Wert: 0.05 (Kieselgel; Dichlormethan/Ethanol = 4:1)
EKA-Massenspektrum: $(M+H)^+$ = 489
$(M+H+Na)^{++}$ = 267
$(M+2H)^{++}$ = 256

Beispiel 88

1-Methyl-2-[N-(4-amidinophenyl)-aminomethyl]-benzimidazol-5-yl-carbonsäure-N-(3-methylphenyl)-N-(2-ethoxycarbonylethyl)-amid-hydrochlorid

Hergestellt analog Beispiel 25d aus 1-Methyl-2-[N-(4-cyanophenyl)-aminomethyl]-benzimidazol-5-yl-carbonsäure-N-(3-methylphenyl)-N-(2-ethoxycarbonylethyl)-amid und ethanolischer Salzsäure, Ethanol und Ammoniumcarbonat.
Ausbeute: 79 % der Theorie,
$C_{29}H_{32}N_6O_3$ (512.6)
$R_f$-Wert: 0.10 (Kieselgel; Dichlormethan/Ethanol = 4:1)
EKA-Massenspektrum: $(M+H)^+ = 513$
$(M+H+Na)^{++} = 268$

Beispiel 89

1-Methyl-2-[N-(4-amidinophenyl)-aminomethyl]-benzimidazol-5-yl-carbonsäure-N-(3-methylphenyl)-N-(2-hydroxycarbonylethyl)-amid

Hergestellt analog Beispiel 26 aus 1-Methyl-2-[N-(4-amidinophenyl)-aminomethyl]-benzimidazol-5-yl-carbonsäure-N-(3-methylphenyl)-N-(2-ethoxycarbonylethyl)-amid-hydrochlorid und Natronlauge.
Ausbeute: 62 % der Theorie,
$C_{27}H_{28}N_6O_3$ (484.6)
EKA-Massenspektrum: $(M+H)^+ = 485$
$(M+H+Na)^{++} = 254$
$(M+Na)^+ = 507$
$(M+2Na)^{++} = 265$

Beispiel 90

1-Methyl-2-[N-[4-(N-n-hexyloxycarbonylamidino)phenyl]-aminomethyl]-benzimidazol-5-yl-carbonsäure-N-phenyl-N-(2-ethoxycarbonylethyl)-amid

1.1 g (2.06 mMol) 1-Methyl-2-[N-(4-amidinophenyl)-aminomethyl]-benzimidazol-5-yl-carbonsäure-N-phenyl-N-(2-ethoxycarbonylethyl)-amid-hydrochlorid wurden in einem Gemisch aus 40 ml Tetrahydrofuran und 10 ml Wasser gelöst, anschließend 570 mg (4.12 mMol) Kaliumcarbonat und 362 mg (2.2 mMol) Chlorameisensäure-n-hexylester zugesetzt und zwei Stunden lang bei Raumtemperatur gerührt. Das Lösungsmittel wurde dann abdestilliert, der Rückstand mit ca. 50 ml gesättigter Kochsalzlösung versetzt und die so erhaltene Lösung dreimal mit je 20 ml Dichlormethan extrahiert. Die Extrakte wurden über Natriumsulfat getrocknet und eingeengt. Das so erhaltene Rohprodukt wurde durch Säulenchromatographie (100 g Kieselgel; Dichlormethan + 5% Ethanol) gereinigt.
Ausbeute: 78 % der Theorie,
$C_{35}H_{42}N_6O_5$ (626.8)
$R_f$-Wert: 0.49 (Kieselgel; Dichlormethan/Ethanol = 19:1)
EKA-Massenspektrum: $(M+H)^+ = 627$
$(M+H+Na)^{++} = 325$
$(M+2H)^{++} = 314$

Beispiel 91

1-Methyl-2-[N-[4-(N-methoxycarbonylamidino)phenyl]-aminomethyl]-benzimidazol-5-yl-carbonsäure-N-phenyl-N-(2-ethoxycarbonylethyl)-amid

Hergestellt analog Beispiel 90 aus 1-Methyl-2-[N-(4-amidinophenyl)-aminomethyl]-benzimidazol-5-yl-carbonsäure-N-phenyl-N-(2-ethoxycarbonylethyl)-amid-hydrochlorid und Chlorameisensäuremethylester.
Ausbeute: 41 % der Theorie,
$C_{30}H_{32}N_6O_5$ (556.6)
$R_f$-Wert: 0.85 (Kieselgel; Dichlormethan/Ethanol = 4:1)
EKA-Massenspektrum: $(M+H)^+ = 557$

(M+H+Na)$^{++}$ = 290
(M+Na)$^{+}$ = 579

Beispiel 92

1-Methyl-2-[N-[4-(N-ethoxycarbonylamidino)phenyl]-aminomethyl]-benzimidazol-5-yl-carbonsäure-N-phenyl-N-(2-methoxycarbonylethyl)-amid

Hergestellt analog Beispiel 90 aus 1-Methyl-2-[N-(4-amidinophenyl)-aminomethyl]-benzimidazol-5-yl-carbonsäure-N-phenyl-N-(2-methoxycarbonylethyl)-amid-hydrochlorid und Chlorameisensäureethylester.
Ausbeute: 62 % der Theorie,
$C_{30}H_{32}N_6O_5$ (556.6)
$R_f$-Wert: 0.51 (Kieselgel; Dichlormethan/Ethanol = 19:1)
EKA-Massenspektrum: (M+H)$^{+}$ = 557
(M+H+Na)$^{++}$ = 290
(M+2H)$^{++}$ = 279

Beispiel 93

1-Methyl-2-[N-[4-(N-cyclohexyloxycarbonylamidino)phenyl]-aminomethyl]-benzimidazol-5-yl-carbonsäure-N-phenyl-N-(2-methoxycarbonylethyl)-amid

Hergestellt analog Beispiel 90 aus 1-Methyl-2-[N-(4-amidinophenyl)-aminomethyl]-benzimidazol-5-yl-carbonsäure-N-phenyl-N-(2-methoxycarbonylethyl)-amid-hydrochlorid und Chlorameisensäurecyclohexylester.
Ausbeute: 25 % der Theorie,
$C_{34}H_{38}N_6O_5$ (610.7)
$R_f$-Wert: 0.44 (Kieselgel; Dichlormethan/Ethanol = 19:1)
EKA-Massenspektrum: (M+H)$^{+}$ = 611
(M+2H)$^{++}$ = 306

Beispiel 94

1-Methyl-2-[N-[4 [N-[2-(methylsulfonyl)ethyloxycarbonyl]-amidino]phenyl]-aminomethyl]-benzimidazol-5-yl-carbonsäure-N-phenyl-N-(2-ethoxycarbonylethyl)-amid

Hergestellt analog Beispiel 90 aus 1-Methyl-2-[N-(4-amidinophenyl)-aminomethyl]-benzimidazol-5-yl-carbonsäure-N-phenyl-N-(2-ethoxycarbonylethyl)-amid-hydrochlorid und Chlorameisensäure-2-(methylsulfonyl)-ethylester.
Ausbeute: 66 % der Theorie,
$C_{32}H_{36}N_6O_7S$ (648.8)
$R_f$-Wert: 0.44 (Kieselgel; Dichlormethan/Ethanol = 19:1)
EKA-Massenspektrum: (M+H)$^{+}$ = 649
(M+H+Na)$^{++}$ = 336
(M+2H)$^{++}$ = 325

Beispiel 95

1-Methyl-2-[N-[4-(N-n-octyloxycarbonylamidino)phenyl]-aminomethyl]-benzimidazol-5-yl-carbonsäure-N-phenyl-N-(2-methoxycarbonylethyl)-amid

Hergestellt analog Beispiel 90 aus 1-Methyl-2-[N-(4-amidinophenyl)-aminomethyl]-benzimidazol-5-yl-carbonsäure-N-phenyl-N-(2-methoxycarbonylethyl)-amid-hydrochlorid und Chlorameisensäure-n-octylester.
Ausbeute: 41 % der Theorie,
$C_{36}H_{44}N_6O_5$ (640.8)
$R_f$-Wert: 0.43 (Kieselgel; Dichlormethan/Ethanol = 19:1)
EKA-Massenspektrum: (M+H)$^{+}$ = 641
(M+Na)$^{+}$ = 663

Beispiel 96

1-Methyl-2-[N-[4-(N-hydroxylamidino)phenyl]-aminomethyl)-benzimidazol-5-yl-carbonsäure-N-phenyl-N-(2-ethoxycarbonylethyl)-amid

1.44 g (3.0 mMol) 1-Methyl-2-[N-(4-cyanophenyl)-aminomethyl]-benzimidazol-5-yl-carbonsäure-N-phenyl-N-(2-ethoxycarbonylethyl)-amid, 0.625 g (9.0 mMol) Hydroxylaminhydrochlorid und 0.425 g (4.0 mMol) Natriumcarbonat wurden in 80 ml Ethanol gelöst und 7 Stunden lang zum Rückfluß erhitzt. Es wurden dann weitere 210 mg Hydroxylaminhydrochlorid und 170 mg Natriumcarbonat hinzugefügt, weitere 5 Stunden gekocht und anschließend im Vakuum eingeengt. Der Rückstand wurde in ca. 30 ml Dichlormethan gelöst, die erhaltene Lösung mit 20 ml Wasser gewaschen, die organische Phase getrocknet und eingeengt. Das so erhaltene Rohprodukt wurde säulenchromatographisch gereinigt (200 g Kieselgel, Dichlormethan + 4% Ethanol).
Ausbeute: 39 % der Theorie,
$C_{28}H_{30}N_6O_4$ (514.6)
$R_f$-Wert: 0.15 (Kieselgel; Dichlormethan/Ethanol = 19:1)
EKA-Massenspektrum: $(M+H)^+ = 515$
$(M+Na)^+ = 537$
$(2M+H)^+ = 1029$
$(2M+Na)^+ = 1051$

Beispiel 97

1-Methyl-2-[N-[4-(N-n-heptyloxycarbonylamidino)phenyl]-aminomethyl]-benzimidazol-5-yl-carbonsäure-N-phenyl-N-(2-methoxycarbonylethyl)-amid

Hergestellt analog Beispiel 90 aus 1-Methyl-2-[N-(4-amidinophenyl)-aminomethyl]-benzimidazol-5-yl-carbonsäure-N-phenyl-N-(2-methoxycarbonylethyl)-amid-hydrochlorid und Chlorameisensäure-n-heptylester.
Ausbeute: 43 % der Theorie,
$C_{35}H_{42}N_6O_5$ (626.8)
$R_f$-Wert: 0.40 (Kieselgel; Dichlormethan/Ethanol = 19:1)
EKA-Massenspektrum: $(M+H)^+ = 627$
$(M+H+Na)^{++} = 325$
$(M+Na)^+ = 649$

Beispiel 98

1-Methyl-2-[N-[4-(N-benzoylamidino)phenyl]-aminomethyl]-benzimidazol-5-yl-carbonsäure-N-phenyl-N-(2-methoxycarbonylethyl)-amid

Hergestellt analog Beispiel 90 aus 1-Methyl-2-[N-(4-amidinophenyl)-aminomethyl]-benzimidazol-5-yl-carbonsäure-N-phenyl-N-(2-methoxycarbonylethyl)-amid-hydrochlorid und Benzoylchlorid.
Ausbeute: 88 % der Theorie,
$C_{34}H_{32}N_6O_4$ (588.7)
$R_f$-Wert: 0.37 (Kieselgel; Dichlormethan/Ethanol = 19:1)
$^1$H-NMR-Spektrum ($D_6$-DMSO): 2.61 (t,2H), 3.54 (s,3H), 3.76 (s,3H), 4.10 (t,2H), 4.61 (d,2H), 6.83 (d,2H), 7.05 bis 7.55 (m,12H),8.03 (d,2H), 8.25 (dd,2H), 8.98 (s,1H), 10.48 (s,1H)

Beispiel 99

1-Methyl-2-[N-[4-(N-n-hexyloxycarbonylamidino)phenyl]-aminomethyl]-benzimidazol-5-yl-carbonsäure-N-phenyl-N-(2-methoxycarbonylethyl)-amid

Hergestellt analog Beispiel 90 aus 1-Methyl-2-[N-(4-amidinophenyl)-aminomethyl]-benzimidazol-5-yl-carbonsäure-N-phenyl-N-(2-methoxycarbonylethyl)-amid-hydrochlorid und Chlorameisensäure-n-hexylester.
Ausbeute: 54 % der Theorie,
$C_{34}H_{40}N_6O_5$ (612.7)
$R_f$-Wert: 0.45 (Kieselgel; Dichlormethan/Ethanol = 19:1)
EKA-Massenspektrum: $(M+H)^+ = 613$

Beispiel 100

1-Methyl-2-[N-[4-(N-n-hexyloxycarbonylamidino)phenyl]-aminomethyl]-benzimidazol-5-yl-carbonsäure-N-phenyl-N-(2-n-propyloxycarbonylethyl)-amid

Hergestellt analog Beispiel 90 aus 1-Methyl-2-[N-(4-amidinophenyl)-aminomethyl]-benzimidazol-5-yl-carbonsäure-N-phenyl-N-(2-n-propyloxycarbonylethyl)-amid-hydrochlorid und Chlorameisensäure-n-hexylester.
Ausbeute: 31 % der Theorie,
$C_{36}H_{44}N_6O_5$ (640.8)
$R_f$-Wert: 0.42 (Kieselgel; Dichlormethan/Ethanol = 19:1)
EKA-Massenspektrum: $(M+H)^+$ = 641
$(M+H+Na)^{++}$ = 332
$(M+Na)^+$ = 663

Beispiel 101

1-Methyl-2-[N-[4-(N-ethoxycarbonylamidino)phenyl]-aminomethyl]-benzimidazol-5-yl-carbonsäure-N-(2-pyridyl)-N-(2-methoxycarbonylethyl)-amid

Hergestellt analog Beispiel 90 aus 1-Methyl-2-[N-(4-amidinophenyl)-aminomethyl]-benzimidazol-5-yl-carbonsäure-N-(2-pyridyl)-N-(2-methoxycarbonylethyl)-amid-hydrochlorid und Chlorameisensäureethylester.
Ausbeute: 72 % der Theorie,
$C_{29}H_{31}N_7O_5$ (557.6)
$R_f$-Wert: 0.58 (Kieselgel; Dichlormethan/Methanol = 9:1)
EKA-Massenspektrum: $(M+H)^+$ = 558
$(M+H+Na)^{++}$ = 290.8
$(M+Na)^+$ = 580

Beispiel 102

1-Methyl-2-[N-[4-(N-n-octyloxycarbonylamidino)phenyl]-aminomethyl]-benzimidazol-5-yl-carbonsäure-N-(2-pyridyl)-N-(2-methoxycarbonylethyl)-amid

Hergestellt analog Beispiel 90 aus 1-Methyl-2-[N-(4-amidinophenyl)-aminomethyl]-benzimidazol-5-yl-carbonsäure-N-(2-pyridyl)-N-(2-methoxycarbonylethyl)-amid-hydrochlorid und Chlorameisensäure-n-octylester.
Ausbeute: 57 % der Theorie,
$C_{35}H_{43}N_7O_5$ (641.8)
$R_f$-Wert: 0.60 (Kieselgel; Dichlormethan/Methanol = 9:1)
EKA-Massenspektrum: $(M+H)^+$ = 642
$(M+H+Na)^{++}$ = 332.8
$(M+Na)^+$ = 664

Beispiel 103

1-Methyl-2-[N-[4-(N-methoxycarbonylamidino)phenyl]-amino-methyl]-benzimidazol-5-yl-carbonsäure-N-(2-pyridyl)-N-(2-ethoxycarbonylethyl)-amid

Hergestellt analog Beispiel 90 aus 1-Methyl-2-[N-(4-amidinophenyl)-aminomethyl]-benzimidazol-5-yl-carbonsäure-N-(2-pyridyl)-N-(2-ethoxycarbonylethyl)-amid-hydrochlorid und Chlorameisensäuremethylester.
Ausbeute: 48 % der Theorie,
$C_{29}H_{31}N_7O_5$ (557.6)
$R_f$-Wert: 0.62 (Kieselgel; Dichlormethan/Methanol = 9:1)
EKA-Massenspektrum: $(M+H)^+$ = 558
$(M+H+Na)^{++}$ = 290.7
$(M+Na)^+$ = 580

Beispiel 104

1-Methyl-2-[N-[4-(N-n-octyloxycarbonylamidino)phenyl]-aminomethyl)-benzimidazol-5-yl-carbonsäure-N-(2-pyridyl) -N-(2-hydroxycarbonylethyl)-amid

0.7 g (1.1 mMol) 1-Methyl-2-[N-[4-(N-n-octyloxycarbonylamidino)-phenyl]-aminomethyl]-benzimidazol-5-yl-carbonsäure-N-(2-pyridyl)-N-(2-methoxycarbonylethyl)-amid wurde in einer Mischung aus 0.12 g (3.0 mMol) Natriumhydroxid, 5 ml Wasser und 10 ml Methanol eine Stunde lang bei Raumtemperatur gerührt. Dann wurde mit 20 ml Wasser verdünnt und mit Eisessig auf pH 6 gestellt. Es wurden dann ca. 5 ml Diethylether zugesetzt und eine Stunde lang heftig gerührt. Das dabei ausgefallene Produkt wurde abgesaugt, mit wenig Wasser, dann mit Diethylether gewaschen und getrocknet.
Ausbeute: 80 % der Theorie,
$C_{34}H_{41}N_7O_5$ (627.8)
EKA-Massenspektrum: $(M+H)^+ = 628$
$(M+H+Na)^{++} = 325.7$
$(M+Na)^+ = 650$
$(M+2Na)^{++} = 337.7$

Beispiel 105

1-Methyl-2-[N-[4-[N-(2-methylsulfonyl-ethyloxycarbonyl)amidino]-phenyl]-aminomethyl]-benzimidazol-5-yl-carbonsäure-N-(2-pyridyl)-N-(2-ethoxycarbonylethyl)-amid

Hergestellt analog Beispiel 90 aus 1-Methyl-2-[N-(4-amidinophenyl)-aminomethyl]-benzimidazol-5-yl-carbonsäure-N-(2-pyridyl)-N-(2-ethoxycarbonylethyl)-amid-hydrochlorid und Chlorameisensäure-2-(methylsulfonyl)-ethylester.
Ausbeute: 65 % der Theorie,
$C_{31}H_{35}N_7O_7S$ (649.7)
$R_f$-Wert: 0.54 (Kieselgel; Dichlormethan/Methanol = 9:1)
EKA-Massenspektrum: $(M+H)^+ = 650$
$(M+H+Na)^{++} = 336.6$
$(M+Na)^+ = 672$
$(M+2Na)^{++} = 347.6$

Beispiel 106

1-Methyl-2-[N-[4-(N-n-butyloxycarbonylamidino)phenyl]-aminomethyl]-benzimidazol-5-yl-carbonsäure-N-(2-pyridyl) -N-(2-methoxycarbonylethyl)-amid

Hergestellt analog Beispiel 90 aus 1-Methyl-2-[N-(4-amidinopheyl)-aminomethyl]-benzimidazol-5-yl-carbonsäure-N-(2-pyridyl)-N-(2-methoxycarbonylethyl)-amid-hydrochlorid und Chlorameisensäure-n-butylester.
Ausbeute: 30 % der Theorie,
$C_{31}H_{35}N_7O_5$ (585.7)
$R_f$-Wert: 0.62 (Kieselgel; Dichlormethan/Methanol = 9:1)
EKA-Massenspektrum: $(M+H)^+ = 586$
$(M+H+Na)^{++} = 304.7$
$(M+2H)^{++} = 293.7$

Beispiel 107

1-Methyl-2-[N-[4-(N-n-hexyloxycarbonylamidino)phenyl]-aminomethyl]-benzimidazol-5-yl-carbonsäure-N-(2-pyridyl) -N-(2-methoxycarbonylethyl)-amid

Hergestellt analog Beispiel 90 aus 1-Methyl-2-[N-(4-amidinophenyl)-aminomethyl]-benzimidazol-5-yl-carbonsäure-N-(2-pyridyl)-N-(2-methoxycarbonylethyl)-amid-hydrochlorid und Chlorameisensäure-n-hexylester.
Ausbeute: 51 % der Theorie,
$C_{33}H_{39}N_7O_5$ (613.7)
$R_f$-Wert: 0.56 (Kieselgel; Dichlormethan/Methanol = 9:1)

EKA-Massenspektrum: $(M+H)^+$ = 614
$(M+H+Na)^{++}$ = 318.7
$(M+2H)^{++}$ = 307.6

Beispiel 108

1-Methyl-2-[N-[4-(N-n-heptyloxycarbonylamidino)-phenyl]-aminomethyl]-benzimidazol-5-yl-carbonsäure-N-(2-pyridyl)-N-(2-methoxycarbonylethyl)-amid

Hergestellt analog Beispiel 90 aus 1-Methyl-2-[N-(4-amidinophenyl)-aminomethyl]-benzimidazol-5-yl-carbonsäure-N-(2-pyridyl)-N-(2-methoxycarbonylethyl)-amid-hydrochlorid und Chlorameisensäure-n-heptylester.
Ausbeute: 21 % der Theorie,
$C_{34}H_{41}N_7O_5$ (627.8)
$R_f$-Wert: 0.60 (Kieselgel; Dichlormethan/Methanol = 9:1)
EKA-Massenspektrum: $(M+H)^+$ = 628
$(M+H+Na)^{++}$ = 325.7
$(M+2H)^{++}$ = 314.7

Beispiel 109

1-Methyl-2-[N-[4-(N-n-pentyloxycarbonylamidino)-phenyl]-aminomethyl]-benzimidazol-5-yl-carbonsäure-N-(2-pyridyl)-N-(2-methoxycarbonylethyl)-amid

Hergestellt analog Beispiel 90 aus 1-Methyl-2-[N-(4-amidinophenyl)-aminomethyl]-benzimidazol-5-yl-carbonsäure-N- (2-pyridyl)-N-(2-methoxycarbonylethyl)-amid-hydrochlorid und Chlorameisensäure-n-pentylester.
Ausbeute: 66 % der Theorie,
$C_{32}H_{37}N_7O_5$ (599.7)
$R_f$-Wert: 0.58 (Kieselgel; Dichlormethan/Methanol = 9:1)
EKA-Massenspektrum: $(M+H)^+$ = 600
$(M+H+Na)^{++}$ = 311.7
$(M+Na)^+$ = 622

Beispiel 110

1-Methyl-2-[N-[4-(N-n-nonyloxycarbonylamidino)phenyl]-aminomethyl]-benzimidazol-5-yl-carbonsäure-N-(2-pyridyl)-N-(2-methoxycarbonylethyl)-amid

Hergestellt analog Beispiel 90 aus 1-Methyl-2-[N-(4-amidinophenyl)-aminomethyl]-benzimidazol-5-yl-carbonsäure-N-(2-pyridyl)-N-(2-methoxycarbonylethyl)-amid-hydrochlorid und Chlorameisensäure-n-nonylester.
Ausbeute: 60 % der Theorie,
$C_{36}H_{45}N_7O_5$ (655.8)
$R_f$-Wert: 0.48 (Kieselgel; Dichlormethan/Methanol = 9:1)
EKA-Massenspektrum: $(M+H)^+$ = 656
$(M+H+Na)^{++}$ = 339.8
$(M+Na)^+$ = 678

Beispiel 111

1-Methyl-2-[N-[4-(N-benzoylamidino)phenyl]-aminomethyl]-benzimidazol-5-yl-carbonsäure-N-(2-pyridyl)-N-(2-methoxycarbonylethyl)-amid

Hergestellt analog Beispiel 90 aus 1-Methyl-2-[N-(4-amidinophenyl)-aminomethyl]-benzimidazol-5-yl-carbonsäure-N- (2-pyridyl)-N-(2-methoxycarbonylethyl)-amid-hydrochlorid und Benzoylchlorid.
Ausbeute: 62 % der Theorie,
$C_{33}H_{31}N_7O_4$ (589.7)
$R_f$-Wert: 0.50 (Kieselgel; Dichlormethan/Methanol = 9:1)
EKA-Massenspektrum: $(M+H)^+$ = 590
$(M+Na)^+$ = 612

Beispiel 112

1-Methyl-2-[N-[4-(N-nicotinoylamidino)phenyl]aminomethyl]-benzimidazol-5-yl-carbonsäure-N-(2-pyridyl)-N-(2-methoxycarbonylethyl)-amid

Hergestellt analog Beispiel 90 aus 1-Methyl-2-[N-(4-amidinophenyl)-aminomethyl]-benzimidazol-5-yl-carbonsäure-N-(2-pyridyl)-N-(2-methoxycarbonylethyl)-amid-hydrochlorid und Nicotinsäurechlorid.
Ausbeute: 40 % der Theorie,
$C_{32}H_{30}N_8O_4$ (590.7)
$R_f$-Wert: 0.47 (Kieselgel; Dichlormethan/Methanol = 9:1)
EKA-Massenspektrum: $(M+H)^+$ = 591
$(M+H+Na)^{++}$ = 307
$(M+Na)^+$ = 613

Beispiel 113

1-Methyl-2-[N-[4-(N-n-hexyloxycarbonylamidino)phenyl]-aminomethyl]-benzimidazol-5-yl-carbonsäure-N-(2-pyridyl) -N-(2-ethoxycarbonylethyl)-amid

Hergestellt analog Beispiel 90 aus 1-Methyl-2-[N-(4-amidinophenyl)-aminomethyl]-benzimidazol-5-yl-carbonsäure-N-(2-pyridyl)-N-(2-ethoxycarbonylethyl) -amid-hydrochlorid und Chlorameisensäure-n-hexylester.
Ausbeute: 51 % der Theorie,
$C_{34}H_{41}N_7O_5$ (627.8)
$R_f$-Wert: 0.53 (Kieselgel; Dichlormethan/Methanol = 9:1)
EKA-Massenspektrum: $(M+H)^+$ = 628
$(M+H+Na)^{++}$ = 325.7
$(M+2H)^{++}$ = 314.7

Beispiel 114

1-Methyl-2-[N-[4-(N-n-octyloxycarbonylamidino)phenyl]-aminomethyl]-benzimidazol-5-yl-carbonsäure-N-(2-pyridyl) -N-(2-ethoxycarbonylethyl)-amid

Hergestellt analog Beispiel 90 aus 1-Methyl-2-[N-(4-amidinophenyl)-aminomethyl]-benzimidazol-5-yl-carbonsäure-N-(2-pyridyl)-N-(2-ethoxycarbonylethyl)-amid-hydrochlorid und Chlorameisensäure-n-octylester.
Ausbeute: 57 % der Theorie,
$C_{36}H_{45}N_7O_5$ (655.8)
$R_f$-Wert: 0.46 (Kieselgel; Dichlormethan/Methanol = 9:1 )
EKA-Massenspektrum: $(M+H)^+$ = 656
$(M+H+Na)^{++}$ = 339.7
$(M+2H)^{++}$ = 328.7

Beispiel 115

1-Methyl-2-[N-[4-[N-(2-methylsulfonyl-ethyloxycarbonyl)amidino] -phenyl]-aminomethyl]-benzimidazol-5-yl-carbonsäure-N- (2-pyridyl)-N-ethoxycarbonylmethyl-amid

Hergestellt analog Beispiel 90 aus 1-Methyl-2-[N-(4-amidinophenyl)-aminomethyl]-benzimidazol-5-yl-carbonsäure-N-(2-pyridyl)-N-ethoxycarbonylmethyl-amid-hydrochlorid und Chlorameisensäure-2-(methylsulfonyl)-ethylester.
Ausbeute: 72 % der Theorie,
$C_{30}H_{33}N_7O_7S$ (635.7)
$R_f$-Wert: 0.23 (Kieselgel; Dichlormethan/Ethanol = 19:1)
EKA-Massenspektrum: $(M+H)^+$ = 636
$(M+H+Na)^{++}$ = 329.8

Beispiel 116

1-Methyl-2-[N-[4-(N-cyclohexyloxycarbonylamidino)-phenyl]aminomethyl]-benzimidazol-5-yl-carbonsäure-N- (2-pyridyl)-N-methoxycarbonylmethyl-amid

Hergestellt analog Beispiel 90 aus 1-Methyl-2-[N-(4-amidinophenyl)-aminomethyl]-benzimidazol-5-yl-carbonsäure-N-(2-pyridyl)-N-methoxycarbonylmethyl-amid-hydrochlorid und Chlorameisensäure-cyclohexylester.
Ausbeute: 40 % der Theorie,
$C_{32}H_{35}N_7O_5$ (597.7)
$R_f$-Wert: 0.26 (Kieselgel; Dichlormethan/Ethanol = 19:1)
EKA-Massenspektrum: $(M+H)^+$ = 598
$(M+Na)^+$ = 620

Beispiel 117

1-Methyl-2- [N-[4-(N-methoxycarbonylamidino)-phenyl]-aminomethyl]-benzimidazol-5-yl-carbonsäure-N-(2-pyridyl) -N-ethoxycarbonylmethyl-amid

Hergestellt analog Beispiel 90 aus 1-Methyl-2-[N-(4-amidinophenyl)-aminomethyl]-benzimidazol-5-yl-carbonsäure-N-(2-pyridyl)-N-ethoxycarbonylmethyl-amid-hydrochlorid und Chlorameisensäuremethylester.
Ausbeute: 62 % der Theorie,
$C_{28}H_{29}N_7O_5$ (543.6)
$R_f$-Wert: .0.19 (Kieselgel; Dichlormethan/Ethanol = 19:1)
EKA-Massenspektrum: $(M+H)^+$ = 544
$(M+H+Na)^{++}$ = 283.8
$(M+Na)^+$ = 566

Beispiel 118

1-Methyl-2-[N-[4-(N-ethoxycarbonylamidino)-phenyl]-aminomethyl]-benzimidazol-5-yl-carbonsäure-N-(2-pyridyl)-N-methoxycarbonylmethyl-amid

Hergestellt analog Beispiel 90 aus 1-Methyl-2-[N-(4-amidinophenyl)-aminomethyl]-benzimidazol-5-yl-carbonsäure-N-(2-pyridyl)-N-methoxycarbonylmethyl-amid-hydrochlorid und Chlorameisensäureethylester.
Ausbeute: 42 % der Theorie,
$C_{28}H_{29}N_7O_5$ (543.6)
$R_f$-Wert: 0.20 (Kieselgel; Dichlormethan/Ethanol = 19:1)
EKA-Massenspektrum: $(M+H)^+$ = 544

Beispiel 119

1-Methyl-2-[N-[4-(N-n-octyloxycarbonyl-amidino)-phenyl]aminomethyl]-benzimidazol-5-yl-carbonsäure-N-(3-pyridyl) -N-(2-ethoxycarbonylethyl)-amid

Hergestellt analog Beispiel 90 aus 1-Methyl-2-[N-(4-amidinophenyl)-aminomethyl]-benzimidazol-5-yl-carbonsäure-N-(3-pyridyl)-N-(2-ethoxycarbonylethyl)-amid-hydrochlorid und Chlorameisensäure-n-octylester.
Ausbeute: 35 % der Theorie,
$C_{36}H_{45}N_7O_5$ (655.8)
$R_f$-Wert: 0.28 (Kieselgel; Dichlormethan/Ethanol = 19:1)
EKA-Massenspektrum: $(M+H)^+$ = 656
$(M+2H)^{++}$ = 328.7

Beispiel 120

1-Methyl-2-[N-[4-(N-n-hexyloxycarbonylamidino)-phenyl]-N-methyl-aminomethyl]-benzimidazol-5-yl-carbonsäure-N-(2-pyridyl)-N-(2-ethoxycarbonylethyl)-amid

Hergestellt analog Beispiel 90 aus 1-Methyl-2-[N-(4-amidinophenyl)-N-methyl-aminomethyl]-benzimidazol-5-yl-carbonsäure-N-(2-pyridyl)-N-(2-ethoxycarbonylethyl)-amid-hydrochlorid und Chlorameisensäure-n-hexylester.
Ausbeute: 58 % der Theorie,
$C_{35}H_{43}N_7O_5$ (641.2)
$R_f$-Wert: 0.42 (Kieselgel; Dichlormethan/Ethanol = 19:1)
EKA-Massenspektrum: $(M+H)^+ = 642$
$(M+H+Na)^{++} = 332.7$

Beispiel 121

1-Methyl-2-[N-[4-(N-n-octyloxycarbonylamidino)-phenyl]-N-methyl-aminomethyl]-benzimidazol-5-yl-carbonsäure-N-(2-pyridyl)-N-(2-ethoxycarbonylethyl)-amid

Hergestellt analog Beispiel 90 aus 1-Methyl-2-[N-(4-amidinophenyl)-N-methyl-aminomethyl]-benzimidazol-5-yl-carbonsäure-N-(2-pyridyl)-N-(2-ethoxycarbonylethyl)-amid-hydrochlorid und Chlorameisensäure-n-octylester.
Ausbeute: 36 % der Theorie,
$C_{37}H_{47}N_7O_5$ (669.8)
EKA-Massenspektrum: $(M+H)^+ = 670$
$(M+H+Na)^{++} = 346.8$
$(M+2H)^{++} = 335.6$

Beispiel 122

1-Methyl-2-[N-[4-(N-n-butyloxycarbonylamidino)-phenyl]-N-methyl-aminomethyl]-benzimidazol-5-yl-carbonsäure-N-(2-pyridyl)-N-(2-ethoxycarbonylethyl)-amid

Hergestellt analog Beispiel 90 aus 1-Methyl-2-[N-(4-amidinophenyl)-N-methyl-aminomethyl]-benzimidazol-5-yl-carbonsäure-N-(2-pyridyl)-N-(2-ethoxycarbonylethyl)-amid-hydrochlorid und Chlorameisensäure-n-butylester.
Ausbeute: 34 % der Theorie,
$C_{33}H_{39}N_7O_5$ (613.7)
EKA-Massenspektrum: $(M+H)^+ = 614$
$(M+H+Na)^{++} = 318.7$
$(M+Na)^+ = 636$

Beispiel 123

1-Methyl-2-[N-[4-(N-benzoylamidino)phenyl]-N-methyl-aminomethyl]-benzimidazol-5-yl-carbonsäure-N-(2-pyridyl)-N-(2-ethoxycarbonylethyl)-amid

Hergestellt analog Beispiel 90 aus 1-Methyl-2-[N-(4-amidinophenyl)-N-methyl-aminomethyl]-benzimidazol-5-yl-carbonsäure-N-(2-pyridyl)-N-(2-ethoxycarbonylethyl)-amid-hydrochlorid und Benzoylchlorid.
Ausbeute: 63 % der Theorie,
$C_{35}H_{35}N_7O_4$ (617.7)
EKA-Massenspektrum: $(M+H)^+ = 618$
$(M+H+Na)^{++} = 320.7$
$(M+Na)^+ = 640$

Beispiel 124

1-Methyl-2-[(4-amidinophenyl)oxymethyl]-benzimidazol-5-yl-(1-ethoxycarbonylmethyl-cyclohex-1-yl)-keton-hydrochlorid

a) 4-Chlorphenyl-(1-hydroxycarbonylmethyl-cyclohex-1-yl)-keton

8.4 g (40 mMol) 3-(4-Chlorbenzoyl)-propionsäure wurden in 300 ml Tetrahydrofuran gelöst und portionsweise 5.8 g (120 mMol) Natriumhydrid (50-60%ige Suspension in Paraffinöl) zugesetzt. Dann wurde 1.5 Stunden lang unter Rühren zum Rückfluß erhitzt, dann 8.9 ml (60 mMol) 1,5-Dijodpentan zugetropft und weitere drei Stunden gekocht. Nach dem Abkühlen wurde die Lösung in 200 ml Eiswasser eingerührt, dann das Tetrahydrofuran im Vakuum abdestilliert, die so erhaltene wässrige Lösung mit 2n Salzsäure angesäuert und dreimal mit je 150 ml Dichlormethan extrahiert. Die organische Phase wurde getrocknet und eingeengt, das so erhaltene Rohprodukt durch Säulenchromatographie (500 g Kieselgel; Laufmittel: Dichlormethan mit 1-2% Ethanol) gereinigt.
Ausbeute: 6.2 g (55% der Theorie) öliges Produkt,
$C_{15}H_{17}ClO_3$ (280.8)
$R_f$-Wert: 0.56 (Kieselgel; Dichlormethan/Ethanol = 19:1)

b) 4-Chlor-3-nitrophenyl-(1-hydroxycarbonylmethyl-cyclohex-1-yl)-keton

7.0 g (25 mMol) 4-Chlorphenyl-(1-hydroxycarbonylmethyl-cyclohex-1-yl)-keton wurden unter Rühren bei -5 bis -10°C portionsweise in 80 ml rauchende Salpetersäure eingetragen. Anschließend wurde noch 10 Minuten lang nachgerührt, dann die Lösung in 200 ml Eiswasser eingerührt, das ausgefallene Produkt mit Wasser gewaschen und getrocknet.
Ausbeute: 7.8 g (96% der Theorie),
$C_{15}H_{16}ClNO_5$ (325.8)
$R_f$-Wert: 0.41 (Kieselgel; Petrolether/Essigester = 4:6)

c) 4-Methylamino-3-nitrophenyl-(1-hydroxycarbonylmethyl-cyclohex-1-yl)-keton

7.8 g ( 23.9 mMol) 4-Chlor-3-nitrophenyl-(1-hydroxycarbonylmethyl-cyclohex-1-yl)-keton wurden in 100 ml einer 40%igen wässrigen Methylaminlösung bei Raumtemperatur 14 Stunden lang gerührt, dann mit ca. 150 ml Wasser verdünnt und mit Eisessig schwach sauer gestellt. Das ausgefallene Produkt wurde abgesaugt, mit Wasser gewaschen und getrocknet.
Ausbeute: 7.1 g (93% der Theorie),
$C_{16}H_{20}N_2O_5$ (320.4)
$R_f$-Wert: 0.34 (Kieselgel; Dichlormethan/Ethanol = 19:1)

d) 4-Methylamino-3-nitrophenyl-(1-methoxycarbonylmethyl-cyclohex-1-yl)-keton

4.9 g (15 mMol) 4-Methylamino-3-nitrophenyl-(1-hydroxycarbonylmethyl-cyclohex-1-yl)-keton wurden in 100 ml Tetrahydrofuran gelöst, 2.4 g (15 mMol) 1,1'-Carbonyl-diimidazol zugegeben und 15 Minuten lang zum Rückfluß erhitzt. Danach wurde das Lösungsmittel abgedampft, 30 ml Methanol hinzugefügt und unter Rühren drei Stunden lang gekocht. Nach Abdestillieren des Methanols wurde das so erhaltene Rohprodukt durch Säulenchromatographie (250 g Kieselgel, Laufmittel: Dichlormethan mit 1 bis 5% Ethanol) gereinigt.
Ausbeute: 2.4 g (48% der Theorie),
$C_{17}H_{22}N_2O_5$ (334.4)
$R_f$-Wert: 0.76 (Kieselgel; Dichlormethan/Ethanol = 19:1)

e) 3-Amino-4-methylaminophenyl-(1-methoxycarbonylmethyl-cyclohex-1-yl)-keton

2.4 g (7.2 mMol) 4-Methylamino-3-nitrophenyl-(1-methoxycarbonylmethyl-cyclohex-1-yl)-keton wurden in 100 ml Methanol bei Raumtemperatur und 5 bar Wasserstoffdruck katalytisch hydriert (10% Palladium auf Kohle). Das so erhaltene Rohprodukt wurde ohne Reinigung weiter umgesetzt.
Ausbeute: 2.1 g (96% der Theorie),
$R_f$-Wert: 0.34 (Kieselgel; Dichlormethan/Ethanol = 19:1)

f) 3-(4-Cyanophenyloxyacetylamino)-4-methylaminophenyl-(1-methoxycarbonylmethyl-cyclohex-1-yl)-keton

620 mg (3.5 mMol) 4-Cyanophenyloxyessigsäure und 570 mg (3.5 mMol) 1,1'-Carbonyl-diimidazol wurden in 50 ml Tetrahydrofuran 15 Minuten lang zum Rückfluß erhitzt. Dann wurde 1.0 g (3.28 mMol) 3-Amino-4-methylaminophenyl-(1-methoxycarbonylmethyl-cyclohex-1-yl)-keton hinzugefügt und weitere 4 Stunden lang gekocht.Anschließend wurde das Lösungsmittel abgedampft und das so erhaltene Rohprodukt durch Säulenchromatographie (150 g Kieselgel; Laufmittel: Dichlormethan mit 0 bis 2% Ethanol) gereinigt.
Ausbeute: 1.4 g (93% der Theorie),
$C_{26}H_{29}N_3O_5$ (463.5)
$R_f$-Wert: 0.44 (Kieselgel; Dichlormethan/Ethanol = 19:1)

g) 1-Methyl-2-[(4-cyanophenyl)oxymethyl]-benzimidazol-5-yl-(1-methoxycarbonylmethyl-cyclohex-1-yl)-keton

1.4 g (3.02 mMol) 3-(4-Cyanophenyloxyacetylamino)-4-methylaminophenyl-(1-methoxycarbonylmethyl-cyclohex-1-yl)-keton wurden in 50 ml Eisessig eine Stunde lang zum Rückfluß erhitzt.Dann wurde der Eisessig abdestilliert, der Rückstand mit 20 ml Wasser versetzt und mit konzentriertem Ammoniak alkalisch gestellt. Diese Lösung wurde dreimal mit je 20 ml Dichlormethan extrahiert, die organischen Extrakte getrocknet und eingeengt. Das so erhaltene Rohprodukt wurde durch Säulenchromatographie (100 g Kieselgel; Laufmittel: Dichlormethan mit 0 bis 2% Ethanol) gereinigt.
Ausbeute: 700 mg (52% der Theorie),
$C_{26}H_{27}N_3O_4$ (445.5)

h) 1-Methyl-2-[(4-amidinophenyl)oxymethyl]-benzimidazol-5-yl-(1-ethoxycarbonylmethyl-cyclohex-1-yl)-keton-hydrochlorid

Hergestellt analog Beispiel 25d aus 700 mg (1.57 mMol) 1-Methyl-2-(4-cyanophenyloxymethyl)-benzimidazol-5-yl-(1-methoxycarbonylmethyl-cyclohex-1-yl)-keton mit ethanolischer Salzsäure und Ammoniumcarbonat.
Ausbeute: 390 mg (50% der Theorie),
$C_{27}H_{32}N_4O_4$ (476.6)
EKA-Massenspektrum: $(M+H)^+$ = 477
$^1$H-NMR-Spektrum ($d_6$-DMSO): 1.10 (t,3H); 1.0-2.15 (m,10H); 3.36 (s,3H); 3.90 (s,2H); 3.94 (q,2H); 5.60 (s,2H); 7.25-7.40 (m,3H); 7.56-7.75 (m,2H); 7.90 (d,2H); 9.20 (breites s,4H) ppm.

Beispiel 125

1-Methyl-2-[N-(4-amidinophenyl)-aminomethyl]-benzimidazol-5-yl-tert.butyl-keton-hydrochlorid

Hergestellt analog Beispiel 25d aus 1-Methyl-2-[N-(4-cyanophenyl)-aminomethyl]-benzimidazol-5-yl-tert.butyl-keton, ethanolischer Salzsäure, Ethanol und Ammoniumcarbonat.
Ausbeute: 59 % der Theorie,
$C_{21}H_{25}N_5O$ (363.5)
EKA-Massenspektrum: $(M+H)^+$ = 364

Beispiel 126

1-Methyl-2-[N-(4-amidinophenyl)-aminomethyl]-benzimidazol-5-yl-(1-methylcyclopent-1-yl)-keton-hydrochlorid

Hergestellt analog Beispiel 25d aus 1-Methyl-2-[N-(4-cyanophenyl)-aminomethyl]-benzimidazol-5-yl-(1-methylcyclopent-1-yl)-keton, ethanolischer Salzsäure, Ethanol und Ammoniumcarbonat.
Ausbeute: 63.5 % der Theorie,
$C_{23}H_{27}N_5O$ (389.5)
EKA-Massenspektrum: $(M+H)^+$ = 390

Beispiel 127

2-[(4-Amidinophenyl)sulfinylmethyl]-benzthiazol-5-yl-carbonsäure-N-phenyl-N-(2-ethoxycarbonylethyl)-amid-hydrochlorid

Eine Lösung aus 0.15 g (0.27 mMol) 2-[(4-Amidinophenyl)thiomethyl]-benzthiazol-5-yl-carbonsäure-N-phenyl-N-(2-ethoxycarbonylethyl)-amid-hydrochlorid in 10 ml Essigsäure wurde mit 0.09 ml (ca. 0.81 mMol) 30%iger Wasserstoffperoxidlösung versetzt und bei Raumtemperatur gerührt. Nach 4 Tagen gab man weitere 0.18 ml Wasserstoffperoxidlösung zu und rührte weitere zwei Tage nach. Nach dem Entfernen des Lösungsmittels im Vakuum wurde das erhaltene Rohprodukt durch Flash-Chromatographie (Kieselgel; Methylenchlorid/Ethanol = 10:1 bis 4:1) gereinigt.
Ausbeute: 58 % der Theorie,
$C_{27}H_{26}N_4O_4S_2$ (534.66)
$R_f$-Wert: 0.24 (Kieselgel; Methylenchlorid/Ethanol = 4:1 + einige Tropfen Essigsäure)
EKA-Massenspektrum: $(M+H)^+$ = 535

Beispiel 128

1-Methyl-2-[(4-amidinophenyl)sulfonylmethyl]-benzimidazol-5-yl-carbonsäure-N-(n-propyl)-N-(2-ethoxycarbonylethyl)-amid-hydrochlorid

Eine Lösung aus 0.40 g (0.70 mMol) 1-Methyl-2-[(4-amidinophenyl)thiomethyl]-benzimidazol-5-yl-carbonsäure-N-(n-propyl)-N-(2-ethoxycarbonylethyl)-amid-hydrochlorid in 10 ml Ameisensäure wurde mit 2 ml 30%iger Wasserstoffperoxid-Lösung versetzt und 16 Stunden bei Raumtemperatur gerührt. Anschließend wurde das Lösungsmittel im Vakuum abdestilltiert, wobei die gewünschte Verbindung als beigefarbener Feststoff (mit etwas 1-Methyl-2-[(4-amidinophenyl)sulfinylmethyl]-benzimidazol-5-yl-carbonsäure-N-(n-propyl)-N-(2-ethoxycarbonylethyl)-amid-hydrochlorid verunreinigt) erhalten wurde.
Ausbeute: 95 % der Theorie,
$C_{25}H_{31}N_6O_5S$ (513.62)
$R_f$-Wert: 0.50 (Kieselgel; Essigester/Ethanol/IN Salzsäure = 50:45:5)
EKA-Massenspektrum: (M+H)+ = 514

Beispiel 129

2-[N-(4-Amidinophenyl)-aminomethyl]-thiazolo[5,4-b]pyridin-6-yl-carbonsäure-N-phenyl-N-(2-ethoxycarbonylethyl)-amid-hydrochlorid

a) 5-Amino-6-chlor-nicotinsäuremethylester

Eine Lösung von 1.08 g (5.00 mMol) 6-Chlor-5-nitro-nicotinsäuremethylester (siehe A.H. Berrie, G.T. Newbold, F.S. Spring in J. Chem. Soc., 2590, 1951) in 25 ml absolutem Ethanol wurde sukzessiv mit 0.53 ml (29 mMol) Wasser, 3.2 g (57 mMol) Eisenpulver und 0.030 ml konzentrierter Salzsäure versetzt und eine Stunde zum Sieden erhitzt. Anschließend wurden nochmals gleiche Mengen an Wasser, Eisenpulver und Salzsäure zugegeben und 30 Minuten zum Sieden erhitzt. Der beim Abkühlen ausfallende Niederschlag wurde abfiltriert, mit Ethanol gewaschen und das Lösungsmittel wurde im Vakuum abdestilliert. Ausbeute: 0.75 g (81% der Theorie) gelbgrüner Feststoff,
Rf-Wert: 0.31 (Kieselgel;Essigester/Petrolether = 1:4)
$C_7H_7ClN_2O_2$ (186.60)
YEF-Massenspektrum: $M^+$ = 186 und 188 (Chlorisotope).

b) 6-Chlor-5-methoxyacetamido-nicotinsäuremethylester

Eine Lösung aus 0.75 g (4.02 mMol) 5-Amino-6-chlor-nicotinsäuremethylester und 0.43 g = 0.35 ml (4.5 mMol) Methoxyacetylchlorid in 20 ml Chlorbenzol wurde eine Stunde bei 110°C gerührt. Nach dem Entfernen des Lösungsmittels im Vakuum wurde das erhaltene Rohprodukt durch Flash-Chromatographie (Kieselgel; Methylenchlorid/Ethanol = 100:1) gereinigt, erneut im Vakuum eingeengt und anschließend mit Petrolether digeriert. Ausbeute: 0.55 g (53% der Theorie) hellgelber amorpher Feststoff,
$R_f$-Wert: 0.33 (Kieselgel; Essigester/Petrolether = 1:4)

c) 2-Methoxymethyl-thiazolo[5,4-b]pyridin-6-yl-carbonsäuremethylester

Ein Gemisch aus 0.53 g (2.05 mMol) 6-Chlor-5-methoxyacetamidonicotinsäuremethylester und 0.42 g (1.0 mMol) Lawessons Reagenz wurde 16 Stunden in 25 ml Xylol unter Rückfluß erhitzt. Nach dem Entfernen des Lösungsmittels im Vakuum wurde das erhaltene Rohprodukt durch Flash-Chromatographie (Kieselgel; Methylenchlorid/Ethanol = 100:1) gereinigt und erneut im Vakuum eingeengt.
Ausbeute: 0.33 g (67% der Theorie) gelber amorpher Feststoff,
$R_f$-Wert: 0.52 (Kieselgel; Essigester/Petrolether = 1:4)

d) 2-Methoxymethyl-thiazolo[5,4-b]pyridin-6-yl-carbonsäure

Ein Gemisch aus 1.1 g (4.62 mMol) 2-Methoxymethyl-thiazolo-[5,4-b]pyridin-6-carbonsäuremethylester und 9.2 ml 2N Natronlauge wurden in 50 ml Ethanol eine Stunde bei Raumtemperatur gerührt. Danach wurden 9.2 ml 2N Salzsäure zugegeben, der Alkohol wurde abdestilliert, und es wurde mit 20 ml Wasser verdünnt. Die wäßrige Phase wurde mit konzentrierter Salzsäure unter Eiskühlung angesäuert, der daraufhin ausfallende beigefarbige Niederschlag abfiltriert, anschließend mit Wasser gewaschen und getrocknet.

Ausbeute: 1.03 g (100% der Theorie),
$R_f$-Wert: 0.10 (Kieselgel; Essigester/Petrolether = 3:7)

e) 2-Methoxymethyl-thiazolo[5,4-b]pyridin-6-yl-carbonsäure-N-phenyl-N-(2-ethoxycarbonylethyl)-amid

Eine Suspension aus 1.03 g (4.62 mMol) 2-Methoxymethyl-thiazolo[5,4-b]pyridin-6-yl-carbonsäure in 40 ml Methylenchlorid wurde mit 1.6 g = 1.0 ml (13.5 mMol) Thionylchlorid versetzt und 90 Minuten am Rückfluß gekocht, dabei löste sich der Feststoff allmählich auf. Nach Abdestillieren der flüssigen Komponenten wurde das Rohprodukt noch zweimal in Methylenchlorid aufgenommen und nochmals konzentriert. Das so erhaltene rohe Säurechlorid (1.2 g) wurde in 40 ml Tetrahydrofuran aufgenommen, zu einem Gemisch aus 0.94 g (4.86 mMol) N-(2-Ethoxycarbonylethyl) anilin und 2.1 ml (13.8 mMol) Triethylamin in 30 ml Tetrahydrofuran getropft und 2 Stunden bei Raumtemperatur gerührt. Anschließend wurde mit 200 ml Essigester verdünnt, mit 100 ml 14%iger Kochsalz-Lösung gewaschen und die organische Phase mit Natriumsulfat getrocknet. Nach dem Entfernen des Lösungsmittels im Vakuum wurde das erhaltene Rohprodukt durch Flash-Chromatographie (Kieselgel; Methylenchlorid/Ethanol = 100:1) gereinigt.
Ausbeute: 1.57 g (87% der Theorie) gelbes Öl,
$R_f$-Wert: 0.55 (Kieselgel; Methylenchlorid/Ethanol = 19:1)

f) 2-[N-(4-Cyanophenyl)-aminomethyl]-thiazolo[5,4-b]pyridin-6-yl-carbonsäure-N-phenyl-N-(2-ethoxycarbonylethyl)-amid

Ein Gemisch aus 1.54 g (3.85 mMol) 2-Methoxymethyl-thiazolo-[5,4-b]pyridin-6-yl-carbonsäure-N-phenyl-N-(2-ethoxycarbonylethyl)-amid und 4.3 ml (4.3 mMol) einer 1 molaren Lösung aus Bortribromid in Methylenchlorid wurde in weiteren 30 ml Methylenchlorid gelöst und 5 Stunden bei Raumtemperatur gerührt. Anschließend wurde mit 40 ml gesättigter Natriumhydrogencarbonatlösung gewaschen, die organische Phase mit Natriumsulfat getrocknet und das Lösungsmittel abdestilliert. Das Rohprodukt (1.9 g) wurde in 15.0 ml N,N-Diisopropyl-ethylamin aufgenommen, mit 0.50 g (4.2 mMol) 4-Aminobenzonitril versetzt und eine Stunde zum Sieden erhitzt. Anschließend wurde das Lösungsmittel im Vakuum abdestilliert, das Rohprodukt in 100 ml Methylenchlorid aufgenommen, die organische Phase mit 100 ml Wasser gewaschen und mit Natriumsulfat getrocknet. Nach dem Entfernen des Lösungsmittels im Vakuum wurde das erhaltene Rohprodukt durch Flash-Chromatographie (Kieselgel; Essigester/Petrolether = 35:65 bis 1:1) gereinigt und erneut im Vakuum eingeengt.
Ausbeute: 0.45 g (24% der Theorie) gelber amporpher Feststoff, $R_f$-Wert: 0.34 (Kieselgel; Essigester/Petrolether = 1:1)

g) 2-[N-(4-Amidinophenyl)-aminomethyl]-thiazolo[5,4-b]pyridin-6-yl-carbonsäure-N-phenyl-N-(2-ethoxycarbonylethyl)-amid-hydrochlorid

0.39 g (0.803 mMol) 2-[N-(4-Cyanophenyl)-aminomethyl]-thiazolo-[5,4-b]pyridin-6-yl-carbonsäure-N-phenyl-N-(2-ethoxycarbonylethyl)-amid wurden in 40 ml mit Chlorwasserstoff gesättigtem Ethanol 5 Stunden erst bei 0°C und dann bei Raumtemperatur gerührt, bis dünnschichtchromatographisch kein Ausgangsmaterial mehr nachweisbar war. Anschließend wurde das Lösungsmittel bei maximal 30°C Badtemperatur abdestilliert, der ölige Rückstand in 40 ml absolutem Ethanol aufgenommen und mit 0.5 g Ammoniumcarbonat versetzt. Nach 18 Stunden wurde das Lösungsmittels im Vakuum entfernt und das erhaltene Rohprodukt durch Flash-Chromatographie (Kieselgel; Methylenchlorid/Ethanol = 9:1 bis 4:1) gereinigt.
Ausbeute: 78 % der Theorie gelber Schaum,
$C_{26}H_{26}N_6O_3S$ (502.60)
$R_f$-Wert: 0.19 (Kieselgel; Methylenchlorid/Ethanol = 4:1 + einige Tropfen Essigsäure)
EKA-Massenspektrum: $(M+H)^+$ = 503

Beispiel 130

1-Methyl-2-[(4-amidinophenyl)methylthio]-benzimidazol-5-yl-carbonsäure-N-phenyl-N-(2-ethoxycarbonylethyl)-amid-hydrochlorid

a) 1-Methyl-2-mercapto-benzimidazol-5-yl-carbonsäure-N-phenyl-N-(2-ethoxycarbonylethyl)-amid

Eine Lösung aus 6.5 g (19 mMol) 3-Amino-4-methylamino-benzoesäure-N-phenyl-N-(2-ethoxycarbonylethyl)-amid und 4.5 g (22.8 mMol) N,N'-Thiocarbonyldiimidazol wurden unter Stickstoffatmosphäre in 100 ml Tetrahydrofuran gelöst, 4 Stunden auf 90°C erhitzt und 16 Stunden bei Raumtemperatur stehengelassen. Nach Entfernen des Lösungsmittels im Vakuum wurde das erhaltene Rohprodukt wurde durch Flash-Chromatographie (Kieselgel; Petrolether/Essigester = 100:0 bis 65:35) gereinigt.
Ausbeute: 6.8 g (93 % der Theorie) beigefarbener, kristalliner Feststoff,
$R_f$-Wert: 0.55 (Kieselgel; Essigester)

b) 1-Methyl-2-[(4-cyanophenyl)methylthio]-benzimidazol-5-yl-carbonsäure-N-phenyl-N-(2-ethoxycarbonylethyl)-amid

Eine Lösung aus 1.30 g (3.4 mMol) 1-Methyl-2-mercapto-benzimidazol-5-yl-carbonsäure-N-phenyl-N-(2-ethoxycarbonylethyl)-amid, 0.52 g (3.74 mMol) Kaliumcarbonat und 0.66 g (3.4 mMol) 4-Brommethylbenzonitril wurden in 40 ml absolutem Ethanol gelöst, 4 Stunden bei 60°C und 16 Stunden bei Raumtemperatur gerührt. Anschließend wurde das Lösungsmittel im Vakuum abdestilliert, das Rohprodukt in 30 ml Methylenchlorid aufgenommen, mit 40 ml Wasser gewaschen und mit Natriumsulfat getrocknet. Nach Filtration und Abdestillieren des Lösungsmittels fiel die gewünschte Verbindung als beige-weißer Feststoff an.
Ausbeute: 1.8 g (100 % der Theorie),
$R_f$-Wert: 0.64 (Kieselgel; Essigester)

c) 1-Methyl-2-[(4-amidinophenyl)methylthio]-benzimidazol-5-yl-carbonsäure-N-phenyl-N-(2-ethoxycarbonylethyl)-amid-hydrochlorid

1.5 g (3.0 mMol) 1-Methyl-2-[(4-cyanophenyl)methylthio]-benzimidazol-5-yl-carbonsäure-N-phenyl-N-(2-ethoxycarbonylethyl)-amid wurden in 80 ml mit Chlorwasserstoff gesättigtem Ethanol 6.5 Stunden erst bei 0°C, dann bei Raumtemperatur gerührt, bis dünnschichtchromatographisch kein Ausgangsmaterial mehr nachweisbar war. Anschließend wurde das Lösungsmittel bei maximal 30°C Badtemperatur abdestilliert, der ölige Rückstand in 80 ml absolutem Ethanol aufgenommen und mit 1.0 g (10.5 mMol) Ammoniumcarbonat versetzt. Nach 18 Stunden wurde das Lösungsmittel im Vakuum abdestilliert und das erhaltene Rohprodukt durch Flash-Chromatographie (Kieselgel; Methylenchlorid/Ethanol = 19:1 bis 10:1) gereinigt.
Ausbeute: 78 % der Theorie hellbeigefarbener Feststoff,
$C_{28}H_{29}N_5O_3S$ (515.63)
$R_f$-Wert: 0.19 (Kieselgel; Methylenchlorid/Ethanol = 4:1)
EKA-Massenspektrum: $(M+H)^+$ = 516
$(M+H+Na)^{++}$ = 269.7
$(M+2H)^{++}$ = 258.7

Beispiel 131

1-Methyl-2-[(4-amidinophenyl)methylthio]-benzimidazol-5-yl-carbonsäure-N-phenyl-N-(2-hydroxycarbonylethyl)-amid-hydrochlorid

Hergestellt analog Beispiel 10 aus 1-Methyl-2-[(4-amidinophenyl)methylthio]-benzimidazol-5-yl-carbonsäure-N-phenyl-N-(2-ethoxycarbonylethyl)-amid-hydrochlorid und Natronlauge.
Ausbeute: 57 % der Theorie,
$C_{26}H_{25}N_5O_3S$ (487.58)
$R_f$-Wert: 0.23 (Reversed Phase Kieselgel RP-8; Methanol/5%ige Kochsalz-Lösung = 6:4)
EKA-Massenspektrum: $(M+H)^+$ = 488
$(M+Na)^+$ = 510
$(M+Na+H)^{++}$ = 255.6

Beispiel 132

1-Methyl-2-[N-(4-amidinophenyl)-aminomethyl]-benzimidazol-5-yl-carbonsäure-N-propargyl-N-(2-ethoxycarbonylethyl)-amid-hydrochlorid

Hergestellt analog Beispiel 25d aus 1-Methyl-2-[N-(4-cyanophenyl)-aminomethyl]-benzimidazol-5-yl-carbonsäure-N-propargyl-N-(2-ethoxycarbonylethyl)-amid, ethanolischer Salzsäure, Ethanol und Ammoniumcarbonat.
Ausbeute: 81 % der Theorie,
$C_{25}H_{28}N_6O_3$ (460.6)
$R_f$-Wert: 0.094 (Kieselgel; Dichlormethan/Ethanol = 4:1)
EKA-Massenspektrum: $(M+H)^+$ = 461
$(M+H+Na)^{++}$ = 242

$(M+2H)^{++}$ = 231

Beispiel 133

1-Methyl-2-[2-[4-(N-n-hexyloxycarbonylamidino)phenyl]ethyl]-benzimidazol-5-yl-carbonsäure-N-(2-pyridyl) -N-(2-ethoxycarbonylethyl)-amid

Hergestellt analog Beispiel 90 aus 1-Methyl-2-[2-(4-amidinophenyl)ethyl]-benzimidazol-5-yl-carbonsäure-N-(2-pyridyl)-N-(2-ethoxycarbonylethyl)-amid-hydrochlorid und Chlorameisensäure-n-hexylester.
Ausbeute: 72 % der Theorie,
$C_{35}H_{42}N_6O_5$ (626.8)
$R_f$-Wert: 0.54 (Kieselgel; Dichlormethan/Methanol = 9:1)
EKA-Massenspektrum: $(M+H)^+$ = 627
$(M+Na)^+$ = 649

Beispiel 134

1-Methyl-2-[2-[4-(N-benzoylamidino)phenyl]ethyl]-benzimidazol-5-yl-carbonsäure-N-(2-pyridyl)-N-(2-ethoxycarbonylethyl)-amid

Hergestellt analog Beispiel 90 aus 1-Methyl-2-[2-(4-amidinophenyl)ethyl]-benzimidazol-5-yl-carbonsäure-N-(2-pyridyl)-N-(2-ethoxycarbonylethyl)-amid-hydrochlorid und Benzoylchlorid.
Ausbeute: 79 % der Theorie,
$C_{35}H_{34}N_6O_4$ (602.7)
$R_f$-Wert: 0.52 (Kieselgel; Dichlormethan/Methanol = 9:1) EKA-Massenspektrum: $(M+H)^+$ = 603
$(M+Na)^+$ = 625

Beispiel 135

1-Methyl-2-[2-[4-(N-nicotinoylamidino)phenyl]ethyl]-benzimidazol-5-yl-carbonsäure-N-(2-pyridyl)-N-(2-ethoxycarbonylethyl)-amid

Hergestellt analog Beispiel 90 aus 1-Methyl-2-[2-(4-amidinophenyl)ethyl]-benzimidazol-5-yl-carbonsäure-N-(2-pyridyl)-N-(2-ethoxycarbonylethyl)-amid-hydrochlorid und Nicotinsäurechlorid.
Ausbeute: 56 % der Theorie,
$C_{34}H_{33}N_7O_4$ (603.7)
$R_f$-Wert: 0.52 (Kieselgel; Dichlormethan/Methanol = 9:1)
EKA-Massenspektrum: $(M+H)^+$ = 604
$(M+Na)^+$ = 626

Beispiel 136

1-Cyclopropyl-2-[N-(4-amidinophenyl)-aminomethyl]-benzimidazol-5-yl-carbonsäure-N-phenyl-N-(2-ethoxycarbonylethyl)-amid-hydrochlorid

Hergestellt analog Beispiel 25d aus 1-Cyclopropyl-2-[N-(4-cyanophenyl)-aminomethyl]-benzimidazol-5-yl-carbonsäure-N-phenyl-N-(2-ethoxycarbonylethyl)-amid, ethanolischer Salzsäure, Ethanol und Ammoniumcarbonat.
Ausbeute: 31 % der Theorie,
$C_{30}H_{33}N_6O_3$ (524.6)
$R_f$-Wert: 0.40 (Kieselgel; Dichlormethan/Methanol = 5:1)
EKA-Massenspektrum: $(M+H)^+$ = 525
$(M+H+Na)^{++}$ = 274
$(M+2H)^{++}$ = 263

Beispiel 137

1-Cyclopropyl-2-[N-(4-amidinophenyl)-aminomethyl]-benzimidazol-5-yl-carbonsäure-N-phenyl-N-(2-hydroxycarbonylethyl)-amid

Hergestellt analog Beispiel 26 aus 1-Cyclopropyl-2-[N-(4-amidinophenyl)-aminomethyl]-benzimidazol-5-yl-carbonsäure-N-phenyl-N-(2-ethoxycarbonylethyl)-amid-hydrochlorid und Natronlauge.
Ausbeute: 64 % der Theorie,
$C_{28}H_{28}N_6O_3$ (496.6)
EKA-Massenspektrum: $(M+H)^+ = 497$
$(M+H+Na)^{++} = 260$
$(M+Na)^+ = 519$
$(M+2Na)^{++} = 271$

Beispiel 138

1-Methyl-2-[N-(4-amidinophenyl)-N-(n-butyl)-aminomethyl]-benzimidazol-5-yl-carbonsäure-N-phenyl-N-(2-ethoxycarbonylethyl)-amid-hydrochlorid

Hergestellt analog Beispiel 25d aus 1-Methyl-2-[N-(4-cyanophenyl)-N-(n-butyl)-aminomethyl]-benzimidazol-5-yl-carbonsäure-N-phenyl-N-(2-ethoxycarbonylethyl)-amid, ethanolischer Salzsäure, Ethanol und Ammoniumcarbonat.
Ausbeute: 62 % der Theorie,
$C_{32}H_{38}N_6O_3$ (554.7)
EKA-Massenspektrum: $(M+H)^+ = 555$
$(M+H+Na)^{++} = 289$
$(M+2H)^{++} = 278$

Beispiel 139

1-Methyl-2-[N-(4-amidino-2-chlor-phenyl)-aminomethyl]-benzimidazol-5-yl-carbonsäure-N-phenyl-N-(2-ethoxycarbonylethyl)-amid-hydrochlorid

Hergestellt analog Beispiel 25d aus 1-Methyl-2-[N-(4-cyano-2-chlor-phenyl)-aminomethyl]-benzimidazol-5-yl-carbonsäure-N-phenyl-N-(2-ethoxycarbonylethyl)-amid, ethanolischer Salzsäure, Ethanol und Ammoniumcarbonat.
Ausbeute: 82 % der Theorie,
$C_{28}H_{29}ClN_6O_3$ (533.1)
EKA-Massenspektrum: $(M+H)^+ = 533/5$
$(M+H+Na)^{++} = 278/9$

Beispiel 140

1-Methyl-2-[N-[4-(n-octyloxycarbonylamidino)phenyl]-aminomethyl]-benzimidazol-5-yl-carbonsäure-N-phenyl-N-(2-ethoxycarbonylethyl)-amid

Hergestellt analog Beispiel 90 aus 1-Methyl-2-[N-(4-amidinophenyl)-aminomethyl]-benzimidazol-5-yl-carbonsäure-N-phenyl-N-(2-ethoxycarbonylethyl)-amid-hydrochlorid und Chlorameisensäure-n-octylester.
Ausbeute: 34 % der Theorie,
$C_{37}H_{46}N_6O_5$ (654.8)
$R_f$-Wert: 0.15 (Kieselgel; Dichlormethan/Ethanol = 19:1)
EKA-Massenspektrum: $(M+H)^+ = 655$
$(M+H+Na)^{++} = 339$
$(M+Na)^+ = 677$

Beispiel 141

1-Methyl-2-[N-(4-amidino-2-ethyl-phenyl)-aminomethyl]-benzimidazol-5-yl-carbonsäure-N-phenyl-N-(2-ethoxycarbonylethyl)-amid-hydrochlorid

Hergestellt analog Beispiel 25d aus 1-Methyl-2-[N-(4-cyano-2-ethyl-phenyl)-aminomethyl]-benzimidazol-5-yl-carbonsäure-N-phenyl-N-(2-ethoxycarbonylethyl)-amid, ethanolischer Salzsäure, Ethanol und Ammoniumcarbonat.
Ausbeute: 61 % der Theorie
$C_{30}H_{34}N_6O_3$ (526.6)
EKA-Massenspektrum: $(M+H)^+ = 527$
$(M+H+Na)^{++} = 275$
$(M+2H)^{++} = 264$

Beispiel 142

1-Methyl-2-[N-(4-amidinophenyl)-aminomethyl]-benzimidazol-5-yl-carbonsäure-benzylamid-hydrochlorid

Hergestellt analog Beispiel 25d aus 1-Methyl-2-[N-(4-cyanophenyl)-aminomethyl]-benzimidazol-5-yl-carbonsäure-benzylamid, ethanolischer Salzsäure, Ethanol und Ammoniumcarbonat.
Ausbeute: 63 % der Theorie,
$C_{24}H_{24}N_6O$ (412.5)
$R_f$-Wert: 0.76 (Kieselgel; Dichlormethan/Ethanol = 4:1)
EKA-Massenspektrum: $(M+H)^+ = 413$

Beispiel 143

1-Methyl-2-[N-[4-(N-(2-(2-ethoxyethoxy)ethyloxy)-carbonylamidino)-phenyl]-aminomethyl]-benzimidazol-5-yl-carbonsäure-N-(2-pyridyl)-N-(2-ethoxycarbonylethyl)-amid

Hergestellt analog Beispiel 90 aus 1-Methyl-2-[N-(4-amidinophenyl)-aminomethyl]-benzimidazol-5-yl-carbonsäure-N-(2-pyridyl)-N-(2-ethoxycarbonylethyl)-amid-hydrochlorid und dem Chlorameisensäureester von Diethylenglycolmonoethylether.
Ausbeute: 43 % der Theorie,
$C_{34}H_{41}N_7O_7$ (659.8)
$R_f$-Wert: 0.56 (Kieselgel; Dichlormethan/Methanol = 9:1)
EKA-Massenspektrum: $(M+H)^+ = 660$
$(M+H+Na)^{++} = 341.7$

Beispiel 144

1-Methyl-2-[N-(4-amidinophenyl)-aminomethyl]-benzimidazol-5-yl-carbonsäure-N-(1-methylpyrazol-4-yl)-N-(2-ethoxycarbonylethyl)-amid-hydrochlorid

Hergestellt analog Beispiel 25d aus 1-Methyl-2-[N-(4-cyanophenyl)-aminomethyl]-benzimidazol-5-yl-carbonsäure-N-(2-pyridyl)-N-(2-ethoxycarbonylethyl)-amid, ethanolischer Salzsäure, Ethanol und Ammoniumcarbonat.
Ausbeute: 60 % der Theorie,
$C_{26}H_{30}N_8O_3$ (502.6)
$R_f$-Wert: 0.13 (Kieselgel; Dichlormethan/Ethanol = 4:1)
EKA-Massenspektrum: $(M+H)^+ = 503$
$(M+H+Na)^{++} = 263$
$(M+2H)^{++} = 252$

Beispiel 145

3-Methyl-2-[(4-amidinophenyl)-thiomethyl]-imidazo[4,5-b]-pyridin-6-yl-carbonsäure-N-phenyl-N-(2-ethoxycarbonylethyl)-amid-hydrochlorid

Hergestellt analog Beispiel 1 aus 3-Methyl-2-[(4-cyanophenyl)-thiomethyl]-imidazo[4,5-b]pyridin-6-yl-carbonsäure-N-phenyl-N-(2-methoxycarbonylethyl)-amid, ethanolischer Salzsäure, Ethanol und Ammoniumcarbonat.
Ausbeute: 88 % der Theorie,
$C_{27}H_{28}N_6O_3S$ (516.63)
$R_f$-Wert: 0.23 (Kieselgel; Essigester/Ethanol/Ammoniak = 50:45:5)
EKA-Massenspektrum: $(M+H)^+ = 517$
$(M+H+Na)^{++} = 270$

Beispiel 146

3-Methyl-2-[N-(4-amidinophenyl)-aminomethyl]-imidazo[4,5-b]pyridin-6-yl-carbonsäure-N-phenyl-N-(2-ethoxycarbonylethyl)-amid-hydrochlorid

Hergestellt analog Beispiel 1 aus 3-Methyl-2-[N-(4-cyanophenyl)-aminomethyl]-imidazo[4,5-b]pyridin-6-yl-carbonsäure-N-phenyl-N-(2-methoxycarbonylethyl)-amid, ethanolischer Salzsäure, Ethanol und Ammoniumcarbonat.
Ausbeute: 82 % der Theorie,
$C_{27}H_{29}N_7O_3$ (499.58)
$R_f$-Wert: 0.20 (Kieselgel; Essigester/Ethanol/Ammoniak = 50:45:5)
EKA-Massenspektrum: $(M+H)^+ = 500$
$(M+H+Na)^{++} = 261.7$

Beispiel 147

3-Methyl-2-[(4-amidinophenyl)-thiomethyl]-imidazo[4,5-b]pyridin-6-yl-carbonsäure-N-phenyl-N-(2-hydroxycarbonylethyl)-amid-hydrochlorid

Hergestellt analog Beispiel 2 aus 3-Methyl-2-[(4-amidinophenyl)-thiomethyl]-imidazo[4,5-b]pyridin-6-yl-carbonsäure-N-phenyl-N-(2-ethoxycarbonylethyl)-amid-hydrochlorid und Natronlauge.
Ausbeute: 88 % der Theorie,
$C_{25}H_{24}N_6O_3S$ (488.56)
$R_f$-Wert: 0.21 (Kieselgel; Essigester/Ethanol/Ammoniak = 50:45:5)
EKA-Massenspektrum: $(M+H)^+ = 489$
$(M+Na)^+ = 511$

Beispiel 148

3-Methyl-2-[N-(4-amidinophenyl)-aminomethyl]-imidazo[4,5-b]pyridin-6-yl-carbonsäure-N-phenyl-N-(2-hydroxycarbonylethyl)-amid-hydrochlorid

Hergestellt analog Beispiel 2 aus 3-Methyl-2-[N-(4-amidinophenyl)-aminomethyl]-imidazo[4,5-b]pyridin-6-yl-carbonsäure-N-phenyl-N-(2-ethoxycarbonylethyl)-amid-hydrochlorid und Natronlauge.
Ausbeute: 80 % der Theorie,
$C_{25}H_{25}N_7O_3$ (471.52)
$R_f$-Wert: 0.19 (Kieselgel; Essigester/Ethanol/Ammoniak = 50:45:5)
EKA-Massenspektrum: $(M+H)^+ = 472$
$(M+Na)^+ = 494$
$(M+2Na)^{++} = 258.6$

Beispiel 149

1-Methyl-2-[N-(4-amidinophenyl)-aminomethyl]-benzimidazol-5-yl-sulfonsäure-N-phenyl-N-(2-ethoxycarbonylethyl)-amid-hydrochlorid

a) 1-Methyl-2[N-(4-cyanophenyl)-aminomethyl]-benzimidazol-5-yl-sulfonsäure-N-phenyl-N-(2-ethoxycarbonylethyl)-amid

2.54 g (6,2 mMol) 3-Nitro-4-methylamino-benzolsulfonsäure-N-phenyl-N- (2-ethoxycarbonylethyl) -amid wurden bei Raumtemperatur und 5 bar Wasserstoffdruck über Palladium/Kohle (10%ig) in einem Gemisch aus 75 ml Ethanol und 75 ml Dichlormethan hydriert. Das so erhaltene rohe 3-Amino-4-methylamino-benzolsulfonsäure-N-phenyl-N-(2-ethoxycarbonylethyl)-amid wurde ohne Reinigung in 30 ml Phosphoroxychlorid aufgenommen, dann 1.1 g (6,2 mMol) N-(4-Cyanophenyl)-glycin hinzugefügt und die Mischung zwei Stunden unter Rückfluß gekocht. Nach dem Abkühlen auf Raumtemperatur wurde das Reaktionsgemisch in ca. 70 ml Wasser unter Kühlung eingetragen und auf diese Weise das überschüssige Phosphoroxychlorid zersetzt. Die so erhaltene Lösung wurde mit festem Natriumcarbonat neutralisiert und dreimal mit je 30 ml Essigester extrahiert. Nach Eindampfen des Lösungsmittels wurde das Rohprodukt durch Säulenchromatographie gereinigt (100 g Kieselgel; Laufmittel: Cyclohexan/Essigester = 2:3).
Ausbeute: 860 mg (26.8 % der Theorie),
Schmelzpunkt: 188-191°C
$C_{27}H_{27}N_5O_3S$ (517.6)
$R_f$-Wert: 0.52 (Kieselgel; Dichlormethan/Methanol = 9:1)
EKA-Massenspektrum: $(M+H)^+$ = 518
$(M+Na)^+$ = 540

b) 1-Methyl-2-[N-(4-amidinophenyl)-aminomethyl]-benzimidazol-5-yl-sulfonsäure-N-phenyl-N-(2-ethoxycarbonylethyl)-amid-hydrochlorid

Hergestellt analog Beispiel 25d aus 1-Methyl-2-[N-(4-cyanophenyl)-aminomethyl]-benzimidazol-5-yl-sulfonsäure-N-phenyl-N-(2-ethoxycarbonylethyl)-amid, ethanolischer Salzsäure, Ethanol und Ammoniumcarbonat.
Ausbeute: 87 % der Theorie,
$C_{27}H_{30}N_6O_4S$ (534.6)
$R_f$-Wert: 0.13 (Kieselgel; Dichlormethan/Ethanol = 9:1)
EKA-Massenspektrum: $(M+H)^+$ = 535
$(M+H+Na)^{++}$ = 279

Beispiel 150

1-Methyl-2-[N-(4-amidinophenyl)-aminomethyl]-benzimidazol-5-yl-sulfonsäure-N-(1-methylpyrazol-4-yl)-N-(2-ethoxycarbonylethyl)-amid-hydrochlorid

Hergestellt analog Beispiel 25d aus 1-Methyl-2-[N-(4-cyanophenyl)-aminomethyl]-benzimidazol-5-yl-sulfonsäure-N-(1-methylpyrazol-4-yl)-N-(2-ethoxycarbonylethyl)-amid, ethanolischer Salzsäure, Ethanol und Ammoniumcarbonat.
Ausbeute: 38 % der Theorie,
$C_{25}H_{30}N_8O_4S$ (538.6)
$R_f$-Wert: 0.09 (Kieselgel; Dichlormethan/Ethanol = 9:1)
EKA-Massenspektrum: $(M+H)^+$ = 539

Beispiel 151

1-Methyl-2-[N-(4-amidinophenyl)-aminomethyl]-5-(2.3-dihydroindol-1-yl-sulfonyl)-benzimidazol-hydrochlorid

Hergestellt analog Beispiel 25d aus 1-Methyl-2-[N-(4-cyanophenyl)-aminomethyl]-5-(2.3-dihydroindol-1-yl-sulfonyl)-benzimidazol und ethanolischer Salzsäure, Ethanol und Ammoniumcarbonat.
Ausbeute: 15 % der Theorie,
$R_f$-Wert: 0.36 (Kieselgel; Dichlormethan/Methanol = 4:1)
$C_{24}H_{24}N_6O_2S$ (460.6)
EKA-Massenspektrum: $(M+H)^+$ = 461

Beispiel 152

1-Methyl-2-[N-(4-amidinophenyl)-aminomethyl]-benzimidazol-5-yl-sulfonsäure-N-phenyl-N-(2-hydroxycarbonylethyl)-amid

Hergestellt analog Beispiel 26 aus 1-Methyl-2-[N-(4-amidinophenyl)-aminomethyl]-benzimidazol-5-yl-sulfonsäure-N-phenyl-N-(2-ethoxycarbonylethyl)-amid-hydrochlorid und Natronlauge.
Ausbeute: 24 % der Theorie,
$R_f$-Wert: 0.55 (Reverse-Phase RP-18 Kieselgel; Methanol/5%ige Kochsalzlösung = 3:2)
$C_{25}H_{26}N_6O_4S$ (506.6)

EKA-Massenspektrum: $(M+H)^+$ = 507
$(M+Na)^+$ = 529
$(M+2Na)^{++}$ = 276

Beispiel 153

1-Methyl-2-[N-(4-amidinophenyl)-aminomethyl]-5-(isoindolin-2-yl-sulfonyl)-benzimidazol-hydrochlorid

Hergestellt analog Beispiel 25d aus 1-Methyl-2-[N-(4-cyanophenyl)-aminomethyl]-5-(isoindolin-2-yl-sulfonyl)-benzimidazol und ethanolischer Salzsäure, Ethanol und Ammoniumcarbonat.
Ausbeute: 33 % der Theorie,
$R_f$-Wert: 0.32 (Kieselgel; Dichlormethan/Methanol = 4:1)
$C_{24}H_{24}N_6O_2S$ (460.6)
EKA-Massenspektrum: $(M+H)^+$ = 461

Beispiel 154

2-[2-(4-Amidinophenyl)-ethyl]-chinazolin-7-yl-carbonsäure-N-phenyl-N-(2-ethoxycarbonylethyl)-amid-hydrochlorid

a. 4-Methyl-3-nitro-benzoesäureethylester

Zu einer Lösung von 3 ml konzentrierter Salzsäure und 4 ml konzentrierter Schwefelsäure wurden unter Rühren bei 5°C 4.9 g (0.03 Mol) p-Tolylsäureethylester zugetropft und 1 Stunde unter Kühlung im Eisbad nachgerührt. Nach Erwärmung auf Raumtemperatur wurde auf Eiswasser gegossen und mit Essigester extrahiert. Die organischen Extrakte wurden mit Natriumhydrogencarbonatlösung gewaschen, getrocknet und eingedampft.
Ausbeute: 5.7 g (90 % der Theorie),
$R_f$-Wert: 0.81 (Kieselgel, Essigester/Cyclohexan = 1:1)

b. 4-(2-Dimethylaminovinyl)-3-nitro-benzoesäuremethylester

1.0 g (4.8 mMol) 4-Methyl-3-nitro-benzoesäureethylester, 0.74 g (6.2 mMol) Dimethylformamiddimethylacetal und 2 ml Dimethylformamid wurden unter Rühren 3 Stunden auf 140°C erhitzt. Anschließend wurde das Lösemittel abdestilliert und das so erhaltene Rohprodukt ohne weitere Reinigung umgesetzt.
Ausbeute: 1.2 g (100 % der Theorie),
$R_f$-Wert: 0.54 (Kieselgel, Essigester/Cyclohexan = 1:1)

c. 4-Formyl-3-nitro-benzoesäuremethylester

1.2 g (4.8 mMol) 4-(2-Dimethylaminovinyl)-3-nitro-benzoesäuremethylester wurden in 120 ml Tetrahydrofuran/Wasser (1:1) gelöst und nach Zugabe von 3.0 g (14.3 mMol) Natriummetaperiodat 20 Stunden bei Raumtemperatur gerührt. Die Suspension wurde anschließend mit Wasser und Methylenchlorid verdünnt und mit Methylenchlorid extrahiert. Die vereinigten organischen Extrakte wurden mit Natriumhydrogencarbonatlösung gewaschen, getrocknet und eingedampft. Der Rückstand wurde an Kieselgel chromatographiert und mit Essigester/Cyclohexan (1:3) eluiert.
Ausbeute: 0.6 g (63 % der Theorie),
$R_f$-Wert: 0.63 (Kieselgel, Essigester/Cyclohexan = 1:1)

d. 3-Amino-4-formyl-benzoesäuremethylester

Zu einer Lösung von 25 ml Ethanol/Eisessig/Wasser (2:2:1) wurden 0.6 g (2.9 mMol) 4-Formyl-3-nitro-benzoesäuremethylester, 1.2 g (21.4 mMol) Eisenpulver und 0.01 ml konzentrierter Salzsäure gegeben und unter Rühren 15 Minuten zum Rückfluß erhitzt. Anschließend wurde das Eisen abgetrennt, die Lösung mit Wasser verdünnt und mit Methylenchlorid extrahiert. Die vereinigten organischen Extrakte wurden mit Wasser gewaschen, getrocknet und eingedampft.
Ausbeute: 0.3 g (58 % der Theorie),
$R_f$-Wert: 0.74 (Kieselgel, Methylenchlorid/Methanol = 9.5:0.5)

e. 3-[3-(4-Cyanophenyl)-propionylamino]-4-formyl-benzoesäuremethylester

1.0 g (5.6 mMol) 3-Amino-4-formyl-benzoesäuremethylester und 1.1 g (5.6 mMol) 4-Cyanophenylpropionsäurechlorid wurden in 50 ml Methylenchlorid gelöst und nach Zugabe von 0.7 g (5.6 mMol) N-Ethyl-diisopropylamin 24 Stunden bei Raumtemperatur gerührt. Anschließend wurde mit Natriumhydrogencarbonatlösung extrahiert, die vereinigten organischen Extrakte getrocknet und eingedampft. Der Rückstand wurde an Kieselgel chromatographiert und mit Essigester/Cyclohexan (1:3) eluiert.
Ausbeute: 0.6 g (32 % der Theorie),
$R_f$-Wert: 0.60 (Kieselgel, Essigester/Cyclohexan = 1:1)

f. 2-[2-(4-Cyanophenyl)-ethyl]-chinazolin-7-carbonsäure-methylester

0.6 g (1.8 mMol) 3-[3-(4-Cyanophenyl)-propionylamino]-4-formyl-benzoesäureethylester und 10 ml methanolische Ammoniaklösung wurden in einem Druckgefäß 36 Stunden geschüttelt. Anschließend wurde das Lösemittel abdestilliert, der Rückstand an Kieselgel chromatographiert und mit Methylenchlorid, welches 0 bis 1 % Methanol enthielt, eluiert.
Ausbeute: 0.35 g (62 % der Theorie),
$R_f$-Wert: 0.38 (Kieselgel, Essigester/Cyclohexan =1:1)

g. 2-[2-(4-Cyanophenyl)-ethyl]-chinazolin-7-carbonsäure

0.3 g (0.94 mMol) 2-[2-(4-Cyanophenyl)-ethyl]-chinazolin-7-carbonsäuremethylester wurden in 4.7 ml 1N Lithiumhydroxidlösung und 4 ml Tetrahydrofuran gelöst und 3 Stunden bei Raumtemperatur gerührt. Anschließend wurden 4.7 ml 1N Salzsäure zugegeben und 30 Minuten gerührt. Das ausgefallene Produkt wurde abgesaugt, mit Wasser gewaschen und getrocknet.
Ausbeute: 0.30 g (100 % der Theorie),
$R_f$-Wert: 0.1 (Kieselgel, Essigester/Cyclohexan = 1:1)

h. 2-[2-(4-Cyanophenyl)-ethyl]-chinazolin-7-yl-carbonsäure-N-phenyl-N-(2-methoxycarbonylethyl)-amid

0.4 g (1.3 mMol) 2-[2-(4-Cyanophenyl)-ethyl]-chinazolin-7-carbonsäure und 5 ml Thionylchlorid wurden 60 Minuten bei 50°C gerührt. Anschließend wurde das Thionylchlorid abdestilliert, der Rückstand in Methylenchlorid gelöst, mit 0.24 g (1.3 mMol) 3-(N-Phenylamino)-propionsäuremethylester und 0.22 ml (1.3 mMol) N-Ethyldiisopropylamin versetzt und 18 Stunden bei Raumtemperatur gerührt. Nach Abdampfen des Lösemittels im Vakuum wurde an Kieselgel chromatographiert und mit Methylenchlorid, welches 1 % Methanol enthielt, eluiert.
Ausbeute: 230 mg (37 % der Theorie),
$R_f$-Wert: 0.64 (Kieselgel, Methylenchlorid/Methanol = 9:1)

i. 2-[2-(4-Amidinophenyl)-ethyl]-chinazolin-7-yl-carbonsäure-N-phenyl-N-(2-ethoxycarbonylethyl)-amid-hydrochlorid

230 mg (0.5 mMol) 2-[2-(4-Cyanophenyl)-ethyl]-chinazolin-7-yl-carbonsäure-N-phenyl-N-(2-methoxycarbonylethyl)-amid wurden in 30 ml gesättigter ethanolischer Salzsäure 8 Stunden bei Raumtemperatur gerührt. Anschließend wurde im Vakuum zur Trockne eingeengt, der Rückstand in 20 ml Ethanol aufgenommen, mit 0.5 g (5.0 mMol) Ammoniumcarbonat versetzt und über Nacht bei Raumtemperatur gerührt. Nach Abdampfen des Lösemittels wurde das Rohprodukt an Kieselgel chromatographiert und mit Methylenchlorid/Ethanol (4:1) eluiert.
Ausbeute: 100 mg (39 % der Theorie),
$R_f$-Wert: 0.5 (Kieselgel, Methylenchlorid/Ethanol = 4:1)
$C_{29}H_{29}N_5O_3$ (495.59)
Massenspektrum: $(M+H)^+$ = 496

Beispiel 155

1-Methyl-2-[N-(4-amidinophenyl)-aminomethyl]-benzimidazol-5-yl-sulfonsäure-N-(1-methylpyrazol-4-yl)-N-(2-hydroxycarbonylethyl)-amid

Hergestellt analog Beispiel 26 aus 1-Methyl-2-[N-(4-amidinophenyl)-aminomethyl]-benzimidazol-5-yl-sulfonsäure-N-(1-methylpyrazol-4-yl)-N-(2-ethoxycarbonylethyl)-amid-hydrochlorid und Natronlauge.
Ausbeute: 95 % der Theorie,

$C_{23}H_{26}N_8O_4S$ (510.6)
$R_f$-Wert: 0.53 (Reversed Phase Kieselgel RP-18, Methanol + 5%ige Kochsalz-Lösung)
EKA-Massenspektrum: $(M+H)^+ = 511$
$(M+Na)^+ = 533$
$(M+2Na)^{++} = 278$

Beispiel 156

1-Methyl-2-[N-(3-amidino-pyridin-6-yl)-aminomethyl]-benzimidazol-5-yl-carbonsäure-N-(2-pyridyl)-N-(2-ethoxycarbonylethyl)-amid-hydrochlorid

a) 3-[(N-tert.Butoxycarbonyl-amino)acetylamino]-4-methylaminobenzoesäure-N-(2-pyridyl)-N-(2-ethoxycarbonylethyl)-amid

19.2 g (0.11 Mol) N-tert.Butyloxycarbonylglycin wurden in 175 ml Dimethylformamid gelöst, mit 35.2 g (0.11 Mol) O-Benzotriazol-1-yl)-N,N,N',N'-tetramethyluroniumtetrafluorborat, 11.0 g Triethylamin und 34.2 g (0.10 Mol) 3-Amino-4-methylamino-benzoesäure-N-(2-pyridyl)-N-(2-ethoxycarbonylethyl)-amid versetzt und 2.5 Stunden bei Raumtemperatur gerührt. Anschließend wurde die Reaktionslösung mit 5 l Eiswasser versetzt und 2 Stunden gerührt. Der gebildete graue Niederschlag wurde abfiltriert, mit Wasser gewaschen, getrocknet und unter Zusatz von Aktivkohle aus Essigester umkristallisiert.
Ausbeute: 39.85 g (80 % der Theorie),
$C_{25}H_{33}N_5O_6$ (499.6)
$R_f$-Wert: 0.55 (Kieselgel; Methylenchlorid/Ethanol = 19:1)

b)1-Methyl-2-(N-tert.butoxycarbonyl-aminomethyl)-benzimidazol-5-yl-carbonsäure-N-(2-pyridyl)-N-(2-ethoxycarbonylethyl)-amid

10.0 g (0.02 Mol) 3-[(N-tert.Butoxycarbonyl-amino)acetylamino]-4-methylamino-benzoesäure-N-(2-pyridyl)-N-(2-ethoxycarbonylethyl)-amid wurden in 50 ml Eisessig gelöst und eine Stunde unter Rückfluß erhitzt. Anschließend wurde das Lösungsmittel abdestilliert, der Rückstand mit Eiswasser versetzt und durch Zusatz von 2N Ammoniak auf pH 8 eingestellt. Nach dreimalier Extraktion mit Essigester wurden die vereinigten organischen Phasen mit Kochsalzlösung gewaschen und über Natriumsulfat getrocknet. Nach Abdampfen des Lösungsmittels wurde das Rohprodukt an Kieselgel chromatographiert, wobei anfangs mit Methylenchlorid, später mit Methylenchlorid/Ethanol (50:1) und (25:1) eluiert wurde. Die gewünschten Fraktionen wurden vereinigt und eingedampft.
Ausbeute: 5.85 g (61 % der Theorie),
$C_{25}H_{31}N_5O_5$ (481.6)
$R_f$-Wert: 0.70 (Kieselgel; Methylenchlorid/Ethanol = 9:1)

c) 1-Methyl-2-aminomethyl-benzimidazol-5-yl-carbonsäure-N-(2-pyridyl)-N-(2-ethoxycarbonylethyl)-amid-trifluoracetat

4.81 g (0.10 Mol) 1-Methyl-2-(N-tert.butoxycarbonyl-aminomethyl)-benzimidazol-5-yl-carbonsäure-N-(2-pyridyl)-N-(2-ethoxycarbonylethyl)-amid wurden in 25 ml Methylenchlorid gelöst, mit 5 ml Trifluoressigsäure versetzt und 5 Stunden bei Raumtemperatur gerührt. Anschließend wurde das Lösungsmittel abgedampft und der Rückstand mit Ether verrührt. Die dabei gebildeten Kristalle wurden abfiltriert, mit Ether gewaschen und getrocknet.
Ausbeute: 3.15 g (68 % der Theorie),
$C_{20}H_{23}N_5O_3$ (381.4)
$R_f$-Wert: 0.18 (Kieselgel; Methylenchlorid/Ethanol = 9:1)

d) 1-Methyl-2-[N-(3-cyano-pyridin-6-yl)-aminomethyl]-benzimidazol-5-yl-carbonsäure-N-(2-pyridyl)-N-(2-ethoxycarbonylethyl)-amid

1.5 g (3.25 mMol) 1-Methyl-2-aminomethyl-benzimidazol-5-yl-carbonsäure-N-(2-pyridyl)-N-(2-ethoxycarbonylethyl)-amid-trifluoracetat wurden in 10 ml N-Ethyl-diisopropylamin verrührt und 15 Minuten auf 100°C erhitzt. Nach Zugabe von 720 mg (5.25 mMol) 2-Chlor-5-cyano-pyridin wurde das Reaktionsgemisch 2 Stunden auf 125°C erhitzt. Nach Abkühlen auf Raumtemperatur und Verrühren mit ca. 20 ml Wasser wurde durch Zugabe von 1N Salzsäure pH 4 eingestellt und 3 x mit Essigester extrahiert. Die vereinigten organischen Phasen wurden mit Kochsalzlösung gewaschen und über Natriumsulfat getrocknet. Nach Abdampfen des Lösungsmittels wurde das Rohprodukt an Kieselgel

chromatographiert, wobei anfangs mit Methylenchlorid, später mit Methylenchlorid/Ethanol (25:1) und (19:1) eluiert wurde. Die gewünschten Fraktionen wurden vereinigt und eingedampft.
Ausbeute: 1.05 g (67 % der Theorie),
$C_{26}H_{25}N_7O$ (483.6)
Massenspektrum: $(M+H)^+$ = 484

e) 1-Methyl-2-[N-(3-amidino-pyridin-6-yl)-aminomethyl]-benzimidazol-5-yl-carbonsäure-N-(2-pyridyl)-N-(2-ethoxycarbonylethyl)-amid-hydrochlorid

Hergestellt analog Beispiel 25d aus 1-Methyl-2-[N-(3-cyanopyridin-6-yl) -aminomethyl]-benzimidazol-5-yl-carbonsäure-N-(2-pyridyl)-N-(2-ethoxycarbonylethyl)-amid und ethanolischer Salzsäure, Ethanol und Ammoniumcarbonat.
Ausbeute: 38 % der Theorie,
$C_{28}H_{28}N_8O_3$ (500.6)
Massenspektrum: $(M+H)^+$ = 501

Beispiel 157

1-Methyl-2-[N-(4-amidinophenyl)aminomethyl]-indol-5-yl-carbonsäure-N-phenyl-N-(2-methoxycarbonylethyl)-amid-hydroiodid

a) 4-Nitro-benzoesäure-N-phenyl-N-(2-methoxycarbonylethyl)amid

16.7 g (0.1 Mol) 4-Nitrobenzoesäure wurden in 50 ml Thionylchlorid und 3 Tropfen Dimethylformamid 1 Stunde am Rückfluß gekocht. Nach Abdestillieren der Lösungsmittel im Vakuum wurde das Rohprodukt in 150 ml Tetrahydrofuran gelöst und zu einer Lösung von 18 g (0.1 Mol) N-(2-Methoxycarbonylethyl)anilin in 250 ml Tetrahydrofuran und 42 ml (0.3 Mol) Triethylamin getropft. Nach einstündigem Rühren bei Raumtemperatur wurde mit 250 ml Ethylacetat verdünnt und 2x mit 200 ml 14%iger Kochsalz-Lösung gewaschen. Nach Abdestillieren der Lösungsmittel und Chromatographie (Kieselgel; Methylenchlorid) isoliert man ein gelbes Öl, das langsam erstarrte.
Ausbeute: 32.6 g (100 % der Theorie),
$R_f$-Wert: 0.37 (Kieselgel; Methylenchlorid/Methanol = 50:1)

b) 4-Amino-benzoesäure-N-phenyl-N-(2-methoxycarbonylethyl)amid

22 g (67 mMol) 4-Nitro-benzoesäure-N-phenyl-N-(2-methoxycarbonylethyl)-amid wurden in 500 ml Methanol mit 2 g 10%igem Palladium auf Kohle bei 3 bar Wasserstoffdruck 3 Stunden lang hydriert. Nach Filtration und Abdestillieren des Lösungsmittels wurde mit 100 ml Ether gewaschen und das weiße kristalline Produkt direkt weiter umgesetzt.
Ausbeute: 18.6 g (94 % der Theorie),
$R_f$-Wert: 0.70 (Kieselgel; Methylenchlorid/Ethanol = 19:1)

c) 2-Methyl-3-thiomethyl-indol-5-yl-carbonsäure-N-phenyl-N-(2-methoxycarbonylethyl)-amid

26.8 g (91 mMol) 4-Amino-benzoesäure-N-phenyl-N-(2-methoxycarbonylethyl)amid wurden in 500 ml Methylenchlorid gelöst, auf -70°C gekühlt und innerhalb von 30 Minuten mit frisch hergestelltem tert.Butylhypochlorit (M. J. Mintz et al., Organic Synthesis, Coll. Vol. 5, Seite 184) versetzt. Es wurde 2 Stunden bei -70°C gerührt, dann wurden 9.46 g (91 mMol) Methylthioaceton in 40 ml Methylenchlorid innerhalb von 10 Minuten zugetropft und weitere 1.5 Stunden gerührt. Anschließend wurden 12.7 ml (9.1 g, 91 mMol) Triethylamin in 25 ml Methylenchlorid zugegeben. Es wurde 30 Minuten bei -78°C belassen und dann langsam über Nacht auf Raumtemperatur erwärmt. Nach 2x Waschen mit je 50 ml Wasser wurde die organische Phase abgetrennt und mit Natriumsulfat getrocknet. Nach Entfernen des Lösungsmittels im Vakuum erhält man nach Reinigung durch Chromatographie (Kieselgel; Essigester/Petrolether = 2:8 bis 3:7) eine weiße amorphe Substanz.
Ausbeute: 24.1 g (69 % der Theorie),
$R_f$-Wert: 0.58 (Kieselgel; Essigester/Petrolether = 1:1)
$C_{21}H_{22}N_2O_3S$ (382.49)
Massenspektrum: $(M)^+$ = 382

d) 1-tert-Butoxycarbonyl-2-methyl-indol-5-yl-carbonsäure-N-phenyl-N-(2-methoxycarbonylethyl)-amid

8.9 g (23 mMol) 2-Methyl-3-thiomethyl-indol-5-yl-carbonsäure-N-phenyl-N-(2-methoxycarbonylethyl)-amid wurden in 600 ml Ethanol gelöst, mit ca. 150 mg Raney-Nickel versetzt und 2 Stunden bei Raumtemperatur gerührt (analog P.G. Gassman et al., Organic Synthesis Coll. Vol. 6, Seite 601). Anschließend wurde filtriert und das Lösungsmittel im Vakuum entfernt. Das so erhaltene Rohprodukt (8 g) wurde in 200 ml absolutem Tetrahydrofuran gelöst, mit 150 mg Dimethylaminopyridin und 6.84 g (32 mMol) Pyrokohlensäure-di-tert.butylester versetzt und 2.5 Stunden bei 50°C gerührt. Anschließend wurde das Lösungsmittel im Vakuum abdestilliert und das Rohprodukt durch Chromatographie (Kieselgel, Essigester/Petrolether = 1:4) gereinigt.
Ausbeute: 10.0 g (98 % der Theorie),
$R_f$-Wert: 0.40 (Kieselgel; Essigester/Petrolether =3:7)

e) 2-[N-(4-Cyanophenyl)aminomethyl]-indol-5-yl-carbonsäure-N-phenyl-N-(2-methoxycarbonylethyl)-amid

3.5 g (8 mMol) 1-tert.Butoxycarbonyl-2-methyl-indol-5-yl-carbonsäure-N-phenyl-N-(2-methoxycarbonylethyl)-amid wurden in 80 ml Tetrachlorkohlenstoff gelöst, mit 1.5 g (8.4 mMol) N-Brom-succinimid und 20 mg Azobisisobutyronitril versetzt und 2.5 Stunden am Rückfluß gekocht. Anschließend wurde die noch warme Lösung filtriert, das erhaltene Filtrat mit gesättigter Natriumhydrogencarbonatlösung gewaschen und mit Natriumsulfat getrocknet. Nach Abdestillieren des Lösungsmittels wurde das Rohprodukt in 30 ml N-Ethyl-diisopropylamin gelöst, mit 1.0 g (8 mMol) 4-Aminobenzonitril versetzt und 2.5 Stunden am Rückfluß erhitzt. Das Lösungsmittel wurde im Vakuum abdestilliert und der erhaltene Rückstand durch Chromatographie (Kieselgel; Essigester/Petrolether = 1:4 bis 1:1) gereinigt.
Ausbeute: 1.1 g (30 % der Theorie),
$R_f$-Wert: 0.21 (Kieselgel; Essigester/Petrolether = 1:1)

f) 1-Methyl-2-[N-(4-thiocarbamoyl-phenyl)aminomethyl]-indol-5-yl-carbonsäure-N-phenyl-N-(2-methoxycarbonylethyl)-amid

1.5 g (3.3 mMol) 2-[N-(4-Cyanophenyl)aminomethyl]-indol-5-yl-carbonsäure-N-phenyl-N-(2-methoxycarbonylethyl)-amid wurden in 60 ml Xylol gelöst, mit 0.45 g (3.3 mMol) Kaliumcarbonat und 0.5 ml (3.3 mMol) p-Toluolsulfonsäuremethylester versetzt und 4 Stunden am Rückfluß erhitzt. Anschließend wurden nochmals dieselben Mengen Kaliumcarbonat und Toluolsulfonsäuremethylester zugegeben und über Nacht unter Rückfluß erhitzt. Es wurde filtriert und mit Aceton gewaschen. Nach Einengen des so erhaltenen Filtrats wurde der erhaltene Rückstand durch Chromatographie (Kieselgel; Essigester/Petrolether = 1:4 bis 2:3) gereinigt. Das erhaltene N-methylierte Indol (Ausbeute: 0.64 g, 41 % der Theorie) wurde in 20 ml Pyridin gelöst und mit 0.67 ml (1.37 mMol) Triethylamin versetzt. Anschließend wurde in die so erhaltene Lösung Schwefelwasserstoffgas eingeleitet. Nach 4.5 Tagen wurde 30 Minuten lang Stickstoff durch die Reaktionslösung geleitet, das Lösungsmittel abdestilliert und der erhaltene Rückstand durch Chromatographie (Kieselgel; Methylenchlorid/Ethanol 99:1 bis 98:2) gereinigt.
Ausbeute: 0.30 g (43 % der Theorie),
$C_{28}H_{28}N_4O_3S$ (500.62)
EKA-Massenspektrum: $(M+H)^+ = 501$
$(M+Na)^+ = 523$

g) 1-Methyl-2-[N-(4-amidinophenyl)aminomethyl]-indol-5-yl-carbonsäure-N-phenyl-N-(2-methoxycarbonylethyl)-amid-hydroiodid

0.30 g (0.60 mMol) 1-Methyl-2-[N-(4-thiocarbamoyl)-phenyl)-aminomethyl]-indol-5-yl-carbonsäure-N-phenyl-N-(2-methoxycarbonylethyl)-amid wurden zusammen mit 0.75 ml (12 mMol) Methyliodid in 20 ml Aceton gelöst und 2 Stunden bei Raumtemperatur gerührt. Anschließend wurde das Lösungsmittel abdestilliert und das Rohprodukt zusammen mit 1.0 g Ammoniumacetat in 12 ml Ethanol und 5 ml Methylenchlorid 20 Stunden bei 40°C gerührt. Das Lösungsmittel wurde im Vakuum abdestilliert und der erhaltene Rückstand mittels Chromatographie (Kieselgel; Methylenchlorid/Ethanol = 9:1 bis 4:1) gereinigt.
Ausbeute: 55 % der Theorie,
$C_{28}H_{29}N_5O_3$ (483.58)
$R_f$-Wert: 0.20 (Kieselgel; Methylenchlorid/Ethanol = 4:1 + 1 Tropfen Essigsäure)
EKA-Massenspektrum: $(M+H)^+ = 484$

Beispiel 158

1-Methyl-2-[N-(4-amidinophenyl)aminomethyl]-thieno[2.3-d]imidazol-5-yl-carbonsäure-N-phenyl-N-(2-ethoxycarbonylethyl)-amid-hydrochlorid

a) Methoxyessigsäureiminoethylester-hydrochlorid

Eine Lösung von 35.5 g (0.50 Mol) Methoxyacetonitril in 29 ml (23 g , 0.50 Mol) Ethanol und 30 ml absolutem Diethylether wurde auf 0°C gekühlt und innerhalb von 1 Stunde 22.5 g (0.62 Mol) Chlorwasserstoff-Gas eingeleitet, wobei gegen Ende der Gaseinleitung das Reaktionsprodukt auskristallisierte. Zur Vervollständigung der Fällung wurde mit 130 ml Diethylether versetzt und die farblosen Nadeln abfiltriert.
Ausbeute: 66.4 g (86 % der Theorie),
Schmelzpunkt: 117-118°C.

b) 4-Hydroxymethyl-2-methoxymethyl-imidazol

Ein Gemisch aus 30.6 g (0.20 Mol) Methoxyessigsäureiminoethylester-hydrochlorid, 18 g (0.20 Mol) 1.3-Dihydroxyaceton und 200 ml flüssigem Ammoniak wurde 3 Stunden im Rührautoklaven bei einem Druck von 27 bar auf 68°C erhitzt (analog: P. Dziuron et al. Arch. Pharm. 307, 1974, S.470). Anschließend wurde der Ammoniak entfernt und mit 200 ml Methylenchlorid versetzt. Der ausgefallene weiße Niederschlag wurde abfiltiert und mit Methylenchlorid gewaschen wurde. Das Filtrat wurde eingeengt und der erhaltene Rückstand chromatographisch gereinigt (Aluminiumoxid; Methylenchlorid/Ethanol = 90:10 bis 85:15).
Ausbeute: 26.7 g (94 % der Theorie),
$R_f$-Wert: 0.43 (Kieselgel; Methylenchlorid/Ethanol = 9:1)
$C_6H_{10}N_2O_2$ (142.20)
Massenspektrum: $(M)^+ = 142$

c) 4-Hydroxymethyl-2-methoxymethyl-1-methyl-imidazol als 1:1 Gemisch mit 5-Hydroxymethyl-2-methoxymethyl-1-methyl-imidazol

Ein Gemisch aus 7.1 g (50 mMol) 4-Hydroxymethyl-2-methoxymethylimidazol, 3.0 g (53 mMol) pulverisiertem Kaliumhydroxid und 3.4 ml (0.55 mMol) Methyliodid wurde in 100 ml Dimethylformamid 4 Stunden auf 50°C erhitzt (analog I. Sinclair et al., J. Med. Chem., 29, 1986, 261). Anschließend wurde das Lösungsmittel im Vakuum abdestilliert und das Rohprodukt säulenchromatographisch (Aluminiumoxid; Methylenchlorid/Ethanol = 99:1 bis 95: 5) gereinigt.
Ausbeute: 6.1 g (78 % der Theorie; 1:1 Gemisch der beiden Regioisomere)
$R_f$-Wert: 0.32 (Kieselgel; Methylenchlorid/Ethanol = 19:1)

d) 5-Chlor-4-hydroxymethyl-2-methoxymethyl-1-methyl-imidazol

Ein 1:1-Gemisch aus 7.7 g (49 mMol) 4-Hydroxymethyl-2-methoxymethyl-1-methyl-imidazol und 5-Hydroxymethyl-2-methoxymethyl-1-methyl-imidazol sowie 7.3 g (55 mMol) N-Chlor-succinimid wurde in 48 ml Ethylenglykolmonoethylether und 70 ml Dioxan 10 Stunden auf 50°C erhitzt. Anschließend wurde das Lösungsmittel im Vakuum abdestilliert und das Rohprodukt durch Chromatographie (Kieselgel; Methylenchlorid/Ethanol = 99:1 bis 90:10) zur isomerenreinen Titelverbindung gereinigt.
Ausbeute: 3.4 g (36 % der Theorie),
$R_f$-Wert: 0.40 (Kieselgel; Methylenchlorid/Ethanol = 19:1)

e) 5-Chlor-4-formyl-2-methoxymethyl-1-methyl-imidazol

3.4 g (18 mMol) 5-Chlor-4-hydroxymethyl-2-methoxymethyl-1-methyl-imidazol wurden in 100 ml Methylenchlorid gelöst und im 2-Stunden-Abstand mit Mangandioxid (2 x 6.0 g, insgesamt 0.14 Mol) versetzt. Nach 4 Stunden wurde die anorganische Komponente abfiltriert, das Lösungsmittel entfernt und das erhaltene Rohprodukt ohne weitere Reinigung weiter umgesetzt.
Ausbeute: 3.0 g (89 % der Theorie),
$R_f$-Wert: 0.44 (Kieselgel; Methylenchlorid/Ethanol = 50:1)

f) 1-Methyl-2-methoxymethyl-thieno[2.3-d]imidazol-5-yl-carbonsäureethylester

Zu einer frisch hergestellten Natriumethanolat-Lösung (aus 391 mg, 17 mMol Natrium) in 15 ml Ethanol tropfte man 1.9 ml (2.1 g, 17 mMol) Thioglykolsäureethylester. Nach 1-stündigen Rühren bei Raumtemperatur gab man 1.6 g (8.5 mMol) 5-Chlor-4-formyl-2-methoxymethyl-1-methyl-imidazol in 20 ml absolutem Ethanol zu und erhitzte auf 80°C (analog B. Iddon et al., J. Chem. Soc. Perkin Trans. I, 1987, 1457). Nach 5 Stunden destillierte man das Lösungsmittel ab, nahm den Rückstand in 50 ml Methylenchlorid auf und wusch mit 20 ml Wasser. Die wäßrige Phase wurde nochmals mit 20 ml Methylenchlorid gewaschen und anschließend die vereinigten organischen Phasen mit Natriumsulfat getrocknet. Nach Entfernen des Lösungsmittels im Vakuum wurde das erhaltene Rohprodukt durch Säulenchromatographie (Aluminiumoxid; Methylenchlorid) gereinigt.
Ausbeute: 1.0 g (46 % der Theorie),
$R_f$-Wert: 0.48 (Kieselgel; Methylenchlorid/Ethanol = 50:1)
$C_{11}H_{14}N_2O_3S$ (254.31)
EKA-Massenspektrum: $(M+H)^+$ = 255
$(M+Na)^+$ = 277

g) 1-Methyl-2-methoxymethyl-thieno[2.3-d]imidazol-5-yl-carbonsäure

Zu einer Lösung von 0.90 g (3.54 mMol) 1-Methyl-2-methoxymethyl-thieno[2.3-d]imidazol-5-yl-carbonsäureethylester in 30 ml Ethanol tropfte man 5 ml 2 N Natronlauge und rührte 2 Stunden bei Raumtemperatur. Anschließend wurde das Lösungsmittel im Vakuum abdestilliert, der Rückstand in 5 ml Wasser aufgenommen und mit 10 ml Diethylether gewaschen. Die wäßrige Phase wurde mit 6 ml 2N Salzsäure angesäuert, auf 0°C gekühlt und die ausgefallenen Kristalle abfiltriert.
Ausbeute: 0.50 g (63% der Theorie)
$R_f$-Wert: 0.21 (Kieselgel; Methylenchlorid/Ethanol = 9:1 + einige Tropfen Essigsäure)
$C_9H_{10}N_2O_3S$ (226.26)
Massenspektrum: $(M)^+$ = 226

h) 1-Methyl-2-methoxymethyl-thieno[2.3-d]imidazol-5-yl-carbonsäure-N-phenyl-N-(2-methoxycarbonylethyl)-amid

Eine Suspension aus 0.50 g (2.2 mMol) 1-Methyl-2-methoxymethyl-thieno[2.3-d]imidazol-5-yl-carbonsäure in 20 ml Methylenchlorid wurde mit 2.0 ml (3.2 g, 27 mMol) Thionylchlorid versetzt und 60 Minuten am Rückfluß gekocht, dabei löste sich der Feststoff allmählich auf. Nach Abdestillieren der flüssigen Komponenten wurde das Rohprodukt noch 2x in Methylenchlorid aufgenommen. Nach erneutem Entfernen des Lösungsmittels wurde das rohe Säurechlorid in 20 ml Tetrahydrofuran aufgenommen und zu einem Gemisch aus 0.42 g (2.3 mMol) N-(2-Methoxycarbonylethyl) anilin und 0.92 ml (6.6 mMol) Triethylamin in 30 ml Tetrahydrofuran getropft. Nach 16-stündigem Rühren bei 50°C wurde das Lösungsmittel entfernt und das erhaltene Rohprodukt durch Chromatographie (Kieselgel; Methylenchlorid/ Ethanol = 100:1) gereinigt.
Ausbeute: 0.66 g (77% der Theorie),
$R_f$-Wert: 0.47 (Kieselgel; Methylenchlorid/Ethanol = 19:1)

i)1-Methyl-2-(N-4-cyanophenylaminomethyl)-thieno[2.3-d]imidazol-5-yl-carbonsäure-N-phenyl-N-(2-methoxycarbonylethyl)-amid

Zu einer Lösung von 0.73 g (1.88 mMol) 1-Methyl-2-methoxymethyl-thieno[2.3-d]imidazol-5-yl-carbonsäure-N-phenyl-N-(2-methoxycarbonylethyl)-amid in 30 ml Methylenchlorid tropfte man bei 5°C 2.9 ml (2.9 mMol) einer 1-molaren Lösung aus Bortribromid in Methylenchlorid. Nach 16-stündigem Rühren bei Raumtemperatur wurde mit 20 ml gesättigter Natriumhydrogencarbonatlösung gewaschen, die organische Phase abgetrennt, mit Natriumsulfat getrocknet und filtriert. Das Filtrat versetzte man mit 14 ml N-Ethyl-diisopropylamin und 0.43 g (3.64 mMol) 4-Aminobenzonitril. Anschließend wurde das Methylenchlorid im Vakuum abdestilliert, der erhaltene Rückstand 1 Stunde auf 50°C erhitzt und danach das restliche Lösungsmittel im Vakuum abdestilliert. Man erhielt nach Chromatographie (Kieselgel; Methylenchlorid/Ethanol = 99:1 bis 97:3) ein gelbes langsam erstarrendes Öl.
Ausbeute: 0.37 g (42% der Theorie),
$R_f$-Wert: 0.29 (Kieselgel; Methylenchlorid/Ethanol = 50:1 + einige Tropfen Ammoniak)

j)1-Methyl-2-[N-(4-amidinophenyl)aminomethyl]-thieno[2.3-d]-imidazol-5-yl-carbonsäure-N-phenyl-N-(2-ethoxycarbonylethyl)-amid-hydrochlorid

0.38 g (0.80 mMol) 1-Methyl-2-(N-4-cyanophenylaminomethyl)-thieno[2.3-d]imidazol-5-yl-carbonsäure-N-phenyl-N-(2-methoxycarbonylethyl)-amid wurden in 40 ml mit Chlorwasserstoff gesättigtem Ethanol 5 Stunden erst bei 0°C, später bei Raumtemperatur gerührt bis dünnschichtchromatographisch kein Ausgangsmaterial mehr nachweisbar war. Anschließend wurde das Lösungsmittel bei maximal 28°C Badtemperatur abdestilliert, der ölige Rückstand wurde in 40 ml absolutem Ethanol aufgenommen und mit 1.1 g Ammoniumcarbonat versetzt. Nach 18 Stunden wurde das Lösungsmittel im Vakuum abdestilliert und das Rohprodukt durch Chromatographie (Kieselgel; Methylenchlorid/Ethanol = 9:1 bis 4:1) gereinigt.
Ausbeute : 57 % der Theorie
$C_{26}H_{28}N_6O_3S$ (504.62)
$R_f$-Wert: 0.21 (Kieselgel; Methylenchlorid/Ethanol = 4:1 + einige Tropfen Essigsäure)
EKA-Massenspektrum: $(M+H)^+$ = 505
$(M+H+Na)^{++}$ = 264

Beispiel 159

1-Methyl-2-[N-(4-amidinophenyl)aminomethyl]-thieno[2.3-d]imidazol-5-yl-carbonsäure-N-phenyl-N-(2-hydroxycarbonylethyl)-amid-hydrochlorid

Hergestellt analog Beispiel 2 aus 1-Methyl-2-[N-(4-amidinophenyl)aminomethyl]-thieno[2.3-d]imidazol-5-yl-carbonsäure-N-phenyl-N-(2-ethoxycarbonylethyl)-amid-hydrochlorid und Natronlauge.
Ausbeute: 85 % der Theorie,
$C_{24}H_{24}N_6O_3S$ (476.56)
$R_f$-Wert: 0.36 (Reversed Phase Kieselgel RP-8; Methanol + 5 % Kochsalz-Lösung)
EKA-Massenspektrum: $(M+H)^+$ = 477
$(M+Na)^+$ = 499
$(M+2Na)^{++}$ = 250

Beispiel 160

1-Methyl-3-[N-(4-amidinophenyl)thiomethyl]-chinoxalin-2-on-6-yl-carbonsäure-N-phenyl-N-(2-ethoxycarbonylethyl)-amid-hydrochlorid

a) 1-Methyl-3-[N-(4-cyanophenyl)thiomethyl]-chinoxalin-2-on-6-yl-carbonsäure-N-phenyl-N-(2-methoxycarbonylethyl)-amid

Eine Lösung aus 2.5 g (7.6 mMol) 3-Amino-4-methylamino-benzoesäure-N-phenyl-N-(2-methoxycarbonylethyl)-amid und 2.4 g (9.6 mMol) 3-(4-Cyanophenyl)thio-2-oxo-propionsäureethylester wurden in 50 ml Ethanol 30 Minuten zum Sieden erhitzt. Nach Entfernen des Lösungsmittels wurde das erhaltene Rohprodukt chromatographisch gereinigt (Kieselgel; Methylenchlorid).
Ausbeute: 1.6 g (40 % der Theorie),
$R_f$-Wert: 0.63 (Kieselgel; Essigester/Ethanol/Ammmoniak = 90:10:1)

b) 1-Methyl-3-[N-(4-amidinophenyl)thiomethyl]-chinoxalin-2-on-6-yl-carbonsäure-N-phenyl-N-(2-ethoxycarbonylethyl)-amid-hydrochlorid

Hergestellt analog Beispiel 1 aus 1-Methyl-3-[N-(4-cyanophenyl)thiomethyl]-chinoxalin-2-on-6-yl-carbonsäure-N-phenyl-N-(2-ethoxycarbonylethyl)-amid und ethanolischer Salzsäure, Ethanol und Ammoniumcarbonat.
Ausbeute: 23 % der Theorie,
$C_{28}H_{27}N_5O_4S$ (543.64)
$R_f$-Wert: 0.25 (Kieselgel; Essigester/Ethanol/Ammoniak = 50:45:5)
EKA-Massenspektrum: $(M+H)^+$ = 544
$(M+Na)^+$ = 566

Beispiel 161

3-Methyl-2-[2-(4-amidinophenyl)ethyl]-imidazo[1.2-a]pyridin-7-yl-carbonsäure-N-phenyl-N-(2-ethoxycarbonylethyl)-amid-hydrochlorid

a) 3-Methyl-2-[2-(4-cyanophenyl)ethyl]-imidazo[1.2-a]pyridin-7-yl-carbonsäure-N-phenyl-N-(2-ethoxycarbonylethyl)-amid

1.4 g (4.6 mMol) 3-Methyl-2-[2-(4-cyanophenyl)ethyl]-imidazo[1.2-a]pyridin-7-yl-carbonsäure (hergestellt aus 4-Brom-1-(4-cyanophenyl)-1-penten-3-on und 2-Aminopyridin-4-carbonsäuremethylester analog Y. Katsura et al. Chem. Pharm. Bull. 1992, 40, 1424-1438) wurden in 15 ml Thionylchlorid suspendiert und bis zur vollständigen Lösung 1 Stunde lang zum Sieden erhitzt. Nach dem Abdestillieren des Thionylchlorids wurde das Säurechlorid ohne weitere Reinigung in 15 ml Pyridin gelöst und bei 0°C mit 1.0 g (5.2 mMol) N-(2-ethoxycarbonylethyl)anilin versetzt. Nach 1 Stunde wurde das Lösungsmittel abdestilliert, der Rückstand in 30 ml Methylenchlorid aufgenommen, mit 15 ml 1N Salzsäure gewaschen und mit Natriumsulfat getrocknet. Nach Abdestillieren des Lösungsmittels und Chromatographie (Kieselgel; Methylenchlorid/Ethanol = 0 bis 2 %) erhielt man ein braunes Öl.
Ausbeute: 1.48 g (64 % der Theorie),
$R_f$-Wert: 0.73 (Kieselgel; Essigester/Ethanol/Ammmoniak = 90:10:1)

b) 3-Methyl-2-[2-(4-amidinophenyl)ethyl]-imidazo[1.2-a]pyridin-7-yl-carbonsäure-N-phenyl-N-(2-ethoxycarbonylethyl)-amid-hydrochlorid

Hergestellt analog Beispiel 1 aus 3-Methyl-2-[2-(4-cyanophenyl)ethyl]-imidazo[1.2-a]pyridin-7-yl-carbonsäure-N-phenyl-N-(2-ethoxycarbonylethyl)-amid und ethanolischer Salzsäure, Ethanol und Ammoniumcarbonat.
Ausbeute: 62 % der Theorie,
$C_{29}H_{31}N_5O_3$ (497.60)
$R_f$-Wert: 0.23 (Kieselgel; Essigester/Ethanol/Ammoniak = 50:45:5)
EKA-Massenspektrum: $(M+H)^+$ = 498

Beispiel 162

3-Methyl-2-[2-(4-amidinophenyl)ethyl]-imidazo[1.2-a]pyridin-7-yl-carbonsäure-N-phenyl-N-(2-hydroxycarbonylethyl)-amid-hydrochlorid

Hergestellt analog Beispiel 2 aus 3-Methyl-2-[2-(4-amidinophenyl)ethyl]-imidazo[1.2-a]pyridin-7-yl-carbonsäure-N-phenyl-N-(2-ethoxycarbonylethyl)-amid-hydrochlorid und Natronlauge. Ausbeute: 92 % der Theorie,
$C_{27}H_{27}N_5O_3$ (469.55)
$R_f$-Wert: 0.19 (Kieselgel; Essigester/Ethanol/Ammoniak = 50:45:5)
EKA-Massenspektrum: $(M+H)^+$ = 470
$(M+Na)^+$ = 492
$(M+2H)^{++}$ = 235.7
$(M+H+Na)^{++}$ = 246.7
$(M+2Na)^{++}$ = 257.7

Beispiel 163

1-Methyl-2-[N-(4-amidinophenyl)-aminomethyl]-benzimidazol-5-yl-carbonsäure-N-phenyl-N-[(N-ethoxycarbonylethyl-N-methyl)-2-aminoethyl]-amid-dihydrochlorid

Hergestellt analog Beispiel 25d aus 1-Methyl-2-[N-(4-cyanophenyl)-aminomethyl]-benzimidazol-5-yl-carbonsäure-N-phenyl-N-[(N-ethoxycarbonylethyl-N-methyl)-2-aminoethyl]-amid und ethanolischer Salzsäure, Ethanol und Ammoniumcarbonat.
Ausbeute: 80 % der Theorie,
$C_{31}H_{37}N_7O_3$ (555.7)
$R_f$-Wert: 0.24 (Kieselgel; Dichlormethan/Methanol = 4:1)
EKA-Massenspektrum: $(M+H)^+$ = 556
$(M+H+Na)^{++}$ = 289.8
$(M+2H)^{++}$ = 278.8

Beispiel 164

1-Methyl-2-[N-(4-amidinophenyl)-aminomethyl]-benzimidazol-5-yl-carbonsäure-N-phenyl-N-[(N-hydroxycarbonylethyl-N-methyl)-2-aminoethyl]-amid-hydrochlorid

Hergestellt analog Beispiel 26 aus 1-Methyl-2-[N-(4-amidinophenyl)-aminomethyl]-benzimidazol-5-yl-carbonsäure-N-phenyl-N-[(N-ethoxycarbonylethyl-N-methyl)-2-aminoethyl]-amid-dihydrochlorid und Natronlauge.
Ausbeute: 79 % der Theorie,
$C_{29}H_{33}N_7O_3$ (527.6)
$R_f$-Wert: 0.43 (Reversed Phase Kieselgel RP-18; Methanol/5%ige wäßrige Kochsalz-Lösung = 6:4)
EKA-Massenspektrum: $(M+H)^+$ = 528
$(M+H+Na)^{++}$ = 275.6
$(M+2H)^{++}$ = 264.6

Beispiel 165

1-Methyl-2-[N-(4-amidinophenyl)-aminomethyl]-benzimidazol-5-yl-carbonsäure-N-phenyl-N-(3-hydroxy-n-propyl)-amid-hydrochlorid

Hergestellt aus 1-Methyl-2-[N-(4-amidinophenyl)-aminomethyl]-benzimidazol-5-yl-carbonsäure-N-phenyl-N-(3-benzyloxy-n-propyl)-amid-hydrochlorid durch Hydrieren über Palladium/Kohle (10%) bei 5 bar Wasserstoffdruck und Raumtemperatur.
Ausbeute: 61 % der Theorie,
$C_{26}H_{28}N_6O_2$ (456.6)
$R_f$-Wert: 0.70 (Reversed Phase Kieselgel RP-18; Methanol/5%ige wäßrige Kochsalz-Lösung = 9:1)
EKA-Massenspektrum: $(M+H)^+$ = 457
$(M+H+Na)^{++}$ = 240

Beispiel 166

1-Methyl-2-[N-[4-(N-n-hexyloxycarbonylamidino)phenyl]-aminomethyl]-benzimidazol-5-yl-carbonsäure-N-(2-pyridyl) -N-(2-hydroxycarbonylethyl)-amid

Hergestellt analog Beispiel 26 aus 1-Methyl-2-[N-[4-(N-n-hexyloxycarbonylamidino)phenyl]-aminomethyl]-benzimidazol-5-yl-carbonsäure-N-(2-pyridyl)-N-(2-ethoxycarbonylethyl)-amid und Natronlauge.
Ausbeute: 97 % der Theorie,
$C_{32}H_{37}N_7O_5$ (599.7)
$R_f$-Wert: 0.22 (Kieselgel; Dichlormethan/Methanol = 9:1)
EKA-Massenspektrum: $(M+H)^+$ = 600
$(M+H+Na)^{++}$ = 311.7
$(M+2H)^{++}$ = 300.8
$(M+2Na)^{++}$ = 322.8

Beispiel 167

1-Methyl-2-[N-[4-(N-n-hexyloxycarbonylamidino)phenyl]-aminomethyl]-benzimidazol-5-yl-carbonsäure-N-phenyl-N-(3-hydroxy-n-propyl)-amid

Hergestellt analog Beispiel 165 aus 1-Methyl-2-[N-[4-(N-nhexyloxycarbonylamidino)phenyl]-aminomethyl]-benzimidazol-5-yl-carbonsäure-N-phenyl-N-(3-benzyloxy-n-propyl)-amid durch katalytische Debenzylierung.
Ausbeute: 26 % der Theorie,
$C_{33}H_{40}N_6O_4$ (584.7)
$R_f$-Wert: 0.39 (Kieselgel; Dichlormethan/Ethanol = 9:1 )
EKA-Massenspektrum: $(M+H)^+$ = 585
$(M+H+Na)^{++}$ = 304
$(M+Na)^+$ = 607

Beispiel 168

1-Methyl-2-[N-(4-amidinophenyl)-aminomethyl]-benzimidazol-5-yl-carbonsäure-N-(3-fluorphenyl)-N-(2-ethoxycarbonylethyl)-amid-hydrochlorid

Hergestellt analog Beispiel 25d aus 1-Methyl-2-[N-(4-cyanophenyl)-aminomethyl]-benzimidazol-5-yl-carbonsäure-N-(3-fluorphenyl)-N-(2-ethoxycarbonylethyl)-amid und ethanolischer Salzsäure, Ethanol und Ammoniumcarbonat.
Ausbeute: 42 % der Theorie,
$C_{28}H_{29}FN_6O_3$ (516.6)
$R_f$-Wert: 0.31 (Kieselgel; Dichlormethan/Methanol = 5:1 )
EKA-Massenspektrum: $(M+H)^+$ = 517
$(M+H+Na)^{++}$ = 270

Beispiel 169

1-Methyl-2-[N-(4-amidinophenyl)-aminomethyl]-benzimidazol-5-yl-carbonsäure-N-(4-fluorphenyl)-N-(2-ethoxycarbonylethyl)-amid-hydrochlorid

Hergestellt analog Beispiel 25d aus 1-Methyl-2-[N-(4-cyanophenyl)-aminomethyl]-benzimidazol-5-yl-carbonsäure-N-(4-fluorphenyl)-N-(2-ethoxycarbonylethyl)-amid und ethanolischer Salzsäure, Ethanol und Ammoniumcarbonat.
Ausbeute: 90 % der Theorie,
$C_{28}H_{29}FN_6O_3$ (516.6)
$R_f$-Wert: 0.29 (Kieselgel; Dichlormethan/Methanol = 5:1)
EKA-Massenspektrum: $(M+H)^+$ = 517
$(M+H+Na)^{++}$ = 270

Beispiel 170

1-Methyl-2-[N-(4-amidinophenyl)-aminomethyl]-benzimidazol-5-yl-carbonsäure-N-(3-fluorphenyl)-N-(2-hydroxycarbonylethyl)-amid

Hergestellt analog Beispiel 26 aus 1-Methyl-2-[N-(4-amidinophenyl)-aminomethyl]-benzimidazol-5-yl-carbonsäure-N-(3-fluorphenyl)-N-(2-ethoxycarbonylethyl)-amid-hydrochlorid und Natronlauge.
Ausbeute: 97 % der Theorie,
$C_{26}H_{25}FN_6O_3$ (488.5)
$R_f$-Wert: 0.13 (Kieselgel; Dichlormethan/Ethanol = 4:1)
EKA-Massenspektrum: $(M+H)^+$ = 489
$(M+Na)^+$ = 511
$(M+2Na)^{++}$ = 267

Beispiel 171

1-Methyl-2-[N-(4-amidinophenyl)-aminomethyl]-benzimidazol-5-yl-carbonsäure-N-(4-fluorphenyl)-N-(2-hydroxycarbonylethyl)-amid

Hergestellt analog Beispiel 26 aus 1-Methyl-2-[N-(4-amidinophenyl)-aminomethyl]-benzimidazol-5-yl-carbonsäure-N-(4-fluorphenyl)-N-(2-ethoxycarbonylethyl)-amid-hydrochlorid
und Natronlauge.
Ausbeute: 89 % der Theorie,
$C_{26}H_{25}FN_6O_3$ (488.5)
$R_f$-Wert: 0.15 (Kieselgel; Dichlormethan/Ethanol = 4:1)
EKA-Massenspektrum: $(M+H)^+$ = 489
$(M+Na)^+$ = 511
$(M+2Na)^{++}$ = 267

Beispiel 172

1-Methyl-2-[N-(4-amidino-2-methoxy-phenyl)-aminomethyl]-benzimidazol-5-yl-carbonsäure-N-phenyl-N-(2-ethoxycarbonylethyl)-amid-hydrochlorid

Hergestellt analog Beispiel 25d aus 1-Methyl-2-[N-(4-cyano-2-methoxy-phenyl)-aminomethyl]-benzimidazol-5-yl-carbonsäure-N-phenyl-N-(2-ethoxycarbonylethyl)-amid und ethanolischer Salzsäure, Ethanol und Ammoniumcarbonat.
Ausbeute: 89 % der Theorie,
$C_{29}H_{32}N_6O_4$ (528.6)
$R_f$-Wert: 0.13 (Kieselgel; Dichlormethan/Ethanol = 4:1)
EKA-Massenspektrum: $(M+H)^+$ = 529
$(M+H+Na)^{++}$ = 276
$(M+2H)^{++}$ = 265

Beispiel 173

1-Methyl-2-[N-[4-(N-4-ethylbenzoylamidino)phenyl]-aminomethyl]-benzimidazol-5-yl-carbonsäure-N-(2-pyridyl)-N-(2-ethoxycarbonylethyl)-amid

Hergestellt analog Beispiel 90 aus 1-Methyl-2-[N-(4-amidinophenyl)-aminomethyl]-benzimidazol-5-yl-carbonsäure-N-(2-pyridyl)-N-(2-ethoxycarbonylethyl)-amid-hydrochlorid und 4-Ethylbenzoylchlorid.
Ausbeute: 64 % der Theorie,
$C_{36}H_{37}N_7O_4$ (631.7)
$R_f$-Wert: 0.78 (Kieselgel; Dichlormethan/Methanol = 9:1)
EKA-Massenspektrum: $(M+H)^+$ = 632
$(M+H+Na)^{++}$ = 327.8
$(M+Na)^+$ = 654

Beispiel 174

1-Methyl-2-[N-[4-(N-benzyloxycarbonylamidino)phenyl]-aminomethyl]-benzimidazol-5-yl-carbonsäure-N-(2-pyridyl)-N-(2-ethoxycarbonylethyl)-amid

Hergestellt analog Beispiel 90 aus 1-Methyl-2-[N-(4-amidinophenyl)-aminomethyl]-benzimidazol-5-yl-carbonsäure-N-(2-pyridyl)-N-(2-ethoxycarbonylethyl)-amid-hydrochlorid und Chlorameisensäurebenzylester.
Ausbeute: 64 % der Theorie,
$C_{35}H_{35}N_7O_5$ (633.6)
$R_f$-Wert: 0.60 (Kieselgel; Dichlormethan/Methanol = 9:1)
EKA-Massenspektrum: $(M+H)^+$ = 634
$(M+H+Na)^{++}$ = 328.8
$(M+Na)^+$ = 656

Beispiel 175

1-Methyl-2-[N-(4-amidino-2-methoxy-phenyl)-aminomethyl]-benzimidazol-5-yl-carbonsäure-N-phenyl-N-(2-hydroxycarbonylethyl)-amid

Hergestellt analog Beispiel 26 aus 1-Methyl-2-[N-(4-amidino-2-methoxy-phenyl)-aminomethyl]-benzimidazol-5-yl-carbonsäure-N-phenyl-N-(2-ethoxycarbonylethyl)-amid-hydrochlorid und Natronlauge.
Ausbeute: 71 % der Theorie,
$C_{27}H_{28}N_6O_4$ (500.6)
$R_f$-Wert: 0.15 (Kieselgel; Dichlormethan/Ethanol = 4:1)
EKA-Massenspektrum: $(M+H)^+$ = 501
$(M+Na)^+$ = 523
$(M+2Na)^{++}$ = 273

Beispiel 176

1-Methyl-2-[N-(4-amidino-2-methoxy-phenyl)-aminomethyl]-benzimidazol-5-yl-carbonsäure-N-(2-pyridyl)-N-(2-ethoxycarbonylethyl)-amid-hydrochlorid

Hergestellt analog Beispiel 25d aus 1-Methyl-2-[N-(4-cyano-2-methoxy-phenyl)-aminomethyl]-benzimidazol-5-yl-carbonsäure-N-(2-pyridyl)-N-(2-ethoxycarbonylethyl)-amid und ethanolischer Salzsäure, Ethanol und Ammoniumcarbonat.
Ausbeute: 67 % der Theorie,
$C_{28}H_{31}N_7O_4$ (529.6)
$R_f$-Wert: 0.16 (Kieselgel; Dichlormethan/Ethanol = 4:1)
EKA-Massenspektrum: $(M+H)^+$ = 530

Beispiel 177

1-Methyl-2-[N-(4-amidino-2-methoxy-phenyl)-aminomethyl]-benzimidazol-5-yl-carbonsäure-N-(2-pyridyl)-N-(2-hydroxycarbonylethyl)-amid

Hergestellt analog Beispiel 26 aus 1-Methyl-2-[N-(4-amidino-2-methoxy-phenyl)-aminomethyl]-benzimidazol-5-yl-carbonsäure-N-(2-pyridyl)-N-(2-ethoxycarbonylethyl)-amid-hydrochlorid und Natronlauge.
Ausbeute: 78 % der Theorie,
$C_{26}H_{27}N_7O_4$ (501.6)
$R_f$-Wert: 0.12 (Kieselgel; Dichlormethan/Ethanol = 4:1)
EKA-Massenspektrum: $(M+H)^+$ = 502

Beispiel 178

1-Methyl-2-[N-[4-(N-benzyloxycarbonylamidino)phenyl]-aminomethyl]-benzimidazol-5-yl-carbonsäure-N-(2-pyridyl)-N-(2-hydroxycarbonylethyl)-amid

Hergestellt analog Beispiel 104 aus 1-Methyl-2-[N-[4-(N-benzyloxycarbonylamidino)phenyl]-aminomethyl]-benzimidazol-5-yl-carbonsäure-N-(2-pyridyl)-N-(2-ethoxycarbonylethyl)-amid und Natronlauge.
Ausbeute: 62 % der Theorie,
$C_{33}H_{31}N_7O_5$ (605.7)
$R_f$-Wert: 0.26 (Kieselgel; Dichlormethan/Methanol = 9:1)
EKA-Massenspektrum: $(M+H)^+$ = 606
$(M+Na)^+$ = 628
$(M-H+2Na)^+$ = 650
$(M+2H)^{++}$ = 303.8
$(M+H+Na)^{++}$ = 314.8
$(M+2Na)^{++}$ = 325.7

Beispiel 179

1-Methyl-2-[N-(4-amidinophenyl)-aminomethyl]-benzimidazol-5-yl-carbonsäure-N-phenyl-N-(3-benzyloxy-n-propyl)-amid-hydrochlorid

Hergestellt analog Beispiel 25 aus 1-Methyl-2-[N-(4-cyanophenyl)-aminomethyl]-benzimidazol-5-yl-carbonsäure-N-phenyl-N-(3-benzyloxy-n-propyl)-amid und ethanolischer Salzsäure, Ethanol und Ammoniumcarbonat.
Ausbeute: 61 % der Theorie,
$C_{33}H_{34}N_6O_2$ (546.7)
$R_f$-Wert: 0.19 (Kieselgel; Dichlormethan/Ethanol = 4:1)
EKA-Massenspektrum: $(M+H)^+$ = 547
$(M+H+Na)^{++}$ = 285

Beispiel 180

1-Methyl-2-[N-[4-(N-n-hexyloxycarbonylamidino)phenyl]-aminomethyl]-benzimidazol-5-yl-carbonsäure-N-phenyl-N-(3-benzyloxy-n-propyl)-amid

Hergestellt analog Beispiel 90 aus 1-Methyl-2-[N-(4-amidinophenyl)-aminomethyl]-benzimidazol-5-yl-carbonsäure-N-phenyl-N-(3-benzyloxy-n-propyl)-amid-hydrochlorid und Chlorameisensäure-n-hexylester.
Ausbeute: 73 % der Theorie,
$C_{40}H_{46}N_6O_4$ (674.9)
$R_f$-Wert: 0.46 (Kieselgel; Dichlormethan/Ethanol = 9:1)
EKA-Massenspektrum: $(M+H)^+$ = 675
$(M+H+Na)^{++}$ = 349
$(M+Na)^+$ = 697
$(M+K)^+$ = 713

Beispiel 181

3-Methyl-2-[2-(4-amidinophenyl)ethyl]-imidazo[1.2-a]pyridin-7-yl-carbonsäure-N-(2-pyridyl)-N-(2-ethoxycarbonylethyl)-amid-hydrochlorid

Hergestellt analog Beispiel 1 aus 3-Methyl-2-[2-(4-cyanophenyl)ethyl]-imidazo[1.2-a]pyridin-7-yl-carbonsäure-N-(2-pyridyl)-N-(2-methoxycarbonylethyl)-amid-hydrochlorid und ethanolischer Salzsäure, Ethanol und Ammoniumcarbonat.
Ausbeute: 53 % der Theorie,
$C_{28}H_{30}N_6O_3$ (498.59)
$R_f$-Wert: 0.42 (Kieselgel; Essigester/Ethanol/Ammoniak = 50:45:5)
EKA-Massenspektrum: $(M+H)^+$ = 499
$(M+2Na)^{++}$ = 272
$(M+H+Na)^{++}$ = 261
$(M+2H)^{++}$ = 250

Beispiel 182

1-Methyl-2-[N-(3-amidino-pyridin-6-yl)-aminomethyl]-benzimidazol-5-yl-carbonsäure-N-(2-pyridyl)-N-(2-hydroxycarbonylethyl)-amid

Hergestellt analog Beispiel 26 aus 1-Methyl-2-[N-(3-cyanopyridin-6-yl)-aminomethyl]-benzimidazol-5-yl-carbonsäure-N-(2-pyridyl)-N-(2-hydroxycarbonylethyl)-amid und Natronlauge.
Ausbeute: 40 % der Theorie,
$C_{24}H_{24}N_8O_3$ (472.9)
$R_f$-Wert: 0.67 (Reversed Phase Kieselgel RP-8; Methanol/5%ige Kochsalz-Lösung = 1:1)
EKA-Massenspektrum: $(M+H)^+$ = 473

Beispiel 183

1-Methyl-2-[N-[4-(N-hydroxylamidino)phenyl]-aminomethyl]-benzimidazol-5-yl-carbonsäure-N-(2-pyridyl)-N-[2-(methansulfonylaminocarbonyl)-ethyl]-amid

a.1-Methyl-2-[N-(4-cyanophenyl)-aminomethyl]-benzimidazol-5-yl-carbonsäure-N-(2-pyridyl)-N-[2-(methansulfonylaminocarbonyl)-ethyl]-amid

2.0 g (4.5 mMol) 1-Methyl-2-[N-(4-cyanophenyl)-aminomethyl]-benzimidazol-5-yl-carbonsäure-N-(2-pyridyl)-N-(2-hydroxycarbonylethyl)-amid und 0.73 g (4.7 mMol) Carbonyldiimidazol wurden in 80 ml Tetrahydrofuran und 5 ml Dimethylformamid gelöst und 30 Minuten bei Raumtemperatur und 2 Stunden bei 90°C gerührt. Parallel dazu wurden 0.55 g (5.8 mMol) Methansulfonsäureamid und 0.28 g (5.8 mMol) Natriumhydrid in 15 ml Dimethylformamid suspendiert und 2 Stunden bei Raumtemperatur gerührt. Anschließend wurde diese Suspension bei Raumtemperatur zur Tetrahydrofuran-Lösung zugegeben. Nach 12 Stunden bei Raumtemperatur wurden 50 ml Wasser zugesetzt und der pH-Wert auf 6.8 eingestellt. Die Lösung wurde 4x mit Methylenchlorid extrahiert, die vereinigten organischen Phasen

wurden über Natriumsulfat getrocknet und eingeengt. Das Rohprodukt wurde an Kieselgel chromatographiert (Methylenchlorid/Ethanol (40:1)). Die gewünschten Fraktionen wurden vereinigt und eingedampft.
Ausbeute: 1.05 g (44 % der Theorie),
$C_{26}H_{25}N_7O_4S$ (531.6)
$R_f$-Wert: 0.72 (Kieselgel; Dichlormethan/Methanol = 9:1)

b. 1-Methyl-2-[N-[4-(N-hydroxylamidino)phenyl]-aminomethyl]-benzimidazol-5-yl-carbonsäure-N-(2-pyridyl)-N-[2-(methansulfonylaminocarbonyl)-ethyl]-amid

Hergestellt analog Beispiel 96 aus 1-Methyl-2-[N-(4-cyanophenyl)-aminomethyl]-benzimidazol-5-yl-carbonsäure-N-(2-pyridyl)-N-[2-(methansulfonylaminocarbonyl)-ethyl]-amid und Hydroxylamin.
Ausbeute: 27 % der Theorie,
$C_{26}H_{28}N_8O_5S$ (564.6)
$R_f$-Wert: 0.75 (Kieselgel; Dichlormethan/Ethanol = 7:3 + 1 % Eisessig)
EKA-Massenspektrum: $(M+H)^+ = 565$
$(M+Na)^+ = 587$

Beispiel 184

1-Methyl-2-[N-(5-amidino-thiazol-2-yl)-aminomethyl]-benzimidazol-5-yl-carbonsäure-N-(2-pyridyl)-N-(2-ethoxycarbonylethyl)-amid-hydrochlorid

Hergestellt analog Beispiel 25d aus 1-Methyl-2-[N-(5-cyanothiazol-2-yl)-aminomethyl]-benzimidazol-5-yl-carbonsäure-N-(2-pyridyl)-N-(2-ethoxycarbonylethyl)-amid und ethanolischer Salzsäure, Ethanol und Ammoniumcarbonat.

Beispiel 185

1-Methyl-2-[N-(5-amidino-thiazol-2-yl)-aminomethyl]-benzimidazol-5-yl-carbonsäure-N-(2-pyridyl)-N-(2-hydroxycarbonylethyl)-amid

Hergestellt analog Beispiel 26 aus 1-Methyl-2-[N-(5-amidinothiazol-2-yl)-aminomethyl]-benzimidazol-5-yl-carbonsäure-N-(2-pyridyl)-N-(2-ethoxycarbonylethyl)-amid-hydrochlorid und Natronlauge.

Beispiel 186

1-Methyl-2-[N-(2-amidino-pyrazin-5-yl)-aminomethyl]-benzimidazol-5-yl-carbonsäure-N-(2-pyridyl)-N-(2-ethoxycarbonylethyl)-amid-hydrochlorid

Hergestellt analog Beispiel 25d aus 1-Methyl-2-[N-(2-cyanopyrazin-5-yl)-aminomethyl]-benzimidazol-5-yl-carbonsäure-N-(2-pyridyl)-N-(2-ethoxycarbonylethyl)-amid und ethanolischer Salzsäure, Ethanol und Ammoniumcarbonat.
Ausbeute: 19 % der Theorie,
$C_{25}H_{27}N_9O_3$ (501.6)
$R_f$-Wert: 0.28 (Kieselgel; Dichlormethan/Methanol = 4:1 + 1 % Eisessig)
EKA-Massenspektrum: $(M+H)^+ = 502$
$(M+H+Na)^{++} = 262.5$

Beispiel 187

1-Methyl-2-[N-(2-amidino-pyrazin-5-yl)-aminomethyl]-benzimidazol-5-yl-carbonsäure-N-(2-pyridyl)-N-(2-hydroxycarbonylethyl)-amid

Hergestellt analog Beispiel 26 aus 1-Methyl-2-[N-(2-amidino-pyrazin-5-yl)-aminomethyl]-benzimidazol-5-yl-carbonsäure-N-(2-pyridyl)-N-(2-ethoxycarbonylethyl)-amid-hydrochlorid und Natronlauge.
Ausbeute: 11 % der Theorie,
$C_{23}H_{23}N_9O_3$ (473.5)

$R_f$-Wert: 0.55 (Reversed Phase Kieselgel RP-8; 5%ige Kochsalz-Lösung/Methanol = 6:4)
EKA-Massenspektrum: $(M+H)^+$ = 474
$(M+Na)^+$ = 496.6

Beispiel 188

1-Methyl-2-[2-[4-(N-n-hexyloxycarbonylamidino)phenyl]-ethyl]-benzimidazol-5-yl-carbonsäure-N-phenyl-N-[2-(1H-tetrazol-5-yl)-ethyl]-amid

Hergestellt analog Beispiel 90 aus 1-Methyl-2-[2-(4-amidinophenyl)-ethyl]-benzimidazol-5-yl-carbonsäure-N-phenyl-N-[2-(1H-tetrazol-5-yl)-ethyl]-amid und Chlorameisensäure-n-hexylester.

Beispiel 189

1-Methyl-2-[N-(2-methoxy-4-n-pentoxycarbonylamidino-phenyl)-aminomethyl]-benzimidazol-5-yl-carbonsäure-N-phenyl-N-(2-ethoxycarbonylethyl)-amid

Hergestellt analog Beispiel 90 aus 1-Methyl-2-[N-(4-amidino-2-methoxy-phenyl)-aminomethyl]-benzimidazol-5-yl-carbonsäure-N-phenyl-N-(2-ethoxycarbonylethyl)-amid-hydrochlorid und Chlorameisensäure-n-pentylester.
Ausbeute: 53 % der Theorie,
$C_{35}H_{42}N_6O_6$ (642.7)
$R_f$-Wert: 0.54 (Kieselgel; Dichlormethan/Ethanol = 9:1 )
EKA-Massenspektrum: $(M+H)^+$ = 643
$(M+H+Na)^{++}$ = 333.4

Beispiel 190

1-Methyl-2-[N-(4-n-heptyloxycarbonylamidino-2-methoxy-phenyl)-aminomethyl]-benzimidazol-5-yl-carbonsäure-N-phenyl-N-(2-ethoxycarbonylethyl)-amid

Hergestellt analog Beispiel 90 aus 1-Methyl-2-[N-(4-amidino-2-methoxy-phenyl)-aminomethyl]-benzimidazol-5-yl-carbonsäure-N-phenyl-N-(2-ethoxycarbonylethyl)-amid-hydrochlorid und Chlorameisensäure-n-heptylester.
Ausbeute: 68 % der Theorie,
$C_{37}H_{46}N_6O_6$ (670.8)
$R_f$-Wert: 0.56 (Kieselgel; Dichlormethan/Ethanol = 9:1 )
EKA-Massenspektrum: $(M+H)^+$ = 671
$(M+H+Na)^{++}$ = 347.4

Beispiel 191

1-Methyl-2-[N-(4-ethoxycarbonylamidino-2-methoxy-phenyl)-aminomethyl]-benzimidazol-5-yl-carbonsäure-N-(2-pyridyl)-N-(2-ethoxycarbonylethyl)-amid

Hergestellt analog Beispiel 90 aus 1-Methyl-2-[N-(4-amidino-2-methoxy-phenyl)-aminomethyl]-benzimidazol-5-yl-carbonsäure-N-(2-pyridyl)-N-(2-ethoxycarbonylethyl)-amid-hydrochlorid und Chlorameisensäure-ethylester.
Ausbeute: 43 % der Theorie,
$C_{31}H_{35}N_7O_6$ (601.7)
$R_f$-Wert: 0.44 (Kieselgel; Dichlormethan/Ethanol = 9:1)
EKA-Massenspektrum: $(M+H)^+$ = 602
$(M+H+Na)^{++}$ = 312.8

Beispiel 192

1-Methyl-2-[N-(2-methoxy-4-n-pentoxycarbonylamidino-phenyl)-aminomethyl]-benzimidazol-5-yl-carbonsäure-N-(2-pyridyl)-N-(2-ethoxycarbonylethyl)-amid

Hergestellt analog Beispiel 90 aus 1-Methyl-2-[N-(4-amidino-2-methoxy-phenyl)-aminomethyl]-benzimidazol-5-yl-carbonsäure-N-(2-pyridyl)-N-(2-ethoxycarbonylethyl)-amid-hydrochlorid und Chlorameisensäure-n-pentylester.

Ausbeute: 72 % der Theorie,
$C_{34}H_{41}N_7O_6$ (643.7)
$R_f$-Wert: 0.49 (Kieselgel; Dichlormethan/Ethanol = 9:1)
EKA-Massenspektrum: $(M+H)^+ = 644$
$(M+H+Na)^{++} = 333.9$

Beispiel 193

1-Methyl-2-[N-(2-methoxy-4-n-heptyloxycarbonylamidino-phenyl)-aminomethyl]-benzimidazol-5-yl-carbonsäure-N-(2-pyridyl)-N-(2-ethoxycarbonylethyl)-amid

Hergestellt analog Beispiel 90 aus 1-Methyl-2-[N-(4-amidino-2-methoxy-phenyl)-aminomethyl]-benzimidazol-5-yl-carbonsäure-N-(2-pyridyl)-N-(2-ethoxycarbonylethyl)-amid-hydrochlorid und Chlorameisensäure-n-heptylester.
Ausbeute: 55 % der Theorie,
$C_{36}H_{45}N_7O_6$ (671.8)
$R_f$-Wert: 0.54 (Kieselgel; Dichlormethan/Ethanol = 9:1)
EKA-Massenspektrum: $(M+H)^+ = 672$
$(M+H+Na)^{++} = 347.9$

Beispiel 194

Trockenampulle mit 75 mg Wirkstoff pro 10 ml

Zusammensetzung:

| | |
|---|---|
| Wirkstoff | 75,0 mg |
| Mannitol | 50,0 mg |
| Wasser für Injektionszwecke | ad 10,0 ml |

Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

Beispiel 195

Trockenampulle mit 35 mg Wirkstoff pro 2 ml

Zusammensetzung:

| | |
|---|---|
| Wirkstoff | 35,0 mg |
| Mannitol | 100,0 mg |
| Wasser für Injektionszwecke | ad 2,0 ml |

Herstellung:

Wirkstoff und Mannitol werden in Wasser gelöst. Nach Abfüllung wird gefriergetrocknet.
Die Auflösung zur gebrauchsfertigen Lösung erfolgt mit Wasser für Injektionszwecke.

Beispiel 196

Tablette mit 50 mg Wirkstoff

Zusammensetzung:

| | | |
|---|---|---|
| (1) | Wirkstoff | 50,0 mg |
| (2) | Milchzucker | 98,0 mg |
| (3) | Maisstgrke | 50,0 mg |
| (4) | Polyvinylpyrrolidon | 15,0 mg |
| (5) | Magnesiumstearat | 2,0 mg |
| | | 215,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreBt, biplan mit beidseitiger Facette und einseitiger Teilkerbe.
Durchmesser der Tabletten: 9 mm.

Beispiel 197

Tablette mit 350 mg Wirkstoff

Zusammensetzung:

| | | |
|---|---|---|
| (1) | Wirkstoff | 350,0 mg |
| (2) | Milchzucker | 136,0 mg |
| (3) | Maisstärke | 80,0 mg |
| (4) | Polyvinylpyrrolidon | 30,0 mg |
| (5) | Magnesiumstearat | 4,0 mg |
| | | 600,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe.
Durchmesser der Tabletten: 12 mm.

Beispiel 198

Kapseln mit 50 mg Wirkstoff

Zusammensetzung:

| | | |
|---|---|---|
| (1) | Wirkstoff | 50,0 mg |
| (2) | Maisstgrke getrocknet | 58,0 mg |
| (3) | Milchzucker pulverisiert | 50,0 mg |

(fortgesetzt)

| | | |
|---|---|---|
| (4) | Magnesiumstearat | 2,0 mg |
| | | 160,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 3 abgefüllt.

<u>Beispiel 199</u>

<u>Kapseln mit 350 mg Wirkstoff</u>

Zusammensetzung:

| | | |
|---|---|---|
| (1) | Wirkstoff | 350,0 mg |
| (2) | Maisstärke getrocknet | 46,0 mg |
| (3) | Milchzucker pulverisiert | 30,0 mg |
| (4) | Magnesiumstearat | 4,0 mg |
| | | 430,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Gr6Be 0 abgefüllt.

<u>Beispiel 200</u>

<u>Suppositorien mit 100 mg Wirkstoff</u>

| 1 | Zäpfchen enthält: | |
|---|---|---|
| | Wirkstoff | 100,0 mg |
| | Polyethylenglykol (M.G. 1500) | 600,0 mg |
| | Polyethylenglykol (M.G. 6000) | 460,0 mg |
| | Polyethylensorbitanmonostearat | 840,0 mg |
| | | 2 000,0 mg |

**Patentansprüche**

**1.** Disubstituierte bicyclische Heterocyclen der allgemeinen Formel

$$R_a - A - Het - B - Ar - E , \qquad (I)$$

in der

A eine mit dem Benzo-, Pyrido- oder Thienoteil des Restes Het verknüpfte Carbonyl- oder Sulfonylgruppe,

B eine Ethylengruppe, in der die Methylengruppe, die mit dem Rest Ar verknüpft ist, durch ein Sauerstoff- oder Schwefelatom oder durch eine $-NR_1$-Gruppe ersetzt sein kann, wobei

$R_1$ ein Wasserstoffatom oder eine $C_{1-4}$-Alkylgruppe darstellt,

E eine $R_bNH-C(=NH)$-Gruppe, in der

$R_b$ ein Wasserstoffatom, eine Hydroxy-, $C_{1-9}$-Alkoxycarbonyl-, Cyclohexyloxycarbonyl-, Phenyl-$C_{1-3}$-alkoxycarbonyl-, Benzoyl-, p-$C_{1-3}$-Alkyl-benzoyl- oder Pyridinoylgruppe, wobei der Ethoxyteil in 2-Stellung der vorstehend erwähnten $C_{1-9}$-Alkoxycarbonylgruppe zusätzlich durch eine $C_{1-3}$-Alkylsulfonyl- oder 2-($C_{1-3}$-Alkoxy)-ethylgruppe substituiert sein kann, darstellt,

Ar eine gegebenenfalls durch ein Chloratom, durch eine Methyl-, Ethyl- oder Methoxygruppe substituierte 1,4-Phenylengruppe oder eine 2,5-Thienylengruppe,

Het einen 1- ($C_{1-3}$-Alkyl)-2,5-benzimidazolylen-, 1-Cyclopropyl-2,5-benzimidazolylen-, 2,5-Benzthiazolylen-, 1-($C_{1-3}$-Alkyl)-2,5-indolylen-, 1-($C_{1-3}$-Alkyl)-2,5-imidazo[4,5-b]pyridinylen-, 3-($C_{1-3}$-Alkyl)-2,7-imidazo[1,2-a] pyridinylen- oder 1-($C_{1-3}$-Alkyl)-2,5-thieno[2,3-d]imidazolylengruppe und

$R_a$ eine $R_2NR_3$-Gruppe, in der

$R_2$ eine $C_{1-4}$-Alkylgruppe, die durch eine Carboxy-, $C_{1-6}$-Alkyloxycarbonyl-, Benzyloxycarbonyl-, $C_{1-3}$-Alkylsulfonylaminocarbonyl- oder 1H-Tetrazol-5-ylgruppe substituiert sein kann,

eine durch eine Hydroxy-, Benzyloxy-, Carboxy-$C_{1-3}$-alkylamino-, $C_{1-3}$-Alkoxycarbonyl-$C_{1-3}$-alkylamino-, N-($C_{1-3}$-Alkyl)-carboxy-$C_{1-3}$-alkylamino- oder N-($C_{1-3}$-Alkyl)-$C_{1-3}$-alkoxycarbonyl-$C_{1-3}$-alkylaminogruppe substituierte $C_{2-4}$-Alkylgruppe, wobei in den vorstehend erwähnten Gruppen das zum benachbarten Stickstoffatom stehende $\alpha$-Kohlenstoffatom nicht substituiert sein kann,

$R_3$ eine $C_{3-7}$-Cycloalkylgruppe, eine Propargylgruppe, wobei der ungesättigte Teil nicht direkt mit dem Stickstoffatom der $R_2NR_3$-Gruppe verknüpft sein kann, eine gegebenenfalls durch ein Fluor- oder Chloratom, durch eine Methyl- oder Methoxygruppe substituierte Phenylgruppe, eine gegebenenfalls durch eine Methylgruppe substituierte Pyrazolyl-, Pyridazolyl- oder Pyridinylgruppe oder

$R_2$ und $R_3$ zusammen mit dem dazwischenliegenden Stickstoffatom eine gegebenenfalls durch eine Carboxy- oder $C_{1-4}$-Alkoxycarbonylgruppe substituierte 5- bis 7-gliedrige Cycloalkyleniminogruppe, an welche zusätzlich ein Phenylring ankondensiert sein kann, darstellen.

bedeuten, deren Tautomere, deren Stereoisomere und deren Salze.

**2.** Disubstituierte bicyclische Heterocyclen der allgemeinen Formel I gemäß Anspruch 1, in der

A eine mit dem Benzo-, Pyrido- oder Thienoteil des Restes Het verknüpfte Carbonyl- oder Sulfonylgruppe,

B eine Ethylengruppe, in der die Methylengruppe, die mit dem Rest Ar verknüpft ist, durch ein Sauerstoff- oder Schwefelatom oder durch eine $-NR_1$-Gruppe ersetzt sein kann, wobei

$R_1$ ein Wasserstoffatom oder eine Methylgruppe darstellt,

E eine $R_bNH-C(=NH)$-Gruppe, in der

$R_b$ ein Wasserstoffatom, eine Hydroxy-, $C_{1-9}$-Alkoxycarbonyl-, Cyclohexyloxycarbonyl-, Benzyloxycarbonyl-, Benzoyl-, p-$C_{1-3}$-Alkyl-benzoyl- oder Nicotinoylgruppe, wobei der Ethoxyteil in 2-Stellung der vorstehend erwähnten $C_{1-9}$-Alkoxycarbonylgruppe zusätzlich durch eine $C_{1-3}$-Alkylsulfonyl- oder 2-($C_{1-3}$-Alkoxy)-ethylgruppe substituiert sein kann, darstellt,

Ar eine gegebenenfalls durch ein Chloratom, durch eine Methyl-, Ethyl- oder Methoxygruppe substituierte 1,4-Phenylengruppe oder eine 2,5-Thienylengruppe,

Het einen 1-Methyl-2,5-benzimidazolylen-, 1-Cyclopropyl-2,5-benzimidazolylen-, 2,5-Benzthiazolylen-, 1-Methyl-2,5-indolylen-, 1-Methyl-2,5-imidazo[4,5-b]pyridinylen-, 3-Methyl-2,7-imidazo [1,2-a]pyridinylen- oder 1-Methyl-2,5-thieno[2,3-d]-imidazolylengruppe und

$R_a$ eine $R_2NR_3$-Gruppe, in der

$R_2$ eine $C_{1-3}$-Alkylgruppe, die durch eine Carboxy-, $C_{1-6}$-Alkyloxycarbonyl-, Benzyloxycarbonyl-, Methylsulfonylaminocarbonyl- oder 1H-Tetrazol-5-ylgruppe substituiert sein kann,

eine durch eine Hydroxy-, Benzyloxy-, Carboxy-$C_{1-3}$-alkylamino-, $C_{1-3}$-Alkoxycarbonyl-$C_{1-3}$-alkylamino-, N-($C_{1-3}$-Alkyl)-carboxy-$C_{1-3}$-alkylamino- oder N-($C_{1-3}$-Alkyl)-$C_{1-3}$-alkoxycarbonyl-$C_{1-3}$-alkylaminogruppe substituierte $C_{2-3}$-Alkylgruppe, wobei in den vorstehend erwähnten Gruppen das zum benachbarten Stickstoffatom stehende $\alpha$-Kohlenstoffatom nicht substituiert sein kann, und

$R_3$ eine Propargylgruppe, wobei der ungesättigte Teil nicht direkt mit dem Stickstoffatom der $R_2NR_3$-Gruppe verknüpft sein kann, eine gegebenenfalls durch ein Fluor- oder Chloratom, durch eine Methyl- oder Methoxygruppe substituierte Phenylgruppe oder eine Pyridinylgruppe darstellen,

bedeuten, deren Tautomere, deren Stereoisomere und deren Salze.

**3.** Disubstituierte bicyclische Heterocyclen der allgemeinen Formel I gemäß Anspruch 1, in der

A eine mit dem Benzo- oder Thienoteil des Restes Het verknüpfte Carbonylgruppe,

B eine Ethylengruppe, in der die Methylengruppe, die mit dem Rest Ar verknüpft ist, durch eine $-NR_1$-Gruppe ersetzt sein kann, wobei

$R_1$ ein Wasserstoffatom oder eine Methylgruppe darstellt,

E eine $R_bNH-C(=NH)$-Gruppe, in der

$R_b$ ein Wasserstoffatom, eine Hydroxy-, $C_{1-9}$-Alkoxycarbonyl-, Cyclohexyloxycarbonyl-, Benzyloxycarbonyl-, Benzoyl-, p-$C_{1-3}$-Alkyl-benzoyl- oder Nicotinoylgruppe, wobei der Ethoxyteil in 2-Stellung der vorstehend erwähnten $C_{1-9}$-Alkoxycarbonylgruppe zusätzlich durch eine Methylsulfonyl - oder 2-Ethoxy-ethylgruppe substituiert sein kann, darstellt,

Ar eine gegebenenfalls durch eine Methoxygruppe substituierte 1,4-Phenylengruppe oder eine 2,5-Thienylengruppe,

Het einen 1-Methyl-2,5-benzimidazolylen-, 2,5-Benzthiazolylen-, 1-Methyl-2,5-indolylen- oder 1-Methyl-2,5-thieno[2,3-d]imidazolylengruppe und

$R_a$ eine $R_2NR_3$-Gruppe, in der

$R_2$ eine $C_{1-3}$-Alkylgruppe, die durch eine Carboxy-, $C_{1-6}$-Alkyloxycarbonyl-, Benzyloxycarbonyl-, Methylsulfonylaminocarbonyl- oder 1H-Tetrazol-5-ylgruppe substituiert sein kann,

eine durch eine Hydroxy-, Benzyloxy-, Carboxy-$C_{1-3}$-alkylamino-, $C_{1-3}$-Alkoxycarbonyl-$C_{1-3}$-alkylamino-, N-($C_{1-3}$-Alkyl)-carboxy-$C_{1-3}$-alkylamino- oder N-($C_{1-3}$-Alkyl)-$C_{1-3}$-alkoxycarbonyl-$C_{1-3}$-alkylaminogruppe substituierte $C_{2-3}$-Alkylgruppe, wobei in den vorstehend erwähnten Gruppen das zum benachbarten Stickstoffatom stehende $\alpha$-Kohlenstoffatom nicht substituiert sein kann, und

$R_3$ eine gegebenenfalls durch ein Fluoratom substituierte Phenylgruppe oder eine 2-Pyridinylgruppe darstellen,

bedeuten, deren Tautomere, deren Stereoisomere und deren Salze.

**4.** Folgende Verbindungen der allgemeinen Formel I:

(a) 2-[N-(4-Amidinophenyl)-aminomethyl]-benzthiazol-5-carbonsäure-N-phenyl-N-(2-carboxyethyl)-amid,

(b) 2-[N-(4-Amidinophenyl)-N-methyl-aminomethyl]-benzthiazol-5-yl-carbonsäure-N-phenyl-N-(2-hydroxycarbonylethyl)-amid,

(c) 1-Methyl-2-[N-(4-amidinophenyl)-aminomethyl]-benzimidazol-5-yl-carbonsäure-N-phenyl-N-(2-hydroxycarbonylethyl)-amid,

(d) 1-Methyl-2-[N-(4-amidinophenyl)-aminomethyl]-benzimidazol-5-yl-carbonsäure-N-phenyl-N-(3-hydroxycarbonylpropyl)-amid,

(e) 1-Methyl-2-[N-(4-amidinophenyl)-aminomethyl]-benzimidazol-5-yl-carbonsäure-N-(2-pyridyl)-N-(hydroxycarbonylmethyl)-amid,

(f) 1-Methyl-2-[2-(2-amidinothiophen-5-yl)ethyl]-benzimidazol-5-yl-carbonsäure-N-(2-pyridyl)-N-(2-hydroxycarbonylethyl)-amid,

(g) 1-Methyl-2-[N-(4-amidinophenyl)-aminomethyl]-benzimidazol-5-yl-carbonsäure-N-(2-pyridyl)-N-(2-hydroxycarbonylethyl)-amid,

(h) 1-Methyl-2-[2-(4-amidinophenyl)ethyl]-benzimidazol-5-yl-carbonsäure-N-(2-pyridyl)-N-(2-hydroxycarbonylethyl)-amid,

(i) 1-Methyl-2-[2-(4-amidinophenyl)ethyl]-benzimidazol-5-yl-carbonsäure-N-phenyl-N-(2-hydroxycarbonylethyl)-amid,

(j) 1-Methyl-2-[2-(4-amidinophenyl)ethyl]-benzimidazol-5-yl-carbonsäure-N-phenyl-N-[2-(1H-tetrazol-5-yl)ethyl]-amid,

(k) 1-Methyl-2-[N-(4-amidinophenyl)-aminomethyl]-benzimidazol-5-yl-carbonsäure-N-phenyl-N-[2-(1H-tetrazol-5-yl)ethyl]-amid,

(l) 1-Methyl-2-[N-(4-amidinophenyl)-N-methyl-aminomethyl]-benzimidazol-5-yl-carbonsäure-N-(2-pyridyl)-N-(2-hydroxycarbonylethyl) -amid,

(m) 1-Methyl-2-[N-(4-amidinophenyl)-N-methyl-aminomethyl]-benzimidazol-5-yl-carbonsäure-N-(3-pyridyl)-N-(2-hydroxycarbonylethyl)-amid,

(n) 1-Methyl-2-[N-(4-amidinophenyl)-N-methyl-aminomethyl]-benzimidazol-5-yl-carbonsäure-N-phenyl-N-(2-hydroxycarbonylethyl)-amid,

(o) 1-Methyl-2-[N-(4-amidinophenyl)-aminomethyl]-benzimidazol-5-yl-carbonsäure-N-phenyl-N-[(N-hydroxycarbonylethyl-N-methyl)-2-aminoethyl]-amid,

(p) 1-Methyl-2-[N-(4-amidinophenyl)-aminomethyl]-benzimidazol-5-yl-carbonsäure-N-(3-fluorphenyl)-N-(2-hydroxycarbonylethyl)-amid,

(q) 1-Methyl-2-[N-(4-amidinophenyl)-aminomethyl]-benzimidazol-5-yl-carbonsäure-N-(4-fluorphenyl)-N-(2-hydroxycarbonylethyl)-amid,

(r) 1-Methyl-2-[N-(4-amidino-2-methoxy-phenyl)-aminomethyl]-benzimidazol-5-yl-carbonsäure-N-phenyl-N-(2-hydroxycarbonylethyl)-amid,

(s) 1-Methyl-2-[N-(4-amidino-2-methoxy-phenyl)-aminomethyl]-benzimidazol-5-yl-carbonsäure-N-(2-pyridyl)-N-(2-hydroxycarbonylethyl)-amid,

(t) 1-Methyl-2-[N-(4-amidinophenyl)aminomethyl]-indol-5-yl-carbonsäure-N-phenyl-N-(2-methoxycarbonylethyl)-amid und

(u)1-Methyl-2-[N-(4-amidinophenyl)aminomethyl]-thieno[2.3-d]-imidazol-5-yl-carbonsäure-N-phenyl-N-(2-hydroxycarbonylethyl)-amid,

deren Tautomere, deren Stereoisomere und deren Salze.

**5.** 1-Methyl-2-[N-(4-amidinophenyl)-aminomethyl]-benzimidazol-5-yl-carbonsäure-N-phenyl-N-(2-hydroxycarbonylethyl)-amid, und dessen Salze.

**6.** 1-Methyl-2-[N-(4-amidinophenyl)-aminomethyl]-benzimidazol-5-yl-carbonsäure-N-(2-pyridyl)-N-(2-hydroxycarbonylethyl)-amid, und dessen Salze.

**7.** 1-Methyl-2-[N-(4-amidino-2-methoxy-phenyl)-aminomethyl]-benzimidazol-5-yl-carbonsäure-N-(2-pyridyl)-N-(2-hydroxycarbonylethyl)-amid, und dessen Salze.

**8.** 1-Methyl-2-[N-[4-(N-n-hexyloxycarbonylamidino)phenyl]-aminomethyl]-benzimidazol-5-yl-carbonsäure-N-(2-pyridyl)-N-(2-ethoxycarbonylethyl)-amid und dessen Salze.

**9.** Physiologisch verträgliche Salze der Verbindungen gemäß den Ansprüchen 1 bis 8.

**10.** Arzneimittel, enthaltend eine Verbindung nach mindestens einem der Ansprüche 1 bis 8 oder ein Salz gemäß Anspruch 9 neben gegebenenfalls einem oder mehreren inerten Trägerstoffen und/oder Verdünnungsmitteln.

**11.** Verwendung einer Verbindung nach mindestens einem der Ansprüche 1 bis 8 oder ein Salz gemäß Anspruch 9 zur Herstellung eines Arzneimittels mit einer die Thrombinzeit verlängernder Wirkung, einer thrombinhemmender Wirkung und einer Hemmwirkung auf verwandte Serinproteasen.

**12.** Verfahren zur Herstellung eines Arzneimittels gemäß Anspruch 10, **dadurch gekennzeichnet, daß** auf nichtchemischem Wege eine Verbindung nach mindestens einem der Ansprüche 1 bis 8 oder ein Salz gemäß Anspruch 9 in einen oder mehrere inerte Trägerstoffe und/oder Verdünnungsmittel eingearbeitet wird.

**13.** Verfahren zur Herstellung der Verbindungen gemäß den Ansprüchen 1 bis 9, **dadurch gekennzeichnet, daß**

a. zur Herstellung einer Verbindung der allgemeinen Formel I, in der E eine $R_bNH-C(=NH)$-Gruppe bedeutet, in der $R_b$ ein Wasserstoffatom, eine Hydroxy- oder $C_{1-3}$-Alkylgruppe darstellt, eine gegebenenfalls im Reaktionsgemisch gebildete Verbindung der allgemeinen Formel

$$R_a - A - Het - B - Ar - C(=NH) - Z_1 , \qquad (II)$$

in der

A, B, Ar, Het und $R_a$ wie in den Ansprüchen 1 bis 8 definiert sind und
$Z_1$ eine Alkoxy-, Aralkoxy-, Alkylthio- oder Aralkylthiogruppe darstellt, mit einem Amin der allgemeinen Formel

$$H_2N - R_b' , \qquad (III)$$

in der
$R_b'$ ein Wasserstoffatom, eine Hydroxy- oder $C_{1-3}$-Alkylgruppe darstellt, umgesetzt wird oder

b. zur Herstellung einer Verbindung der allgemeinen Formel I, in der die $R_a$-A-Gruppe und E mit der Maßgabe wie in den Ansprüchen 1 bis 8 erwähnt definiert sind, daß die $R_a$-A-Gruppe eine Carboxygruppe enthält und E wie in den Ansprüchen 1 bis 8 definiert ist oder die $R_a$-A-Gruppe wie in den Ansprüchen 1 bis 8 erwähnt definiert ist und E eine $NH_2-C(=NH)$-Gruppe darstellt oder die $R_a$-A-Gruppe eine Carboxygruppe enthält und E eine $NH_2-C(=NH)$-Gruppe darstellt, eine Verbindung der allgemeinen Formel

$$R_a' - A - Het - B - Ar - E' , \quad (IV)$$

in der

A, B, Ar und Het wie in den Ansprüchen 1 bis 8 definiert sind und
die $R_a$'-A-Gruppe und E' die für die $R_a$-A-Gruppe und E in den Ansprüchen 1 bis 8 erwähnten Bedeutungen mit der Maßgabe besitzen, daß die $R_a$'-A-Gruppe eine durch Hydrolyse, Behandeln mit einer Säure oder Base, Thermolyse oder Hydrogenolyse in eine Carboxylgruppe überführbare Gruppe enthält und E' wie in den Ansprüchen 1 bis 8 definiert ist oder E' eine durch Hydrolyse, Behandeln mit einer Säure oder Base, Thermolyse oder Hydrogenolyse in eine $NH_2$-C(=NH)-Gruppe überführbare Gruppe darstellt und die $R_a$'-A-Gruppe die für die $R_a$-A-Gruppe in den Ansprüchen 1 bis 8 erwähnten Bedeutungen aufweist oder die $R_a$'-A-Gruppe eine durch Hydrolyse, Behandeln mit einer Säure oder Base, Thermolyse oder Hydrogenolyse in eine Carboxylgruppe überführbare Gruppe enthält und E' eine durch Hydrolyse, Behandeln mit einer Säure oder Base, Thermolyse oder Hydrogenolyse in eine $NH_2$-C(=NH)-Gruppe überführbare Gruppe darstellt,
mittels Hydrolyse, Behandeln mit einer Säure oder Base, Thermolyse oder Hydrogenolyse in eine Verbindung der allgemeinen Formel I übergefürt wird, in der die $R_a$-A-Gruppe und E mit der Maßgabe wie in den Ansprüchen 1 bis 8 erwähnt definiert sind, daß die $R_a$-A-Gruppe eine Carboxygruppe enthält und E wie in den Ansprüchen 1 bis 8 definiert ist oder die $R_a$-A-Gruppe die in den Ansprüchen 1 bis 8 erwähnten Bedeutungen aufweist und E eine $NH_2$-C(=NH)-Gruppe darstellt oder die $R_a$-A-Gruppe eine Carboxygruppe enthält und E eine $NH_2$-C(=NH)-Gruppe darstellt, übergeführt wird oder

c. zur Herstellung einer Verbindung der allgemeinen Formel I, in der die $R_a$-A-Gruppe eine der bei der Definition der $R_a$-A-Gruppe in den Ansprüchen 1 bis 8 erwähnten Estergruppen enthält, eine Verbindung der allgemeinen Formel

$$R_a'' - A - Het - B - Ar - E , \quad (V)$$

in der

B, E, Ar und Het wie in den Ansprüchen 1 bis 8 definiert sind und
$R_a$"-A-Gruppe die für die $R_a$-A-Gruppe in den Ansprüchen 1 bis 8 erwähnten Bedeutungen mit der Maßgabe aufweist, daß die $R_a$"-A-Gruppe eine Carboxylgruppe oder eine mittels eines Alkohols in eine entsprechende Estergruppe überführbare Gruppe enthält, mit einem Alkohol der allgemeinen Formel

$$HO - R_7 , \quad (VI)$$

in der

$R_7$ eine $C_{1-6}$-Alkyl- oder Benzylgruppe darstellt, oder mit deren Formamidacetalen

oder mit einer Verbindung der allgemeinen Formel

$$Z_2 - R_7 , \quad (VII)$$

in der

$R_7$ eine $C_{1-6}$-Alkyl- oder Benzylgruppe und
$Z_2$ eine Austrittsgruppe darstellen, umgesetzt wird oder

d. zur Herstellung einer Verbindung der allgemeinen Formel I, in der $R_b$ eine $C_{1-9}$-Alkoxycarbonyl-, Cyclohexyloxycarbonyl-, Phenyl-$C_{1-3}$-alkoxycarbonyl-, Benzoyl-, p-$C_{1-3}$-Alkyl-benzoyl- oder Pyridinoylgruppe, wobei der Ethoxyteil in 2-Stellung der vorstehend erwähnten $C_{1-9}$-Alkoxycarbonylgruppe zusätzlich durch eine $C_{1-3}$-

Alkylsulfonyl- oder 2-($C_{1-3}$-Alkoxy)-ethylgruppe substituiert sein kann, darstellt, eine Verbindung der allgemeinen Formel

$$R_a - A - Het - B - Ar - C(=NH) - NH_2 , \qquad (VIII)$$

in der

$R_a$, A, Het, B und Ar wie in den Ansprüchen 1 bis 8 definiert sind, mit einer Verbindung der allgemeinen Formel

$$Z_2 - R_8 , \qquad (IX)$$

in der

$R_8$ eine $C_{1-9}$-Alkoxycarbonyl-, Cyclohexyloxycarbonyl-, Phenyl-$C_{1-3}$-alkoxycarbonyl-, Benzoyl-, p-$C_{1-3}$-Alkyl-benzoyl- oder Pyridinoylgruppe, wobei der Ethoxyteil in 2-Stellung der vorstehend erwähnten $C_{1-9}$-Alkoxycarbonylgruppe zusätzlich durch eine $C_{1-3}$-Alkylsulfonyl- oder 2-($C_{1-3}$-Alkoxy)-ethylgruppe substituiert sein kann, und
$Z_2$ eine nukleofuge Austrittsgruppe bedeuten, umgesetzt wird oder

e. zur Herstellung einer Benzimidazolyl- oder Benzthiazolyl verbindung der allgemeinen Formel I, in der B eine Ethylengruppe darstellt, eine Verbindung der allgemeinen Formel

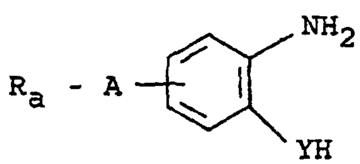 , (XV)

in der

$R_a$ und A wie in den Ansprüchen 1 bis 8 erwähnt definiert sind und Y ein Schwefelatom oder ein durch eine $C_{1-3}$-Alkyl- oder Cyclopropylgruppe substituiertes Stickstoffatom bedeutet, mit einer Verbindung der allgemeinen Formel

$$HO\text{-}CO - CH_2CH_2 - Ar - E , \qquad (XVI)$$

in der

Ar und E wie in den Ansprüchen 1 bis 8 erwähnt definiert sind, oder mit deren reaktionsfähigen Derivaten umgesetzt wird oder

f. zur Herstellung einer Verbindung der allgemeinen Formel I, in der $R_2$ eine $C_{1-4}$-Alkylgruppe, die durch eine Alkylsulfonylaminocarbonylgruppe substituiert ist, eine Verbindung der allgemeinen Formel

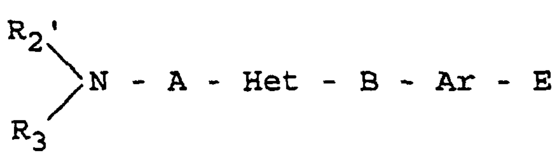 , (IXX)

in der

$R_3$, A, B, E, und Het wie in den Ansprüchen 1 bis 8 erwähnt definiert sind und
$R_2$' eine $C_{1-4}$-Alkylgruppe, die durch eine Carboxygruppe substituiert ist, darstellt oder deren reaktionsfähigen Derivaten,
mit einem Salz einer Verbindung der allgemeinen Formel

$$C_{1-3}\text{-Alkyl-}SO_2\text{-}NH_2 \ , \qquad (XX)$$

umgesetzt wird und

erforderlichenfalls ein während den Umsetzungen zum Schutze von reaktiven Gruppen verwendeter Schutzrest abgespalten wird und/oder

gewünschtenfalls anschließend eine so erhaltene Verbindung der allgemeinen Formel I in ihre Stereoisomere aufgetrennt wird und/oder

eine so erhaltene Verbindung der allgemeinen Formel I in ihre Salze, insbesondere für die pharmazeutische Anwendung in ihre physiologisch verträglichen Salze mit einer anorganischen oder organischen Säure oder Base, übergeführt wird.

## Claims

**1.** Disubstituted bicyclic heterocycles of general formula

$$R_a \text{ - A - Het - B - Ar - E} \ , \qquad (I)$$

wherein

A denotes a carbonyl or sulphonyl group linked to the benzo, pyrido or thieno moiety of the group Het,

B denotes an ethylene group in which the methylene group linked to the group Ar may be replaced by an oxygen or sulphur atom or by an $-NR_1-$ group, wherein

$R_1$ denotes a hydrogen atom or a $C_{1-4}$-alkyl group,

E denotes an $R_bNH-C(=NH)-$ group wherein

$R_b$ denotes a hydrogen atom, a hydroxy, $C_{1-9}$-alkoxycarbonyl, cyclohexyloxycarbonyl, phenyl-$C_{1-3}$-alkoxycarbonyl, benzoyl, p-$C_{1-3}$-alkyl-benzoyl or pyridinoyl group, whilst the ethoxy moiety in the 2-position of the abovementioned $C_{1-9}$-alkoxycarbonyl group may additionally be substituted by a $C_{1-3}$-alkyl-sulphonyl or 2-($C_{1-3}$-alkoxy)-ethyl group,

Ar denotes a 1,4-phenylene group optionally substituted by a chlorine atom or by a methyl, ethyl or methoxy group or it denotes a 2,5-thienylene group,

Het denotes a 1-($C_{1-3}$-alkyl)-2,5-benzimidazolylene, 1-cyclopropyl-2,5-benzimidazolylene, 2,5-benzothiazolylene, 1-($C_{1-3}$-alkyl)-2,5-ihdolylene, 1-($C_{1-3}$-alkyl)-2,5-imidazo[4,5-b]pyridinylene, 3-($C_{1-3}$-alkyl)-2,7-imidazo[1,2-a]pyridinylene or 1-($C_{1-3}$-alkyl)-2,5-thieno[2,3-d]imidazolylene group and

$R_a$ denotes an $R_2NR_3-$ group wherein

$R_2$ is a $C_{1-4}$-alkyl group which may be substituted by a carboxy, $C_{1-6}$-alkyloxycarbonyl, benzyloxycarbonyl, $C_{1-3}$-alkylsulphonylaminocarbonyl or 1H-tetrazol-5-yl group,

a $C_{2-4}$-alkyl group substituted by a hydroxy, benzyloxy, carboxy-$C_{1-3}$-alkylamino, $C_{1-3}$-alkoxycarbonyl-$C_{1-3}$-alkylamino, N-($C_{1-3}$-alkyl)-carboxy-$C_{1-3}$-alkylamino or N-($C_{1-3}$-alkyl)-$C_{1-3}$-alkoxycarbonyl-$C_{1-3}$-alkylamino group, whilst in the abovementioned groups the carbon atom in the $\alpha$-position to the adjacent nitrogen atom may not be substituted,

$R_3$ denotes a $C_{3-7}$-cycloalkyl group, a propargyl group, wherein the unsaturated part may not be linked directly to the nitrogen atom of the $R_2NR_3$ group, a phenyl group optionally substituted by a fluorine or chlorine atom, or by a methyl or methoxy group, a pyrazolyl, pyridazolyl or pyridinyl group optionally substituted by a methyl group or

$R_2$ and $R_3$ together with the nitrogen atom between them denote a 5- to 7-membered cycloalkyleneimino group, optionally substituted by a carboxy or
$C_{1-4}$-alkoxycarbonyl group, to which a phenyl ring may additionally be fused,

the tautomers, the stereoisomers and the salts thereof.

**2.** Disubstituted bicyclic heterocycles of general formula I according to claim 1, wherein

A denotes a carbonyl or sulphonyl group linked to the benzo, pyrido or thieno moiety of the group Het,

B denotes an ethylene group in which the methylene group linked to the group Ar may be replaced by an oxygen or sulphur atom or by an -$NR_1$- group, wherein

$R_1$ denotes a hydrogen atom or a methyl group,

E denotes an $R_bNH$-C(=NH)- group, wherein

$R_b$ denotes a hydrogen atom or a hydroxy, $C_{1-9}$-alkoxycarbonyl, cyclohexyloxycarbonyl, benzyloxycarbonyl, benzoyl, p-$C_{1-3}$-alkylbenzoyl or nicotinoyl group, whilst the ethoxy moiety in the 2-position of the abovementioned $C_{1-9}$-alkoxycarbonyl group may additionally be substituted by a $C_{1-3}$-alkylsulphonyl or 2-($C_{1-3}$-alkoxy)-ethyl group,

Ar denotes a 1,4-phenylene group optionally substituted by a chlorine atom or by a methyl, ethyl or methoxy group, or it denotes a 2,5-thienylene group,

Het denotes a 1-methyl-2,5-benzimidazolylene, 1-cyclopropyl-2,5-benzimidazolylene, 2, 5-benzothiazolylene, 1-methyl-2,5-indolylene, 1-methyl-2,5-imidazo[4,5-b]pyridinylene, 3-methyl-2,7-imidazo[1,2-a]pyridinylene or 1-methyl-2,5-thieno[2,3-d]imidazolylene group and

$R_a$ denotes a $R_2NR_3$- group wherein

$R_2$ denotes a $C_{1-3}$-alkyl group which may be substituted by a carboxy, $C_{1-6}$-alkyloxycarbonyl, benzyloxycarbonyl, methylsulphonylaminocarbonyl or 1H-tetrazol-5-yl group, a $C_{2-3}$-alkyl group substituted by a hydroxy, benzyloxy, carboxy-$C_{1-3}$-alkylamino, $C_{1-3}$-alkoxycarbonyl-$C_{1-3}$-alkylamino, N-($C_{1-3}$-alkyl)-carboxy-$C_{1-3}$-alkylamino or N-($C_{1-3}$-alkyl)-$C_{1-3}$-alkoxycarbonyl-$C_{1-3}$-alkylamino group, whilst in the abovementioned groups the carbon atom in the $\alpha$-position to the adjacent nitrogen atom may not be substituted, and

$R_3$ denotes a propargyl group, wherein the unsaturated moiety may not be linked directly to the nitrogen atom of the $R_2NR_3$ group, a phenyl group optionally substituted by a fluorine or chlorine atom, or by a methyl or methoxy group or it denotes a pyridinyl group,

the tautomers, the stereoisomers and the salts thereof.

**3.** Disubstituted bicyclic heterocycles of general formula I according to claim 1, wherein

A denotes a carbonyl group linked to the benzo or thieno moiety of the group Het,

B denotes an ethylene group wherein the methylene group attached to the group Ar may be replaced by an $-NR_1$ group, whilst

$R_1$ denotes a hydrogen atom or a methyl group,

E denotes an $R_bNH-C(=NH)-$ group wherein

$R_b$ is a hydrogen atom, a hydroxy, $C_{1-9}$-alkoxycarbonyl, cyclohexyloxycarbonyl, benzyloxycarbonyl, benzoyl, p-$C_{1-3}$-alkyl-benzoyl or nicotinoyl group, whilst the ethoxy moiety in the 2-position of the abovementioned $C_{1-9}$-alkoxycarbonyl group may additionally be substituted by a methylsulphonyl or 2-ethoxy-ethyl group,

Ar denotes a 1,4-phenylene group optionally substituted by a methoxy group or it denotes a 2,5-thienylene group,

Het denotes a 1-methyl-2,5-benzimidazolylene, 2,5-benzothiazolylene, 1-methyl-2,5-indolylene or 1-methyl-2,5-thieno[2,3-d]imidazolylene group and

$R_a$ denotes an $R_2NR_3$- group wherein

$R_2$ denotes a $C_{1-3}$-alkyl group which may be substituted by a carboxy, $C_{1-6}$-alkyloxycarbonyl, benzyloxycarbonyl, methylsulphonylaminocarbonyl or 1H-tetrazol-5-yl group,

a $C_{2-3}$-alkyl group substituted by a hydroxy, benzyloxy, carboxy-$C_{1-3}$-alkylamino, $C_{1-3}$-alkoxycarbonyl-$C_{1-3}$-alkylamino, N-($C_{1-3}$-alkyl)-carboxy-$C_{1-3}$-alkylamino or N-($C_{1-3}$-alkyl)-$C_{1-3}$-alkoxycarbonyl-$C_{1-3}$-alkylamino group, whilst in the abovementioned groups the carbon atom in the $\alpha$-position to the adjacent nitrogen atom may not be substituted, and

$R_3$ denotes a phenyl group optionally substituted by a fluorine atom, or it denotes a 2-pyridinyl group,

the tautomers, stereoisomers and the salts thereof.

**4.** The following compounds of general formula I:

(a) 2-[N-(4-amidinophenyl)-aminomethyl]-benzthiazole-5-carboxylic acid-N-phenyl-N-(2-carboxyethyl)-amide,

(b)2-[N-(4-midinophenyl)-N-methyl-aminomethyl]-benzthiazol-5-yl-carboxylicacid-N-phenyl-N-(2-hydroxycarbonylethyl)-amide,

(c) 1-Methyl-2-[N-(4-amidinophenyl)-aminomethyl]-benzimidazol-5-yl-carboxylic acid-N-phenyl-N-(2-hydroxycarbonylethyl)-amide,

(d) 1-Methyl-2-[N-(4-amidinophenyl)-aminomethyl]-benzimidazol-5-yl-carboxylic acid-N-phenyl-N-(3-hydroxycarbonylpropyl)-amide,

(e) 1-Methyl-2-[N-(4-amidinophenyl)-aminomethyl]-benzimidazol-5-yl-carboxylicacid-N-(2-pyridyl)-N-(hydroxycarbonylmethyl)-amide,

(f) 1-Methyl-2-[2-(2-amidinothiophen-5-yl)ethyl]-benzimidazol-5-yl-carboxylic acid-N-(2-pyridyl)-N-(2-hydroxycarbonylethyl)-amide,

(g) 1-Methyl-2-[N-(4-amidinophenyl)-aminomethyl]-benzimidazol-5-yl-carboxylic acid-N-(2-pyridyl)-N-(2-hydroxycarbonylethyl)-amide,

(h) 1-Methyl-2-[2-(4-amidinophenyl)ethyl]-benzimidazol-5-yl-carboxylic acid-N-(2-pyridyl)-N-(2-hydroxycarbonylethyl)-amide,

(i) 1-Methyl-2-[2-(4-amidinophenyl)ethyl]-benzimidazol-5-yl-carboxylic acid-N-phenyl-N-(2-hydroxycarbonylethyl)-amide,

(j) 1-Methyl-2-[2-(4-amidinophenyl)ethyl]-benzimidazol-5-yl-carboxylic acid-N-phenyl-N-[2-(1H-tetrazol-5-yl) ethyl]-amide,

(k) 1-Methyl-2-[N-(4-amidinophenyl)-aminomethyl]-benzimidazol-5-yl-carboxylic acid-N-phenyl-N-[2-(1H-tetrazol-5-yl)ethyl]-amide,

(l) 1-Methyl-2-[N-(4-amidinophenyl)-N-methyl-aminomethyl]-benzimidazol-5-yl-carboxylic acid-N-(2-pyridyl) -N-(2-hydroxycarbonylethyl)-amide,

(m) 1-Methyl-2-[N-(4-amidinophenyl)-N-methyl-aminomethyl]-benzimidazol-5-yl-carboxylic acid-N-(3-pyridyl) -N-(2-hydroxycarbonylethyl)-amide,

(n) 1-Methyl-2-[N-(4-amidinophenyl)-N-methyl-aminomethyl]-benzimidazol-5-yl-carboxylic acid-N-phenyl-N-(2-hydroxycarbonylethyl)-amide,

(o) 1-Methyl-2-[N-(4-amidinophenyl)-aminomethyl]-benzimidazol-5-yl-carboxylic acid-N-phenyl-N-[(N-hydroxycarbonylethyl-N-methyl)-2-aminoethyl]-amide,

(p) 1-Methyl-2-[N-(4-amidinophenyl)-aminomethyl]-benzimidazol-5-yl-carboxylic acid-N-(3-fluorophenyl) -N-(2-hydroxycarbonylethyl)-amide,

(q) 1-Methyl-2-[N-(4-amidinophenyl)-aminomethyl]-benzimidazol-5-yl-carboxylic acid-N-(4-fluorophenyl) -N-(2-hydroxycarbonylethyl)-amide,

(r) 1-Methyl-2-[N-(4-amidino-2-methoxy-phenyl)-aminomethyl]-benzimidazol-5-yl-carboxylic acid-N-phenyl-N-(2-hydroxycarbonylethyl)-amide,

(s) 1-Methyl-2-[N-(4-amidino-2-methoxy-phenyl)-aminomethyl]-benzimidazol-5-yl-carboxylic acid-N-(2-pyridyl)-N-(2-hydroxycarbonylethyl)-amide,

(t)1-Methyl-2-[N-(4-amidinophenyl)aminomethyl]-indol-5-yl-carboxylicacid-N-phenyl-N-(2-methoxycarbonylethyl)-amide and

(u) 1-Methyl-2-[N-(4-amidinophenyl)aminomethyl]-thieno[2.3-d]imidazol-5-yl-carboxylic acid-N-phenyl-N-(2-hydroxycarbonylethyl)-amide,

the tautomers, stereoisomers and the salts thereof.

**5.** 1-Methyl-2-[N-(4-amidinophenyl)-aminomethyl]-benzimidazol-5-yl-carboxylic acid-N-phenyl-N-(2-hydroxycarbonylethyl)-amide and the salts thereof.

**6.** 1-Methyl-2-[N-(4-amidinophenyl)-aminomethyl]-benzimidazol-5-yl-carboxylicacid-N-(2-pyridyl)-N-(2-hydroxycarbonylethyl)-amide and the salts thereof.

**7.** 1-Methyl-2-[N-(4-amidino-2-methoxy-phenyl)-aminomethyl]-benzimidazol-5-yl-carboxylicacid-N-(2-pyridyl)-N-(hydroxycarbonylethyl)-amide and the salts thereof.

**8.** 1-Methyl-2-[N-[4-(N-n-hexyloxycarbonylamidino)phenylaminomethyl]-benzimidazol-5-yl-carboxylicacid-N-(2-pyridyl)-N-(ethoxycarbonylethyl)-amide and the salts thereof.

**9.** Physiologically acceptable salts of the compounds according to claims 1 to 8.

**10.** Pharmaceutical compositions containing a compound according to at least one of claims 1 to 8, or a salt according to claim 9, optionally together with one or more inert carriers and/or diluents.

**11.** Use of a compound according to at least one of claims 1 to 8, or a salt according to claim 9, for preparing a pharmaceutical composition having the effect of prolonging the thrombin time, a thrombin-inhibiting effect and an inhibiting effect on related serine proteases.

**12.** Process for preparing a pharmaceutical composition according to claim 10, **characterised in that** a compound according to at least one of claims 1 to 8, or a salt according to claim 9, is incorporated in one or more inert carriers and/or diluents by a non-chemical method.

**13.** Process for preparing compounds according to claims 1 to 9, **characterised in that**

a. in order to prepare a compound of general formula I, wherein E denotes an $R_b$NH-C(=NH)- group, wherein $R_b$ is a hydrogen atom or a hydroxy or $C_{1-3}$-alkyl group, a compound of general formula

$$R_a - A - Het - B - Ar - C(=NH) - Z_1 , \qquad (II)$$

optionally formed in the reaction mixture,
wherein

A, B, Ar, Het and $R_a$ are defined as in claims 1 to 8 and

$Z_1$ denotes an alkoxy, aralkoxy, alkylthio or aralkylthio group, is reacted with an amine of general formula

$$H_2N- R_b' , \qquad (III)$$

wherein

$R_b'$ denotes a hydrogen atom, a hydroxy or $C_{1-3}$-alkyl group, or

b. in order to prepare a compound of general formula I, wherein the $R_a$-A- group and E are defined as in claims 1 to 8, with the proviso that the $R_a$-A- group contains a carboxy group and E is.defined as in claims 1 to 8 or the $R_a$-A-group is defined as in claims 1 to 8 and E denotes an $NH_2$-C(=NH)- group, or the $R_a$-A- group contains a carboxy group and E denotes an $NH_2$-C(=NH)- group, a compound of general formula

$$R_a' - A - Het - B - Ar - E' , \qquad (IV)$$

wherein

A, B, Ar and Het are defined as in claims 1 to 8 and

the $R_a'$-A- group and E' have the meanings given for the $R_a$-A- group and E in claims 1 to 8, with the proviso that the $R_a'$-A- group contains a group which can be converted into a carboxyl group by hydrolysis, treatment with an acid or base, thermolysis or hydrogenolysis and E is defined as in claims 1 to 8 or E' denotes a group which may be converted into an $NH_2$-C(=NH)- group by hydrolysis, treatment with an acid or base, thermolysis or hydrogenolysis and the $R_a'$-A- group has the meanings given for the $R_a$-A-group in claims 1 to 8, or the $R_a'$-A- group contains a group which may be converted into a carboxyl group by hydrolysis, treatment with an acid or base, thermolysis or hydrogenolysis and E' denotes a group which may be converted into an $NH_2$-C(=NH)- group by hydrolysis, treatment with an acid or base, thermolysis or hydrogenolysis,

is converted, by hydrolysis, treatment with an acid or base, thermolysis or hydrogenolysis, into a compound of general formula I, wherein the $R_a$-A- group and E are defined as in claims 1 to 8, with the proviso that the $R_a$-A- group contains a carboxy group and E is defined as in claims 1 to 8 or the $R_a$-A- group has the meanings given in claims 1 to 8 and E denotes an $NH_2$-C(=NH)- group or the $R_a$-A- group contains a carboxy group and E denotes an $NH_2$-C(=NH)- group, or

c. in order to prepare a compound of general formula I wherein the $R_a$-A- group contains one of the ester groups mentioned in the definition of the $R_a$-A- group in claims 1 to 8, a compound of general formula

$$R_a'' - A - Het - B - Ar - E, \qquad (V)$$

wherein

B, E, Ar and Het are defined as in claims 1 to 8 and $R_a''$-A- group has the meanings given for the $R_a$-A- group in claims 1 to 8, with the proviso that the $R_a''$-A- group contains a carboxyl group or a group which may be converted by means of an alcohol into a corresponding ester group, is reacted with an alcohol of general formula

$$HO - R_7 , \qquad (VI)$$

wherein

$R_7$ denotes a $C_{1-6}$-alkyl or benzyl group, or with the formamide acetals thereof

or with a compound of general formula

$$Z_2 - R_7 \qquad (VII)$$

wherein

$R_7$ denotes a $C_{1-6}$-alkyl or benzyl group and
$Z_2$ denotes a leaving group, or

d. in order to prepare a compound of general formula I wherein $R_b$ denotes a $C_{1-9}$-alkoxycarbonyl, cyclohexyloxycarbonyl, phenyl-$C_{1-3}$-alkoxycarbonyl, benzoyl, p-$C_{1-3}$-alkyl-benzoyl or pyridinoyl group, whilst the ethoxy moiety in the 2-position of the abovementioned $C_{1-9}$-alkoxycarbonyl group may additionally be substituted by a $C_{1-3}$-alkylsulphonyl or 2-($C_{1-3}$-alkoxy)-ethyl group, a compound of general formula

$$R_a - A - Het - B - Ar - C(=NH) - NH_2 , \qquad (VIII)$$

wherein

$R_a$, A, Het, B and Ar are defined as in claims 1 to 8, is reacted with a compound of general formula

$$Z_2 - R_8 , \qquad (IX)$$

wherein

$R_8$ denotes a $C_{1-9}$-alkoxycarbonyl, cyclohexyloxycarbonyl, phenyl-$C_{1-3}$-alkoxycarbonyl, benzoyl, p-$C_{1-3}$-alkyl-benzoyl or pyridinoyl group, whilst the ethoxy moiety in the 2-position of the abovementioned $C_{1-9}$-alkoxycarbonyl group may additionally be substituted by a $C_{1-3}$-alkylsulphonyl or 2-($C_{1-3}$-alkoxy)-ethyl group, and
$Z_2$ denotes a nucleofugic leaving group, or

e. in order to prepare a benzimidazolyl or benzothiazolyl compound of general formula I, wherein B denotes an ethylene group, a compound of general formula

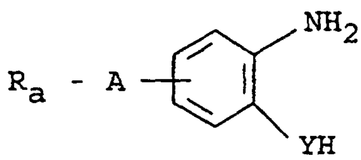

, (XV)

wherein

$R_a$ and A are defined as in claims 1 to 8 and Y denotes a sulphur atom, or a nitrogen atom substituted by a $C_{1-3}$ alkyl or cyclopropyl group, is reacted with a compound of general formula

$$HO\text{-}CO - CH_2CH_2 - Ar - E, \qquad (XVI)$$

wherein

Ar and E are defined as in claims 1 to 8, or with the reactive derivatives thereof and

f. in order to prepare a compound of general formula I wherein $R_2$ denotes a $C_{1-4}$-alkyl group substituted by an alkylsulphonylaminocarbonyl group:

a compound of general formula

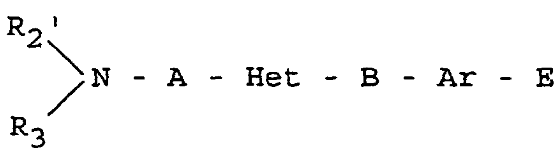

, (IXX)

wherein

$R_3$, A, B, E, and Het are defined as in claims 1 to 8 and $R_2$' denotes a $C_{1-4}$-alkyl group substituted by a carboxy group, or the reactive derivatives thereof,
is reacted with a salt of a compound of general formula

$$C_{1-3}\text{-Alkyl-}SO_2\text{-}NH_2, \qquad (XX),$$

and, if necessary, a protecting group used during the reactions in order to protect reactive groups is cleaved and/or

subsequently, if desired, a compound of general formula I thus obtained is resolved into the stereoisomers thereof and/or

a compound of general formula I thus obtained is converted into the salts thereof, more particularly for pharmaceutical use into the physiologically acceptable salts thereof with an inorganic or organic acid or base.

## Revendications

**1.** Hétérocycles bicycliques disubstitués de formule générale

$$R_a - A - Het - B - Ar - E \quad (I)$$

où

A représente un groupe carbonyle ou sulfonyle lié à la partie benzo, pyrido ou thiéno du reste Het,
B représente un groupe éthylène dans lequel le groupe méthylène qui est lié au reste Ar peut être remplacé par un atome d'oxygène ou de soufre ou par un groupe $-NR_1-$ où
$R_1$ représente un atome d'hydrogène ou un groupe alkyle en $C_{1-4}$,
E représente un groupe $R_bNH-C(=NH)-$ où
$R_b$ représente un atome d'hydrogène, un groupe hydroxyle, alcoxy en $C_{1-9}$-carbonyle, cyclohexyloxycarbonyle, phényle-alcoxy en $C_{1-3}$-carbonyle, benzoyle, p-alkyle en $C_{1-3}$-benzoyle ou pyridinoyle, où la partie éthoxy en position 2 du groupe alcoxy en $C_{1-9}$-carbonyle cité précédemment peut être substituée en outre par un groupe alkyle en $C_{1-3}$-sulfonyle ou 2-(alcoxy en $C_{1-3}$)-éthyle,
Ar représente un groupe 1,4-phénylène éventuellement substitué par un atome de chlore, par un groupe méthyle, éthyle ou méthoxy, ou un groupe 2,5-thiénylène,
Het représente un groupe 1-(alkyle en $C_{1-3}$)-2,5-benzimidazolylène, 1-cyclopropyl-2,5-benzimidazolylène, 2,5-benzothiazolylène, 1-(alkyle en $C_{1-3}$)-2,5-indolylène, 1-(alkyle en $C_{1-3}$)-2,5-imidazo[4,5-b]-pyridinylène, 3-(alkyle en $C_{1-3}$)-2,7-imidazo[1,2-a]-pyridinylène ou 1-(alkyle en $C_{1-3}$)-2,5-thiéno[2,3-d]-imidazolylène et
$R_a$ représente un groupe $R_2NR_3-$ où
$R_2$ représente un groupe alkyle en $C_{1-4}$, qui peut être substitué par un groupe carboxyle, alkyle en $C_{1-6}$-oxycarbonyle, benzyloxycarbonyle, alkyle en $C_{1-3}$-sulfonylaminocarbonyle ou 1H-tétrazol-5-yle,
un groupe alkyle en $C_{2-4}$ substitué par un groupe hydroxyle, benzyloxy, carboxy-alkyle en $C_{1-3}$-amino, alcoxy en $C_{1-3}$-carbonyl-alkyle en $C_{1-3}$-amino, N-(alkyle en $C_{1-3}$)-carboxy-alkyle en $C_{1-3}$-amino ou N-(alkyle en $C_{1-3}$)-alcoxy en $C_{1-3}$-carbonyl-alkyle en $C_{1-3}$-amino, où dans les groupes cités précédemment l'atome de carbone en $\alpha$ de l'atome d'azote voisin ne peut pas être substitué,
$R_3$ représente un groupe cycloalkyle en $C_{3-7}$, un groupe propargyle, où la partie insaturée ne peut pas être liée directement à l'atome d'azote du groupe $R_2NR_3-$, un groupe phényle éventuellement substitué par un atome de fluor ou de chlore, par un groupe méthyle ou méthoxy, un groupe pyrazolyle, pyridazolyle ou pyridinyle éventuellement substitué par un groupe méthyle ou
$R_2$ et $R_3$ représentent avec l'atome d'azote situé entre eux un groupe cycloalkylèneimino à 5 à 7 chaînons éventuellement substitué par un groupe carboxyle ou alcoxy en $C_{1-4}$-carbonyle, avec lequel, en outre, un cycle phényle peut être condensé,
leurs tautomères, leurs stéréoisomères et leurs sels.

**2.** Hétérocycles bicycliques disubstitués de formule générale I selon la revendication 1 où

A représente un groupe carbonyle ou sulfonyle lié à la partie benzo, pyrido ou thiéno du reste Het,
B représente un groupe éthylène dans lequel le groupe méthylène qui est lié au reste Ar peut être remplacé par un atome d'oxygène ou de soufre ou par un groupe $-NR_1-$ où
$R_1$ représente un atome d'hydrogène ou un groupe méthyle,
E représente un groupe $R_bNH-C(=NH)-$ où
$R_b$ représente un atome d'hydrogène, un groupe hydroxyle, alcoxy en $C_{1-9}$-carbonyle, cyclohexyloxycarbonyle, benzyloxycarbonyle, benzoyle, p-alkyle en $C_{1-3}$-benzoyle ou nicotinoyle, où la partie éthoxy en position 2 du groupe alcoxy en $C_{1-9}$-carbonyle cité précédemment peut être substituée en outre par un groupe alkyle en $C_{1-3}$-sulfonyle ou 2-(alcoxy en $C_{1-3}$)-éthyle,
Ar représente un groupe 1,4-phénylène éventuellement substitué par un atome de chlore, par un groupe méthyle, éthyle ou méthoxy, ou un groupe 2,5-thiénylène,
Het représente un groupe 1-méthyl-2,5-benzimidazolylène, 1-cyclopropyl-2,5-benzimidazolylène, 2,5-benzothiazolylène, 1-méthyl-2,5-indolylène, 1-méthyl-2,5-imidazo[4,5-b]pyridinylène, 3-méthyl-2,7-imidazo[1,2-a]pyridinylène ou 1-méthyl-2,5-thiéno[2,3-d]-imidazolylène et
$R_a$ représente un groupe $R_2NR_3-$ où
$R_2$ représente un groupe alkyle en $C_{1-3}$, qui peut être substitué par un groupe carboxyle, alkyle en $C_{1-6}$-oxycarbonyle, benzyloxycarbonyle, méthylsulfonylaminocarbonyle ou 1H-tétrazol-5-yle,
un groupe alkyle en $C_{2-3}$ substitué par un groupe hydroxyle, benzyloxy, carboxy-alkyle en $C_{1-3}$-amino, alcoxy en $C_{1-3}$-carbonyl-alkyle en $C_{1-3}$-amino, N-(alkyle en $C_{1-3}$)-carboxy-alkyle en $C_{1-3}$-amino ou N-(alkyle en $C_{1-3}$)-alcoxy en $C_{1-3}$-carbonyl-alkyle en $C_{1-3}$-amino, où, dans les groupes cités précédemment, l'atome de carbone

en $\alpha$ de l'atome d'azote voisin ne peut pas être substitué, et
$R_3$ représente un groupe propargyle, où la partie insaturée ne peut pas être liée directement à l'atome d'azote du groupe $R_2NR_3$-, un groupe phényle éventuellement substitué par un atome de fluor ou de chlore, par un groupe méthyle ou méthoxy, ou un groupe pyridinyle,

leurs tautomères, leurs stéréoisomères et leurs sels.

**3.** Hétérocycles bicycliques disubstitués de formule générale I selon la revendication 1 où

A représente un groupe carbonyle lié à la partie benzo ou thiéno du reste Het,
B représente un groupe éthylène dans lequel le groupe méthylène qui est lié au reste Ar peut être remplacé par un groupe $-NR_1$- où
$R_1$ représente un atome d'hydrogène ou un groupe méthyle,
E représente un groupe $R_bNH-C(=NH)$- où
$R_b$ représente un atome d'hydrogène, un groupe hydroxyle, alcoxy en $C_{1-9}$-carbonyle, cyclohexyloxycarbonyle, benzyloxycarbonyle, benzoyle, p-alkyle en $C_{1-3}$-benzoyle ou nicotinoyle, où la partie éthoxy en position 2 du groupe alcoxy en $C_{1-9}$-carbonyle cité précédemment peut être substituée en outre par un groupe méthylsulfonyle ou 2-éthoxyéthyle,
Ar représente un groupe 1,4-phénylène éventuellement substitué par un groupe méthoxy, ou un groupe 2,5-thiénylène,
Het représente un groupe 1-méthyl-2,5-benzimidazolylène, 2,5-benzothiazolylène, 1-méthyl-2,5-indolylène ou 1-méthyl-2,5-thiéno[2,3-d]imidazolylène et
$R_a$ représente un groupe $R_2NR_3$- où
$R_2$ représente un groupe alkyle en $C_{1-3}$, qui peut être substitué par un groupe carboxyle, alkyle en $C_{1-6}$-oxycarbonyle, benzyloxycarbonyle, méthylsulfonylaminocarbonyle ou 1H-tétrazol-5-yle,
un groupe alkyle en $C_{2-3}$ substitué par un groupe hydroxyle, benzyloxy, carboxy-alkyle en $C_{1-3}$-amino, alcoxy en $C_{1-3}$-carbonyl-alkyle en $C_{1-3}$-amino, N-(alkyle en $C_{1-3}$)-carboxy-alkyle en $C_{1-3}$-amino ou N-(alkyle en $C_{1-3}$)-alcoxy en $C_{1-3}$-carbonyl-alkyle en $C_{1-3}$-amino, où, dans les groupes cités précédemment, l'atome de carbone en $\alpha$ de l'atome d'azote voisin ne peut pas être substitué, et
$R_3$ représente un groupe phényle éventuellement substitué par un atome de fluor, ou un groupe 2-pyridinyle,

leurs tautomères, leurs stéréoisomères et leurs sels.

**4.** Composés de formule générale I suivants :

(a) N-phényl-N-(2-carboxyéthyl)-amide d'acide 2-[N-(4-amidinophényl)-aminométhyl]-benzothiazol-5-carboxylique,

(b) N-phényl-N-(2-hydroxycarbonyléthyl)-amide d'acide 2-[N-(4-amidinophényl)-N-méthyl-aminométhyl]-benzothiazol-5-yl-carboxylique,

(c) N-phényl-N-(2-hydroxycarbonyléthyl)-amide d'acide 1-méthyl-2-[N-(4-amidinophényl)-aminométhyl]-benzimidazol-5-yl-carboxylique,

(d) N-phényl-N-(3-hydroxycarbonylpropyl)-amide d'acide 1-méthyl-2-[N-(4-amidinophényl)-aminométhyl]-benzimidazol-5-yl-carboxylique,

(e) N-(2-pyridyl)-N-(hydroxycarbonylméthyl)-amide d'acide 1-méthyl-2-[N-(4-amidinophényl)-aminométhyl]-benzimidazol-5-yl-carboxylique,

(f) N-(2-pyridyl)-N-(2-hydroxycarbonyléthyl)-amide d'acide 1-méthyl-2-[2-(2-amidinothiophén-5-yl)éthyl]-benzimidazol-5-yl-carboxylique,

(g) N-(2-pyridyl)-N-(2-hydroxycarbonyléthyl)-amide d'acide 1-méthyl-2-[N-(4-amidinophényl)-aminométhyl]-benzimidazol-5-yl-carboxylique,

(h) N-(2-pyridyl)-N-(2-hydroxycarbonyléthyl)-amide d'acide 1-méthyl-2-[2-(4-amidinophényl)éthyl]-benzimidazol-5-yl-carboxylique,

(i) N-phényl-N-(2-hydroxycarbonyléthyl)-amide d'acide 1-méthyl-2-[2-(4-amidinophényl)éthyl]-benzimidazol-5-yl-carboxylique,

(j) N-phényl-N-[2-(1H-tétrazol-5-yl)éthyl]-amide d'acide 1-méthyl-2-[2-(4-amidinophényl)éthyl]-benzimidazol-5-yl-carboxylique,

(k) N-phényl-N-[2-(1H-tétrazol-5-yl)éthyl]-amide d'acide 1-méthyl-2-[N-(4-amidinophényl)-aminométhyl]-benzimidazol-5-yl-carboxylique,

(l) N-(2-pyridyl)-N-(2-hydroxycarbonyléthyl)-amide d'acide 1-méthyl-2-[N-(4-amidinophényl)-N-méthyl-aminométhyl]-benzimidazol-5-yl-carboxylique,

(m) N-(3-pyridyl)-N-(2-hydroxycarbonyléthyl)-amide d'acide 1-méthyl-2-[N-(4-amidinophényl}-N-méthyl-aminométhyl]-benzimidazol-5-yl-carboxylique,

(n) N-phényl-N-(2-hydroxycarbonyléthyl)-amide d'acide 1-méthyl-2-[N-(4-amidinophényl)-N-méthyl-aminométhyl]-benzimidazol-5-yl-carboxylique,

(o) N-phényl-N-[(N-hydroxycarbonyléthyl-N-méthyl)-2-aminoéthyl]-amide d'acide 1-méthyl-2-[N-(4-amidinophényl)-aminométhyl]-benzimidazol-5-yl-carboxylique,

(p) N-(3-fluorophényl)-N-(2-hydroxycarbonyléthyl)-amide d'acide 1-méthyl-2-[N-(4-amidinophényl)-aminométhyl]-benzimidazol-5-yl-carboxylique,

(q) N-(4-fluorophényl)-N-(2-hydroxycarbonyléthyl)-amide d'acide 1-méthyl-2-[N-(4-amidinophényl)-aminométhyl]-benzimidazol-5-yl-carboxylique,

(r) N-phényl-N-(2-hydroxycarbonyléthyl)-amide d'acide 1-méthyl-2-[N-(4-amidino-2-méthoxyphényl)-aminométhyl]-benzimidazol-5-yl-carboxylique,

(s) N-(2-pyridyl)-N-(2-hydroxycarbonyléthyl)-amide d'acide 1-méthyl-2-[N-(4-amidino-2-méthoxy-phényl)-aminométhyl]-benzimidazol-5-yl-carboxylique,

(t) N-phényl-N-(2-méthoxycarbonyléthyl)-amide d'acide 1-méthyl-2-[N-(4-amidinophényl)aminométhyl]-indol-5-yl-carboxylique et

(u) N-phényl-N-(2-hydroxycarbonyléthyl)-amide d'acide 1-méthyl-2-[N-(4-amidinophényl)aminométhyl]-thiéno [2.3-d]imidazol-5-yl-carboxylique,

leurs tautomères, leurs stéréoisomères et leurs sels.

**5.** N-phényl-N-(2-hydroxycarbonyléthyl)-amide d'acide 1-méthyl-2-[N-(4-amidinophényl)-aminométhyl]-benzimidazol-5-yl-carboxylique et ses sels.

**6.** N-(2-pyridyl)-N-(2-hydroxycarbonyléthyl)-amide d'acide 1-méthyl-2-[N-(4-amidinophényl)-aminométhyl]-benzimidazol-5-yl-carboxylique et ses sels.

**7.** N-(2-pyridyl)-N-(2-hydroxycarbonyléthyl)-amide d'acide 1-méthyl-2-[N-(4-amidino-2-méthoxyphényl)-aminométhyl]-benzimidazol-5-yl-carboxylique et ses sels.

**8.** N-(2-pyridyl)-N-(2-éthoxycarbonyléthyl)-amide d'acide 1-méthyl-2-[N-[4-(N-n-hexyloxycarbonylamidino)phényl]-aminométhyl]-benzimidazol-5-yl-carboxylique et ses sels.

**9.** Sels physiologiquement acceptables des composés selon les revendications 1 à 8.

**10.** Médicament contenant un composé selon au moins l'une des revendications 1 à 8 ou un sel selon la revendication 9 outre éventuellement un ou plusieurs supports et/ou diluants inertes.

**11.** Utilisation d'un composé selon au moins l'une des revendications 1 à 8 ou d'un sel selon la revendication 9 pour la préparation d'un médicament ayant un effet prolongeant le temps de thrombine, un effet inhibant la thrombine et un effet inhibiteur sur les protéases à sérine apparentées.

**12.** Procédé de préparation d'un médicament selon la revendication 10 **caractérisé en ce que**, par voie non chimique, un composé selon au moins l'une des revendications 1 à 8 ou un sel selon la revendication 9 est incorporé dans un ou plusieurs supports et/ou diluants inertes.

**13.** Procédé de préparation des composés selon les revendications 1 à 9 **caractérisé en ce que**

a. pour la préparation d'un composé de formule générale I où E représente un groupe $R_bNH-C(=NH)-$ où $R_b$ représente un atome d'hydrogène, un groupe hydroxyle ou alkyle en $C_{1-3}$, un composé, éventuellement formé dans le mélange réactionnel, de formule générale

$$R_a- A - Het - B - Ar - C(=NH) - Z_1 \quad (II)$$

où

A, B, Ar, Het et $R_a$ sont définis comme dans les revendications 1 à 8 et
$Z_1$ représente un groupe alcoxy, aralcoxy, alkylthio ou aralkylthio, est mis à réagir avec une amine de formule générale

$$H_2N - R_b' \quad (III)$$

où

$R_b'$ représente un atome d'hydrogène, un groupe hydroxyle ou alkyle en $C_{1-3}$, ou bien
b. pour la préparation d'un composé de formule générale I où le groupe $R_a$-A et E sont définis comme dans les revendications 1 à 8 avec la condition que le groupe $R_a$-A contient un groupe carboxyle et E est défini comme dans les revendications 1 à 8 ou le groupe $R_a$-A est défini de la manière indiquée dans les revendications 1 à 8 et E représente un groupe $NH_2$-C(=NH)- ou le groupe $R_a$-A contient un groupe carboxyle et E représente un groupe $NH_2$-C(=NH)-, un composé de formule générale

$$R_a'- A - Het - B - Ar - E' \quad (IV)$$

où

A, B, Ar et Het sont définis comme dans les revendications 1 à 8 et
le groupe $R_a'$-A et E' possèdent les significations indiquées pour le groupe $R_a$-A et pour E dans les revendications 1 à 8 avec la condition que le groupe $R_a'$-A contient un groupe pouvant être converti en un groupe carboxyle par hydrolyse, traitement avec un acide ou une base, thermolyse ou hydrogénolyse et E est défini comme dans les revendications 1 à 8 ou bien E' est un groupe pouvant être converti en un groupe $NH_2$-C(=NH)- par hydrolyse, traitement avec un acide ou une base, thermolyse ou hydrogénolyse et le groupe $R_a'$-A présente les significations indiquées dans les revendications 1 à 8 pour le groupe $R_a$-A ou le groupe $R_a'$-A contient un groupe pouvant être converti en un groupe carboxyle par hydrolyse, traitement avec un acide ou une base, thermolyse ou hydrogénolyse et E' représente un groupe pouvant être converti en un groupe $NH_2$-C(=NH)- par hydrolyse, traitement avec un acide ou une base, thermolyse ou hydrogénolyse,
est converti par hydrolyse, traitement avec un acide ou une base, thermolyse ou hydrogénolyse en un composé de formule générale I où le groupe $R_a$-A et E sont définis de la manière indiquée dans les revendications 1 à 8 avec la condition que le groupe $R_a$-A contient un groupe carboxyle et E est défini comme dans les revendications 1 à 8 ou le groupe $R_a$-A présente les significations indiquées dans les revendications 1 à 8 et E représente un groupe $NH_2$-C(=NH)- ou le groupe $R_a$-A contient un groupe carboxyle et E représente un groupe $NH_2$-C(=NH)-,

ou

c. pour la préparation d'un composé de formule générale I où le groupe $R_a$-A contient l'un des groupes ester cités dans les revendications 1 à 8 lors de la définition du groupe $R_a$-A, un composé de formule générale

$$R_a'' - A - Het - B - Ar - E \quad (V)$$

où

B, E, Ar et Het sont définis comme dans les revendications 1 à 8 et le groupe $R_a$"-A présente les significations indiquées dans les revendications 1 à 8 pour le groupe $R_a$-A avec la condition que le groupe $R_a$"-A contient un groupe carboxyle ou un groupe pouvant être converti en un groupe ester correspondant au moyen d'un alcool, est mis à réagir avec un alcool de formule générale

$$HO - R_7 \quad (VI)$$

où

$R_7$ représente un groupe alkyle en $C_{1-6}$ ou benzyle, ou avec ses formamide-acétals ou avec un composé de formule générale

$$Z_2 - R_7 \quad (VII)$$

où

$R_7$ représente un groupe alkyle en $C_{1-6}$ ou benzyle et
$Z_2$ représente un groupe partant, ou
d. pour la préparation d'un composé de formule générale I où $R_b$ représente un groupe alcoxy en $C_{1-9}$-carbonyle, cyclohexyloxycarbonyle, phényl-alcoxy en $C_{1-3}$-carbonyle, benzoyle, p-alkyle en $C_{1-3}$-benzoyle ou pyridinoyle, où la partie éthoxy en position 2 du groupe alcoxy en $C_{1-9}$-carbonyle évoqué précédemment peut être substituée en outre par un groupe alkyle en $C_{1-3}$-sulfonyle ou 2-(alcoxy en $C_{1-3}$)-éthyle, un composé de formule générale

$$R_a - A - Het - B - Ar - C(=NH) - NH_2 \quad (VIII)$$

où

$R_a$, A, Het, B et Ar sont définis comme dans les revendications 1 à 8, est mis à réagir avec un composé de formule générale

$$Z_2 - R_8 \quad (IX)$$

où

$R_8$ représente un groupe alcoxy en $C_{1-9}$-carbonyle, cyclohexyloxycarbonyle, phényl-alcoxy en $C_{1-3}$-carbonyle, benzoyle, p-alkyle en $C_{1-3}$-benzoyle ou pyridinoyle, où la partie éthoxy en position 2 du groupe alcoxy en $C_{1-9}$-carbonyle évoqué précédemment peut être substituée en outre par un groupe alkyle en $C_{1-3}$-sulfonyle ou 2-(alcoxy en $C_{1-3}$)-éthyle, et
$Z_2$ représente un groupe partant nucléofuge, ou

e. pour la préparation d'un composé benzimidazolyle ou benzothiazolyle de formule générale I où B représente un groupe éthylène, un composé de formule générale

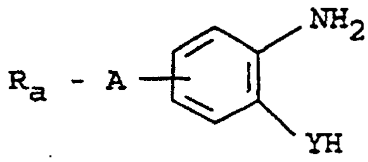

, (XV)

où

$R_a$ et A sont définis comme dans les revendications 1 à 8 et Y représente un atome de soufre ou un atome d'azote substitué par un groupe alkyle en $C_{1-3}$ ou cyclopropyle, est mis à réagir avec un composé de formule générale

$$HO - CO - CH_2CH_2 - Ar - E \qquad (XVI)$$

où

Ar et E sont définis de la manière indiquée dans les revendications 1 à 8, ou avec ses dérivés réactifs ou f. pour la préparation d'un composé de formule générale I où $R_2$ représente un groupe alkyle en $C_{1-4}$ qui est substitué par un groupe alkylsulfonylaminocarbonyle, un composé de formule générale

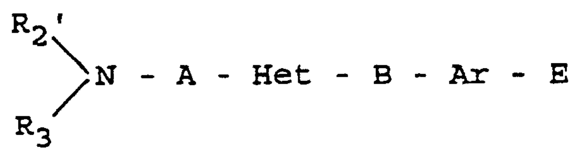

, (IXX)

où

$R_3$, A, B, E et Het sont définis de la manière indiquée dans les revendications 1 à 8 et
$R_2'$ représente un groupe alkyle en $C_{1-4}$ qui est substitué par un groupe carboxyle, ou ses dérivés réactifs, est mis à réagir avec un sel d'un composé de formule générale

$$\text{Alkyle en } C_{1-3}\text{-}SO_2\text{-}NH_2 \qquad (XX)$$

et
si nécessaire, un reste protecteur utilisé pendant les réactions pour la protection de groupes réactifs est clivé et/ou
si on le souhaite, un composé de formule générale I ainsi obtenu est ensuite résolu en ses stéréoisomères et/ou un composé de formule générale I ainsi obtenu est converti en ses sels, en particulier pour l'utilisation pharmaceutique en ses sels physiologiquement acceptables avec un acide ou une base inorganique ou organique.